# EUROPEAN PATENT APPLICATION

(11) **EP 3 964 497 A1**
(43) Date of publication of application: **09.03.2022**
(21) Application number: 20194568.0
(22) Date of filing: 04.09.2020
(51) Int. Cl.: C07C 275/24, C07C 275/26, C07D 333/74, A61P 25/36, A61K 31/36

(54) **SUBSTITUTED VICINAL DIAMINE COMPOUNDS AND THEIR USE IN THE TREATMENT, AMELIORATION OR PREVENTION OF PAIN**

(71) Applicant: Friedrich-Alexander-Universität Erlangen-Nürnberg, 91054 Erlangen (DE)
(72) Inventor: GMEINER, Peter, 91054 Buckenhof (DE); HUEBNER, Harald, 91336 Heroldsbach (DE); HETZER, Florian, 91052 Erlangen (DE)
(74) Representative: Vossius & Partner Patentanwälte Rechtsanwälte mbB

(57) **Abstract**

The present invention relates to a compound of the following formula (I) or (II), optionally in the form of a pharmaceutically acceptable salt, solvate, polymorph, cocrystal, tautomer, racemate, enantiomer, or diastereomer or mixture thereof:

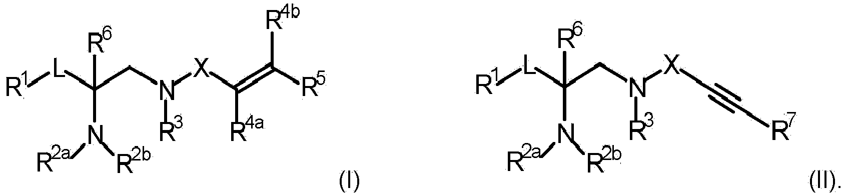

The invention furthermore relates to a pharmaceutical composition comprising the compound having formula (I) or (II) and optionally one or more pharmaceutically acceptable excipient(s) and/or carrier(s), as well as to these compounds for use in the treatment, amelioration or prevention of pain, opioid overdose, addictions, neuropsychiatric disorders, Prader-Willi Syndrome, gastrointestinal disorders, skin disorders, dyspnea, headache and/or temporomandibular joint dysfunction, including methods of treating, ameliorating or preventing pain involving administering to a patient in need thereof an effective amount of the compound having formula (I) or (II).

## Description

The present invention relates to a compound of formula (I) or (II), to a pharmaceutical composition comprising the compound of formula (I) or (II), as well as to these compounds for use in the treatment, amelioration or prevention of pain, opioid overdose, addiction, neuropsychiatric disorder, Prader-Willi Syndrome, gastrointestinal disorder, skin disorder, dyspnea, and temporomandibular joint dysfunction, including methods of treating, ameliorating or preventing pain, opioid overdose, addiction, neuropsychiatric disorder, Prader-Willi Syndrome, gastrointestinal disorder, skin disorder, dyspnea, and temporomandibular joint dysfunction involving administering to a patient in need thereof an effective amount of the compound having formula (I) or (II).

### Background

Prescription opioids are used for the treatment of chronic and severe pain. When used appropriately, they are an essential part of pain therapy. But opioids may also pose serious risks, such as respiratory depression, constipation and addiction. The analgesic effects of opioid drugs are mainly mediated by the activation of the µ-opioid receptor (µOR) subtype. Functionally selective drugs, which are biased towards Gi protein activation over β-arrestin signaling at the µOR have been associated with an improved side effect profile in molecular studies recently. Simultaneously, substances acting on the CNS-located opioid receptors have to be able to reach sufficient brain concentrations for the mediation of analgesia. Thus, pharmacokinetic properties like metabolic stability and blood-brain-barrier permeability have to be comprised.

WO 2017/007695, WO 2018/129393 and WO 2019/036678 describe certain compounds which are intended to be used for treating pain in a subject in need of the treatment, which is described as reducing the risk of respiratory depression or constipation.

In view of the problems of the prior art, the compounds described herein combine Gi protein pathway selective µOR activity with desirable pharmacokinetic properties.

### Summary of the invention

The present invention relates to a compound of the following formula (I) or (II), optionally in the form of a pharmaceutically acceptable salt, solvate, polymorph, cocrystal, tautomer, racemate, enantiomer, or diastereomer or mixture thereof: 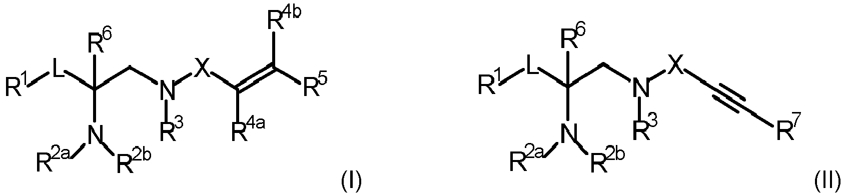 wherein
R¹ is an optionally substituted aryl group or an optionally substituted heteroaryl group,
L is selected from a bond and an optionally substituted alkylene and an optionally substituted heteroalkylene,
R^{2a} and R^{2b} are independently selected from hydrogen, optionally substituted alkyl, optionally substituted heteroalkyl, optionally substituted cycloalkyl, and optionally substituted heterocycloalkyl,
wherein, when at least one of R^{2a} and R^{2b} is optionally substituted alkyl, the optionally substituted alkyl of R^{2a} or R^{2b} is optionally linked to R¹ via a bond between a non-hydrogen atom of R^{2a} or R^{2b} and a non-hydrogen atom of R¹,
R³ is selected from hydrogen and optionally substituted alkyl,
X is selected from >C=O, >C=S, C=N(NO₂), C=C(NO₂)(H), >C=C(F)(H), >C=CF₂, >C=N(F), >C=C(CN)(H), >C=N(CN), >C=C(CN)(CN), >S(O), and >S(O)₂,
R^{4a} and R^{4b} are independently selected from hydrogen, optionally substituted alkyl and optionally substituted heteroalkyl,
R⁵ and R⁷ are independently selected from optionally substituted cycloalkyl, optionally substituted heterocycloalkyl, optionally substituted aryl and optionally substituted heteroaryl,
R⁶ is hydrogen or optionally substituted alkyl, wherein the optionally substituted alkyl of R⁶ is optionally linked to R¹ via a bond between a non-hydrogen atom of R⁶ and a non-hydrogen atom of R¹,
wherein
any hydrogen atoms in formulae (I) and (II) are optionally independently replaced by fluorine,
the one or more optional substituents of the optionally substituted aryl group and the optionally substituted heteroaryl group are independently selected from the group consisting of -halogen, -CN, -C(O)R**, -COOR**, -C(O)NR**R**, -NR**R**, -NR**-C(O)R**, -N(R**)-C(O)-OR**, -N(R**)-C(O) -NR**R**, -N(R**)-S(O)₂R**, -(optionally substituted alkyl), -OR**, -(optionally substituted heterocyclyl), -(optionally substituted alkylene)-(optionally substituted heterocyclyl), -(optionally substituted carbocyclyl), and -(optionally substituted alkylene)-(optionally substituted carbocyclyl), wherein R** is H, -(optionally substituted alkyl), -(optionally substituted heterocyclyl), or -(optionally substituted carbocyclyl), and
wherein the optional substituent of the optionally substituted alkyl and the optionally substituted alkylene can be independently -halogen, -CN, -NO₂, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar}, -N(R^{ar})-S(O)₂-NR^{ar}R^{ar}, -P(O)(OR^{ar})₂, or -O-P(O)(OR^{ar})₂; wherein R^{ar} is H, alkyl, heterocyclyl or carbocyclyl;
wherein the optional substituent of the optionally substituted heterocyclyl, the optionally substituted carbocyclyl can be independently alkyl, -halogen, -CN, -NO₂, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar}, -N(R^{ar})-S(O)₂-NR^{ar}R^{ar}, -P(O)(OR^{ar})₂, or -O-P(O)(OR^{ar})₂; wherein R^{ar} is H, alkyl, heterocyclyl or carbocyclyl;
the one or more optional substituents of the optionally substituted alkyl, optionally substituted alkylene, optionally substituted heteroalkylene, and optionally substituted heteroalkyl are independently selected from -halogen, -CN, -NO₂, oxo, -C(O)R^{alk}, -COOR^{alk}, -C(O)NR^{alk}R^{alk}, -NR^{alk}R^{alk}, -NR^{alk}-C(O)R^{alk}, -N(R^{alk})-C(O)-OR^{alk}, -N(R^{alk})-C(O)-NR^{alk}R^{alk}, -N(R^{alk})-S(O)₂R^{alk}, -OR^{alk}, -O-C(O)R^{alk}, -O-C(O)-NR^{alk}R^{alk}, -SR^{alk}, -S(O)R^{alk}, -S(O)₂R^{alk}, -S(O)₂-NR^{alk}R^{alk}, -N(R^{alk})-S(O)₂-NR^{alk}R^{alk}, -P(O)(OR^{alk})₂, and -O-P(O)(OR^{alk})₂; wherein R^{alk} is H, alkyl or alkyl substituted with one or more F, and
the one or more optional substituents of the optionally substituted cycloalkyl and optionally substituted heterocycloalkyl are independently selected from -alkyl, -halogen, -CN, -NO₂, oxo, -C(O)R^{cyc}, -COOR^{cyc}, -C(O)NR^{cyc}R^{cyc}, -NR^{cyc}R^{cyc}, -NR^{cyc}-C(O)R^{cyc}, -N(R^{cyc})-C(O)-OR^{cyc}, -N(R^{cyc})-C(O)-NR^{cyc}R^{cyc}, -N(R^{cyc})-S(O)₂R^{cyc}, -OR^{cyc}, -O-C(O)R^{cyc}, -O-C(O)-NR^{cyc}R^{cyc}, -SR^{cyc}, -S(O)R^{cyc}, -S(O)₂R^{cyc}, -S(O)₂-NR^{cyc}R^{cyc}, -N(R^{cyc})-S(O)₂-NR^{cyc}R^{cyc}, -P(O)(OR^{cyc})₂, and -O-P(O)(OR^{cyc})₂; wherein R^{cyc} is H, alkyl or alkyl substituted with one or more F.

The present invention furthermore relates to a pharmaceutical composition comprising a compound having formula (I) or (II), optionally in the form of a pharmaceutically acceptable salt, solvate, polymorph, cocrystal, tautomer, racemate, enantiomer, or diastereomer or mixture thereof, and optionally one or more pharmaceutically acceptable excipient(s) and/or carrier(s).

The present invention also concerns a compound having formula (I) or (II), optionally in the form of a pharmaceutically acceptable salt, solvate, polymorph, cocrystal, tautomer, racemate, enantiomer, or diastereomer or mixture thereof, wherein the compound is for use in the treatment, amelioration or prevention of pain, opioid overdose, addiction, neuropsychiatric disorder, Prader-Willi Syndrome, gastrointestinal disorder, skin disorder, dyspnea, and temporomandibular joint dysfunction.

In addition, the present invention relates to a method of treating, ameliorating or preventing pain, opioid overdose, addiction, neuropsychiatric disorder, Prader-Willi Syndrome, gastrointestinal disorder, skin disorder, dyspnea, and temporomandibular joint dysfunction, the method comprising administering to a patient in need thereof an effective amount of a compound having formula (I) or (II), optionally in the form of a pharmaceutically acceptable salt, solvate, polymorph, cocrystal, tautomer, racemate, enantiomer, or diastereomer or mixture thereof.

Furthermore, the present invention refers to the use of a compound having formula (I) or (II), optionally in the form of a pharmaceutically acceptable salt, solvate, polymorph, cocrystal, tautomer, racemate, enantiomer, or diastereomer or mixture thereof, for the manufacture of a medicament for treating, ameliorating or preventing pain, opioid overdose, addiction, neuropsychiatric disorder, Prader-Willi Syndrome, gastrointestinal disorder, skin disorder, dyspnea, and temporomandibular joint dysfunction.

### Brief description of the Figures

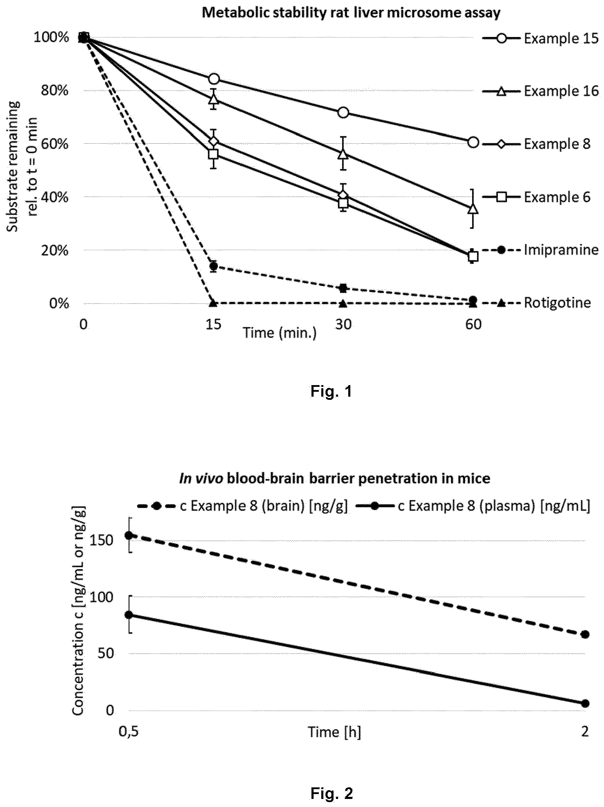

- Fig. 1:: Rat liver microsome assay for the assessment of metabolic stability of selected compounds of the experimental examples (Example A-5: Metabolic stability in rat liver microsomes). Data was pooled from three independent experiments. The reference substances imipramine and rotigotine, which are known to be rapidly metabolized, were tested in parallel. The concentrations of non-metabolized compounds (remaining substrate) were determined by LC-MS after the addition of an internal standard. Samples were collected at 0 min, 15 min, 30 min and 60 min. Unspecific binding to the matrix was assessed by parallel control experiments in the absence of the cofactor NADPH, determined after 60 minutes (blind value).
- Fig. 2:: In vivo blood-brain barrier penetration of example compound 8 of the experimental examples in mice examples (Example A-5: Blood-brain barrier penetration in vivo). Subcutaneous administration at a dose of 20 mg / kg. Plasma and brain samples taken from three individual animals at two time points 0.5 h and 2 h.

### Detailed description of the invention

The present invention relates to a compound of the following formula (I) or (II), optionally in the form of a pharmaceutically acceptable salt, solvate, polymorph, cocrystal, tautomer, racemate, enantiomer, or diastereomer or mixture thereof:

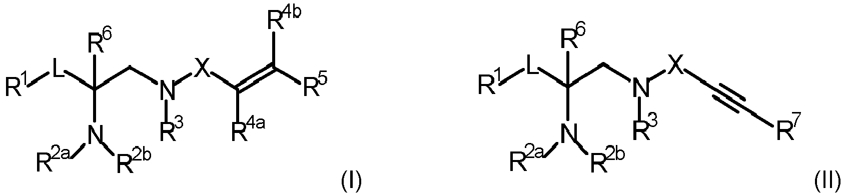

In formulae (I) and (II) R¹, R^{2a}, R^{2b}, R³, R^{4a}, R^{4b}, R⁵, R⁶, R⁷, L and X are defined as follows.

R¹ is an optionally substituted aryl group or an optionally substituted heteroaryl group. Preferably, R¹ is an optionally substituted phenyl group. It is to be understood that the optional substituent of the optionally substituted phenyl group is selected from the same list as the optional substituent of the optionally substituted aryl group. The optional substituent can be present at any available position but is typically present in para-position compared to the point of attachment to L. More preferably, R¹ is a phenyl group containing a hydroxy or amido substituent in para position and optionally one or two further substituents selected from methyl and fluoro. Even more preferably, R¹ is a phenyl group containing a hydroxy or amido (e.g., -C(O)-NH₂) substituent in para position and optionally one or two further methyl substituents. Still more preferably, R¹ is a phenyl group containing at least one hydroxy or amido substituent in para position. Still more preferably, R¹ is a phenyl group having a hydroxy substituent in para position (4-hydroxyphenyl).

When R¹ is linked to R⁶ via a bond between a non-hydrogen atom of R⁶ and a non-hydrogen atom of R¹, this linked group is formed (in theory) by abstracting a hydrogen radical from R¹ and a hydrogen radical from R⁶ and forming a bond between the thus formed two radicals of R¹ and R⁶. It is preferred that the non-hydrogen atoms between which the link is formed be both carbon atoms. Alternatively, one of the two non-hydrogen atoms may be a carbon atom and the other may be an oxygen atom, or one of the two non-hydrogen atoms may be a carbon atom and the other may be a nitrogen atom.

L is selected from a bond and an optionally substituted alkylene and an optionally substituted heteroalkylene. Preferably, L is selected from a bond, an optionally substituted methylene and an optionally substituted ethylene. Further preferably, L is an optionally substituted methylene group, more preferably an unsubstituted methylene group (-CH₂-). It is to be understood that the optional substituent of the optionally substituted methylene group is selected from the same list as the optional substituent of the optionally substituted alkylene group.

R^{2a} and R^{2b} are independently selected from hydrogen, optionally substituted alkyl, optionally substituted heteroalkyl, optionally substituted cycloalkyl, and optionally substituted heterocycloalkyl, provided that, when at least one of R^{2a} and R^{2b} is optionally substituted alkyl, the optionally substituted alkyl of R^{2a} or R^{2b} can optionally be linked to R¹ via a bond between a non-hydrogen atom of R^{2a} or R^{2b} and a non-hydrogen atom of R¹. Preferably, R^{2a} and R^{2b} are independently selected from hydrogen and optionally substituted alkyl. More preferably, R^{2a} and R^{2b} are independently selected from hydrogen, alkyl and haloalkyl. Even more preferably, R^{2a} and R^{2b} are independently selected from hydrogen and alkyl. Still more preferably, R^{2a} and R^{2b} are independently selected from methyl, ethyl and propyl. Most preferably, both R^{2a} and R^{2b} are methyl.

When R¹ is linked to R^{2a} via a bond between a non-hydrogen atom of R^{2a} and a non-hydrogen atom of R¹, this linked group is formed (in theory) by abstracting a hydrogen radical from R¹ and a hydrogen radical from R^{2a} and forming a bond between the thus formed two radicals of R¹ and R^{2a}. It is preferred that the non-hydrogen atoms between which the link is formed be both carbon atoms. Alternatively, one of the two non-hydrogen atoms may be a carbon atom and the other may be an oxygen atom, or one of the two non-hydrogen atoms may be a carbon atom and the other may be a nitrogen atom. Conversely, when R¹ is linked to R^{2b} via a bond between a non-hydrogen atom of R^{2b} and a non-hydrogen atom of R¹, this linked group is formed (in theory) by abstracting a hydrogen radical from R¹ and a hydrogen radical from R^{2b} and forming a bond between the thus formed two radicals of R¹ and R^{2b}. It is preferred that the non-hydrogen atoms between which the link is formed be both carbon atoms. Alternatively, one of the two non-hydrogen atoms may be a carbon atom and the other may be an oxygen atom, or one of the two non-hydrogen atoms may be a carbon atom and the other may be a nitrogen atom.

R³ is selected from hydrogen and optionally substituted alkyl. R³ is preferably selected from hydrogen and alkyl. More preferably, R³ is hydrogen.

X is selected from >C=O, >C=S, C=N(NO₂), C=C(NO₂)(H), >C=C(F)(H), >C=CF₂, >C=N(F), >C=C(CN)(H), >C=N(CN), >C=C(CN)(CN), >S(O), and >S(O)₂. Preferably, X is selected from >C=O, >C=S, >C=C(NO₂)(H), >C=C(CN)(H), >C=C(CN)(CN), >S(O), and >S(O)₂. More preferably, X is selected from >C=O and >C=S, even more preferably >C=O.

R^{4a} and R^{4b} are independently selected from hydrogen, optionally substituted alkyl and optionally substituted heteroalkyl. Preferably, R^{4a} and R^{4b} are independently selected from hydrogen, optionally substituted methyl, optionally substituted ethyl and optionally substituted propyl, wherein preferably at least one of R^{4a} and R^{4b} is hydrogen. More preferably, R^{4a} and R^{4b} are independently selected from hydrogen, methyl, ethyl and propyl, wherein preferably at least one of R^{4a} and R^{4b} is hydrogen, even more preferably hydrogen or methyl. It is to be understood that the optional substituent of the optionally substituted methyl, optionally substituted ethyl and optionally substituted propyl is selected from the same list as the optional substituent of the optionally substituted alkyl group.

R⁵ is selected from optionally substituted cycloalkyl, optionally substituted heterocycloalkyl, optionally substituted aryl and optionally substituted heteroaryl. Preferably, R⁵ is selected from an optionally substituted aryl group and an optionally substituted heteroaryl group. Even more preferably, R⁵ is selected from optionally substituted phenyl, optionally substituted naphthyl, optionally substituted pyridyl and optionally substituted thienyl. In a preferred option, the substituent can be -CF₃.

R⁷ is selected from optionally substituted cycloalkyl, optionally substituted heterocycloalkyl, optionally substituted aryl and optionally substituted heteroaryl. Preferably, R⁷ is selected from an optionally substituted aryl group and an optionally substituted heteroaryl group. Even more preferably, R⁷ is selected from optionally substituted phenyl, optionally substituted naphthyl, optionally substituted pyridyl and optionally substituted thienyl. In a preferred option, the substituent can be -CF₃.

It is to be understood that the optional substituent of the optionally substituted phenyl and the optionally substituted naphthyl is selected from the same list as the optional substituent of the optionally substituted aryl group. Furthermore, it is to be understood that the optional substituent of the optionally substituted pyridyl and optionally substituted thienyl is selected from the same list as the optional substituent of the optionally substituted heteroaryl group.

R⁶ is hydrogen or optionally substituted alkyl, wherein the optionally substituted alkyl of R⁶ can optionally be linked to R¹ via a bond between a non-hydrogen atom of R⁶ and a non-hydrogen atom of R¹. Preferably, R⁶ is hydrogen or optionally substituted alkyl, wherein alkyl is preferably selected from methyl, ethyl and propyl, more preferably methyl or ethyl, even more preferably methyl. R⁶ is most preferably hydrogen.

Any hydrogen atoms in formulae (I) and (II) can be optionally independently replaced by fluorine. Preferably, only hydrogen atoms bound to a carbon atom are optionally independently replaced by fluorine. However, it is preferred that none of the hydrogen atoms in formulae (I) and (II) are optionally independently replaced by fluorine.

The one or more optional substituents of the optionally substituted aryl group and the optionally substituted heteroaryl group are independently selected from the group consisting of -halogen, -CN, -C(O)R**, -COOR**, -C(O)NR**R**, -NR**R**, -NR**-C(O)R**, -N(R**)-C(O)-OR**, -N(R**)-C(O) -NR**R**, -N(R**)-S(O)₂R**, -(optionally substituted alkyl), -OR**, -(optionally substituted heterocyclyl), -(optionally substituted alkylene)-(optionally substituted heterocyclyl), -(optionally substituted carbocyclyl), and -(optionally substituted alkylene)-(optionally substituted carbocyclyl). Preferably, the one or more optional substituents of the optionally substituted aryl group and the optionally substituted heteroaryl group are independently selected from the group consisting of -halogen, -CN, -C(O)R**, -COOR**, -C(O)NR**R**, -NR**R**, -NR**-C(O)R**, -N(R**)-C(O)-OR**, -N(R**)-C(O) -NR**R**, -N(R**)-S(O)₂R**, -(optionally substituted alkyl), and -OR**. More preferably, the one or more optional substituents of the optionally substituted aryl group and the optionally substituted heteroaryl group are independently selected from the group consisting of -halogen, -CN, -C(O)R**, -COOR**, -C(O)NR**R**, -NR**R**, -NR**-C(O)R**, -N(R**)-C(O)-OR**, -N(R**)-C(O) -NR**R**, -N(R**)-S(O)₂R**, -(optionally substituted alkyl), and -OR**. Even more preferably, the one or more optional substituents of the optionally substituted aryl group and the optionally substituted heteroaryl group are independently selected from the group consisting of -halogen, -CN, -C(O)R**, -COOR**, -C(O)NR**R**, -NR**R**, -NR**-C(O)R**, -(optionally substituted alkyl), and -OR**.

R** is H, -(optionally substituted alkyl), -(optionally substituted heterocyclyl), or -(optionally substituted carbocyclyl), preferably R** is H or -(optionally substituted alkyl).

Within the definition of the one or more optional substituents of the optionally substituted aryl group and the optionally substituted heteroaryl group as given above, the optional substituent of the optionally substituted alkyl and the optionally substituted alkylene can be independently -halogen, -CN, -NO₂, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar}, -N(R^{ar})-S(O)₂-NR^{ar}R^{ar}, -P(O)(OR^{ar})₂, or -O-P(O)(OR^{ar})₂; wherein R^{ar} is H, alkyl, heterocyclyl or carbocyclyl. Preferably, the optional substituent of the optionally substituted alkyl and the optionally substituted alkylene can be independently -halogen, -CN, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar} or -N(R^{ar})-S(O)₂-NR^{ar}R^{ar}; wherein R^{ar} is H, alkyl, heterocyclyl or carbocyclyl. More preferably, the optional substituent of the optionally substituted alkyl and the optionally substituted alkylene can be independently -halogen, -CN, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar} or -N(R^{ar})-S(O)₂-NR^{ar}R^{ar}; wherein R^{ar} is H or alkyl.

Within the definition of the one or more optional substituents of the optionally substituted aryl group and the optionally substituted heteroaryl group as given above, the optional substituent of the optionally substituted heterocyclyl and the optionally substituted carbocyclyl can be independently alkyl, -halogen, -CN, -NO₂, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar}, -N(R^{ar})-S(O)₂-NR^{ar}R^{ar}, -P(O)(OR^{ar})₂, or -O-P(O)(OR^{ar})₂. Preferably, the optional substituent of the optionally substituted heterocyclyl and the optionally substituted carbocyclyl can be independently alkyl, -halogen, -CN, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar} or -N(R^{ar})-S(O)₂-NR^{ar}R^{ar}. More preferably, the optional substituent of the optionally substituted heterocyclyl and the optionally substituted carbocyclyl can be independently alkyl, -halogen, -CN, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar} or -N(R^{ar})-S(O)₂-NR^{ar}R^{ar}.

R^{ar} is preferably H, alkyl, heterocyclyl or carbocyclyl. More preferably, R^{ar} is H or alkyl.

The one or more optional substituents of the optionally substituted alkyl, optionally substituted alkylene, optionally substituted heteroalkylene, and optionally substituted heteroalkyl are independently selected from -halogen, -CN, -NO₂, oxo, -C(O)R^{alk}, -COOR^{alk}, -C(O)NR^{alk}R^{alk}, -NR^{alk}R^{alk}, -NR^{alk}-C(O)R^{alk}, -N(R^{alk})-C(O)-OR^{alk}, -N(R^{alk})-C(O)-NR^{alk}R^{alk}, -N(R^{alk})-S(O)₂R^{alk}, -OR^{alk}, -O-C(O)R^{alk}, -O-C(O)-NR^{alk}R^{alk}, -SR^{alk}, -S(O)R^{alk}, -S(O)₂R^{alk}, -S(O)₂-NR^{alk}R^{alk}, -N(R^{alk})-S(O)₂-NR^{alk}R^{alk}, -P(O)(OR^{alk})_{2,} and -O-P(O)(OR^{alk})₂. Preferably, the one or more optional substituents of the optionally substituted alkyl, optionally substituted alkylene, optionally substituted heteroalkylene, and optionally substituted heteroalkyl are independently selected from -halogen, -CN, oxo, -C(O)R^{alk}, -COOR^{alk}, -C(O)NR^{alk}R^{alk}, -NR^{alk}R^{alk}, -NR^{alk}-C(O)R^{alk}, -N(R^{alk})-C(O)-OR^{alk}, -N(R^{alk})-C(O)-NR^{alk}R^{alk}, -N(R^{alk})-S(O)₂R^{alk}, -OR^{alk}, -O-C(O)R^{alk}, -O-C(O)-NR^{alk}R^{alk}, -SR^{alk}, -S(O)R^{alk}, -S(O)₂R^{alk}, -S(O)₂-NR^{alk}R^{alk}, and -N(R^{alk})-S(O)₂-NR^{alk}R^{alk}. More preferably, the one or more optional substituents of the optionally substituted alkyl, optionally substituted alkylene, optionally substituted heteroalkylene, and optionally substituted heteroalkyl are independently selected from -halogen, -CN, oxo, -C(O)R^{alk}, -COOR^{alk}, -S(O)R^{alk}, ⁻S(O)₂R^{alk} and -S(O)₂-NR^{alk}R^{alk}. -N(R^{alk})-S(O)₂R^{alk}, -OR^{alk}, -O-C(O)R^{alk}, -SR^{alk},

R^{alk} is preferably H, alkyl or alkyl substituted with one or more F. More preferably, R^{alk} is H or alkyl.

The one or more optional substituents of the optionally substituted cycloalkyl and optionally substituted heterocycloalkyl are independently selected from -alkyl, -halogen, -CN, -NO₂, oxo, -C(O)R^{cyc}, -COOR^{cyc}, -C(O)NR^{cyc}R^{cyc}, -NR^{cyc}R^{cyc}, -NR^{cyc}-C(O)R^{cyc}, -N(R^{cyc})-C(O)-OR^{cyc}, -N(R^{cyc})-C(O)-NR^{cyc}R^{cyc}, -N(R^{cyc})-S(O)₂R^{cyc}, -OR^{cyc}, -O-C(O)R^{cyc}, -O-C(O)-NR^{cyc}R^{cyc}, -SR^{cyc}, -S(O)R^{cyc}, -S(O)₂R^{cyc}, -S(O)₂-NR^{cyc}R^{cyc}, -N(R^{cyc})-S(O)₂-NR^{cyc}R^{cyc}, -P(O)(OR^{cyc})₂, and -O-P(O)(OR^{cyc})₂. Preferably, the one or more optional substituents of the optionally substituted cycloalkyl and optionally substituted heterocycloalkyl are independently selected from -alkyl, -halogen, -CN, oxo, -C(O)R^{cyc}, -COOR^{cyc}, -C(O)NR^{cyc}R^{cyc}, -NR^{cyc}R^{cyc}, -NR^{cyc}-C(O)R^{cyc}, -N(R^{cyc})-C(O)-OR^{cyc}, -N(R^{cyc})-C(O)-NR^{cyc}R^{cyc}, -N(R^{cyc})-S(O)₂R^{cyc}, -OR^{cyc}, -O-C(O)R^{cyc}, -O-C(O)-NR^{cyc}R^{cyc}, -SR^{cyc}, -S(O)R^{cyc}, -S(O)₂R^{cyc}, -S(O)₂-NR^{cyc}R^{cyc} and -N(R^{cyc})-S(O)₂-NR^{cyc}R^{cyc}. More preferably, the one or more optional substituents of the optionally substituted cycloalkyl and optionally substituted heterocycloalkyl are independently selected from -alkyl, -halogen, -CN, oxo, -C(O)R^{cyc}, -COOR^{cyc}, -C(O)NR^{cyc}R^{cyc}, -NR^{cyc}R^{cyc}, -NR^{cyc}-C(O)R^{cyc}, -N(R^{cyc})-S(O)₂R^{cyc}, -OR^{cyc}, -O-C(O)R^{cyc}, -SR^{cyc}, -S(O)R^{cyc}, -S(O)₂R^{cyc} and -S(O)₂-N R^{cyc}R^{cyc}.

R^{cyc} is preferably H, alkyl or alkyl substituted with one or more F. More preferably, R^{cyc} is H or alkyl.

The compounds of formulae (I) and (II) are preferably represented by the following formulae (I-a) and (II-b), respectively: 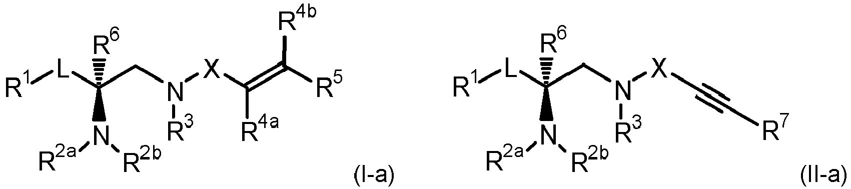 wherein R¹, R^{2a}, R^{2b}, R³, R^{4a}, R^{4b}, R⁵, R⁶, R⁷, L and X are as defined above.

More preferably, the compounds of formulae (I) and (II) are selected from (S)-N-(2-(dimethylamino)-3-(4-hydroxyphenyl)propyl)cinnamamide, (S)-N-(2-(dimethylamino)-3-(4-hydroxyphenyl)propyl)-3-phenylpropiolamide, (*S*,*E*)-N-(2-(dimethylamino)-3-(4-hydroxyphenyl)propyl)-3-phenylbut-2-enamide, (*S*,*E*)-N-(2-(dimethylamino)-3-(4-hydroxyphenyl)propyl)-2-methyl-3-phenylacrylamide, (*S*)-N-(2-(dimethylamino)-3-(4-hydroxyphenyl)-2-methylpropyl)cinnamamide (*S*,*E*)-N-(2-(dimethylamino)-3-(4-hydroxyphenyl)propyl)-3-(naphthalen-1-yl)acrylamide, (*S*,*E*)-N-(2-(dimethylamino)-3-(4-hydroxyphenyl)propyl)-3-(2-(trifluoromethyl)phenyl)acrylamide, (*S,E*)-N-(2-(dimethylamino)-3-(4-hydroxyphenyl)propyl)-3-(3-(trifluoromethyl)phenyl)acrylamide, (*S*,*E*)-N-(2-(dimethylamino)-3-(4-hydroxyphenyl)propyl)-3-(4-(trifluoromethyl)phenyl)acrylamide, (*S*)-N-(2-amino-3-(4-hydroxy-2,6-dimethylphenyl)propyl)cinnamamide, (*S*,*E*)-N-(2-amino-3-(4-hydroxy-2,6-dimethylphenyl)propyl)-3-(3-(trifluoromethyl)phenyl)acrylamide, (*S*,*E*)-N-(3-(4-hydroxy-2,6-dimethylphenyl)-2-(methylamino)propyl)-3-(3-(trifluoromethyl)phenyl)acrylamide, (*S*,*E*)-N-(2-(dimethylamino)-3-(4-hydroxyphenyl)propyl)-3-(m-tolyl)acrylamide, (*S*,*E*)-4-(2-amino-3-(3-(3-(trifluoromethyl)phenyl)acrylamido)propyl)benzamide, (*S*,*E*)-4-(2-(dimethylamino)-3-(3-(3-(trifluoromethyl)phenyl)acrylamido)propyl)benzamide, (*S*,*E*)-N-(2-(dimethylamino)-3-(4-hydroxyphenyl)propyl)-3-(4-fluoro-3-(trifluoromethyl)phenyl)acrylamide, (*S*,*E*)-4-(2-amino-3-(3-(naphthalen-1-yl)acrylamido)propyl)benzamide, (*S*,*E*)-4-(2-(dimethylamino)-3-(3-(naphthalen-1-yl)acrylamido)propyl)benzamide, and (*S*,*E*)-N-(2-(dimethylamino)-3-(4-hydroxyphenyl)propyl)-3-(thiophen-3-yl)acrylamide, each optionally in the form of a pharmaceutically acceptable salt, solvate, polymorph, cocrystal, tautomer, racemate, enantiomer, or diastereomer or mixture thereof.

The present invention furthermore relates to a pharmaceutical composition comprising a compound having formula (I) or (II), optionally in the form of a pharmaceutically acceptable salt, solvate, polymorph, cocrystal, tautomer, racemate, enantiomer, or diastereomer or mixture thereof, and optionally one or more pharmaceutically acceptable excipient(s) and/or carrier(s).

The present invention also concerns a compound having formula (I) or (II), optionally in the form of a pharmaceutically acceptable salt, solvate, polymorph, cocrystal, tautomer, racemate, enantiomer, or diastereomer or mixture thereof, wherein the compound is for use in the treatment, amelioration or prevention of pain, opioid overdose, addiction, neuropsychiatric disorder, Prader-Willi Syndrome, gastrointestinal disorder, skin disorder, dyspnea, and temporomandibular joint dysfunction.

In addition, the present invention relates to a method of treating, ameliorating or preventing pain, opioid overdose, addiction, neuropsychiatric disorder, Prader-Willi Syndrome, gastrointestinal disorder, skin disorder, dyspnea, and temporomandibular joint dysfunction, the method comprising administering to a patient in need thereof an effective amount of a compound having formula (I) or (II), optionally in the form of a pharmaceutically acceptable salt, solvate, polymorph, cocrystal, tautomer, racemate, enantiomer, or diastereomer or mixture thereof.

Furthermore, the present invention refers to the use of a compound having formula (I) or (II), optionally in the form of a pharmaceutically acceptable salt, solvate, polymorph, cocrystal, tautomer, racemate, enantiomer, or diastereomer or mixture thereof, for the manufacture of a medicament for treating, ameliorating or preventing pain, opioid overdose, addiction, neuropsychiatric disorder, Prader-Willi Syndrome, gastrointestinal disorder, skin disorder, dyspnea, and temporomandibular joint dysfunction.

The following definitions apply throughout the present specification, unless specifically indicated otherwise.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. Unless defined otherwise, the term "alkyl" preferably refers to C₁₋₆ alkyl, more preferably C₁₋₄ alkyl. Preferred exemplary alkyl groups are methyl, ethyl, propyl (e.g., n-propyl or isopropyl), or butyl (e.g., n-butyl, isobutyl, sec-butyl, or tert-butyl), more preferably methyl or ethyl, and even more preferably methyl.

As used herein, the term "heteroalkyl" refers to an alkyl group in which one or more, e.g., one or two (preferably one) of the -CH₂- groups have been replaced each independently by a group selected from -O-, -S-, -S(O)-, -S(O)₂- and -N(alkyl)-. Unless defined otherwise, the term "heteroalkyl" preferably refers to C₁₋₆ heteroalkyl, more preferably C₁₋₄ heteroalkyl, even more preferably to methylamino, dimethylamino, ethylamino, diethylamino, methoxy or ethoxy, even more preferably to methoxy or ethoxy, and still more preferably to methoxy.

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂.

As used herein, the term "alkylene" refers to an alkanediyl group, i.e. a divalent saturated acyclic hydrocarbon group which may be linear or branched, preferably linear. Unless defined otherwise, the term "alkylene" preferably refers to C₁₋₆ alkylene, more preferably C₁₋₄ alkylene. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-), more preferably methylene or ethylene, and even more preferably methylene.

As used herein, the term "heteroalkylene" refers to an alkylene group in which one or more (e.g., one or two, preferably one) of the -CH₂- groups have been replaced each independently by a group selected from -O-, -S-, -S(O)-, -S(O)₂- and -N(alkyl)-. Unless defined otherwise, the term "heteroalkylene" preferably refers to C₁₋₆ heteroalkylene , in particular C₁₋₄ heteroalkylene (including, in particular, linear C₁₋₄ heteroalkylene), more preferably to -G-CH₂-, -G-CH₂-CH₂-, -G-CH₂-CH₂-CH₂-, -CH₂-G-, -CH₂-G-CH₂- -CH₂-G-CH₂-CH₂-, -CH₂-CH₂-G-, -CH₂-CH₂-G-CH₂- or -CH₂-CH₂-CH₂-G-, wherein each G is independently selected from -O-, -S-, -S(O)-, -S(O)₂- and -N(alkyl)-, preferably from -N(alkyl)- and -O-, more preferably from -O-. More preferably, the term "heteroalkylene" refers to -G-CH₂-, -G-CH₂-CH₂-, -CH₂-G-, -CH₂-G-CH₂- or -CH₂-CH₂-G-, wherein each G is independently selected from -O-, -S-, -S(O)-, -S(O)₂- and -N(alkyl)-, preferably from -N(alkyl)- and -O- , more preferably from -O-. Even more preferably, the term "heteroalkylene" refers to -G-CH₂- or -CH₂-G-, wherein each G is independently selected from -O-, -S-, -S(O)-, -S(O)₂- and -N(alkyl)-, preferably from -N(alkyl)- and -O-, more preferably from -O-.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl. The number of carbon atoms in the carbocyclyl ring is not particularly limited and is preferably 3 to 14, more preferably 3 to 10, even more preferably 3 to 7.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. Unless defined otherwise, "heterocyclyl" preferably refers to heteroaryl, heterocycloalkyl or heterocycloalkenyl. The number of carbon atoms in the carbocyclyl ring is not particularly limited and is preferably 3 to 14, preferably 3 to 10, more preferably 3 to 7.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). Unless defined otherwise, an "aryl" preferably has 5 to 14 ring atoms, more preferably 6 to 10 ring atoms. "Aryl" may, e.g., refer to phenyl, naphthyl, dialinyl (i.e., 1,2-dihydronaphthyl), tetralinyl (i.e., 1,2,3,4-tetrahydronaphthyl), anthracenyl, or phenanthrenyl, more preferably aryl refers to phenyl or naphthyl, and most preferably aryl refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). Unless defined otherwise, a "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromenyl, xanthenyl, phenoxathiinyl, pyrrolyl (e.g., 2H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolizinyl, isoindolyl, indolyl (e.g., 3H-indolyl), indazolyl, purinyl, isoquinolyl, quinolyl, phthalazinyl, naphthyridinyl, quinoxalinyl, cinnolinyl, pteridinyl, carbazolyl, beta-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, furazanyl, phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzoxazolyl, benzisoxazolyl, benzimidazolyl, 1H-tetrazolyl, 2H-tetrazolyl, coumarinyl, or chromonyl. "Heteroaryl" preferably refers to thienyl (i.e., thiophenyl), benzo[b]thienyl, furyl (i.e., furanyl), benzofuranyl, isobenzofuranyl, imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolizinyl, isoindolyl, indolyl (e.g., 3H-indolyl), indazolyl, isoquinolyl or quinolyl. A more preferred example is thienyl.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). Unless defined otherwise, "cycloalkyl" preferably refers to a C₃₋₁₄ cycloalkyl, and more preferably refers to a C₃₋₇ cycloalkyl. A particularly preferred "cycloalkyl" is a monocyclic saturated hydrocarbon ring having 3 to 7 ring members. "Cycloalkyl" may, e.g., refer to cyclopropyl, cyclobutyl, cyclopentyl, cyclohexyl, cycloheptyl, or adamantyl.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group).Unless defined otherwise, "heterocycloalkyl" preferably refers to a 3 to 14 membered saturated ring, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized. More preferably, "heterocycloalkyl" refers to a 5 to 7 membered saturated monocyclic ring containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized. "Heterocycloalkyl" may, e.g., refer to oxetanyl, tetrahydrofuranyl, piperidinyl, piperazinyl, aziridinyl, azetidinyl, pyrrolidinyl, imidazolidinyl, morpholinyl (e.g., morpholin-4-yl), pyrazolidinyl, tetrahydrothienyl, octahydroquinolinyl, octahydroisoquinolinyl, oxazolidinyl, isoxazolidinyl, azepanyl, diazepanyl, oxazepanyl or 2-oxa-5-aza-bicyclo[2.2.1]hept-5-yl. "Heterocycloalkyl" preferably refers to oxetanyl, tetrahydrofuranyl, piperidinyl, piperazinyl, aziridinyl, azetidinyl, pyrrolidinyl, imidazolidinyl, morpholinyl (e.g., morpholin-4-yl), pyrazolidinyl, tetrahydrothienyl, oxazolidinyl or isoxazolidinyl. "Heterocycloalkyl" more preferably refers to piperidinyl, piperazinyl, pyrrolidinyl, imidazolidinyl, morpholinyl (e.g., morpholin-4-yl), pyrazolidinyl or tetrahydrothienyl.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. Unless defined otherwise, "cycloalkenyl" preferably refers to a C₃₋₁₄ cycloalkenyl, and more preferably refers to a C₃₋₇ cycloalkenyl. A particularly preferred "cycloalkenyl" is a monocyclic unsaturated alicyclic hydrocarbon ring having 3 to 7 ring atoms and containing one or more (e.g., one or two; preferably one) carbon-to-carbon double bonds. "Cycloalkenyl" may, e.g., refer to cyclopropenyl, cyclobutenyl, cyclopentenyl, cyclohexenyl, cyclohexadienyl, cycloheptenyl, or cycloheptadienyl.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms and carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. Unless defined otherwise, "heterocycloalkenyl" preferably refers to a 3 to 14 membered unsaturated alicyclic ring, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-Cl), bromo (-Br), or iodo (-I).

As used herein, the term "oxo" refers to "=O", i.e. a double bond to an oxygen. In other words, it is a divalent substituent. Thus, if, for example, a methylene group is substituted with "oxo", it corresponds to a carbonyl group (>C=O).

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted. Unless defined otherwise, an optional substituent is selected from the lists specified for the one or more optional substituents of the optionally substituted aryl group and the optionally substituted heteroaryl group.

A skilled person will appreciate that the substituent groups comprised in the compounds of formula (I) and/or formula (II) may be attached to the remainder of the respective compound via a number of different positions of the corresponding specific substituent group. Unless defined otherwise, the preferred attachment positions for the various specific substituent groups are as illustrated in the examples.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

A "solvate" refers to an association or complex of one or more solvent molecules and the compound of formula (I) and/or formula (II). Examples of solvents that form solvates include, but are not limited to, water, isopropanol, ethanol, methanol, dimethyl sulfoxide (DMSO), ethyl acetate, acetic acid, acetonitrile, and ethanolamine. The term "hydrate" refers to the complex where the solvent molecule is water. It is to be understood that such solvates of the compounds of formula (I) and/or formula (II) also include solvates of pharmaceutically acceptable salts of the compounds of formula (I) and/or formula (II).

A "cocrystal" refers to a crystalline structure that contains at least two different compounds that are solid in their pure form under ambient conditions. Cocrystals are made from neutral molecular species, and all species remain neutral after crystallization; further, typically and preferably, they are crystalline homogeneous phase materials where two or more building compounds are present in a defined stoichiometric ratio. See Wang Y and Chen A, 2013; and Springuel GR, et al., 2012; and US Patent 6,570,036.

As used herein, any reference to a compound of formula (I) is to be understood as equally well applying to any specific examples of a compound of formula (I), such as compounds of formula (I-a).

As used herein, any reference to a compound of formula (II) is to be understood as equally well applying to any specific examples of a compound of formula (II), such as compounds of formula (II-a).

The "treatment" of a disorder or disease, in particular pain, may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. It is to be understood that a subject/patient may experience a broad range of responses to a treatment.

The term "amelioration" of a disorder or disease typically refers to the partial response of the subject/patient suffering from the disorder or disease.

The term "prevention" (or "prophylaxis") of a disorder or disease as used herein is also well known in the art. For example, a patient/subject suspected of being prone to suffer from a disorder or disease may particularly benefit from a prevention of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of the compounds of the present invention before any clinical and/or pathological symptoms are diagnosed or determined or can be diagnosed or determined by the attending physician.

The disease or condition to be treated or prevented by the compounds of the present invention is preferably selected from pain, opioid overdose, addiction, neuropsychiatric disorder, Prader-Willi Syndrome, gastrointestinal disorder, skin disorder, dyspnea, and temporomandibular joint dysfunction.

More specifically, the disease or condition includes various diseases and conditions such as
a) pain:
   The pain can be due to various causes such as
   i) pain associated with pulmonary edema, kidney stones, vaginitis, candidiasis, arthralgia (including osteoarthrosis such as lumbar spondylarthrosis), vascular diseases (including intermittent claudication, Raynaud's disease, peripheral vascular disease), headaches (including migraine headaches, sinus headaches, tension headaches), dental pain (including gingivitis), periarteritis nodosa, thyroiditis (in particular subacute thyroiditis), aplastic anemia, Hodgkin's disease, sclerodoma, rheumatic fever, type I diabetes (including diabetic peripheral neuropathy and diabetic amyotrophy), type II diabetes (including diabetic peripheral neuropathy and diabetic amyotrophy), carpal-tunnel syndrome, myasthenia gravis, multiple sclerosis, sarcoidosis, nephrotic syndrome, Behcet's syndrome, polymyositis, hypersensitivity (e.g. towards a substance or a stimulus), and/or myocardial ischemia, ,
   ii) pain associated with invasive procedures (e.g., lumbar puncture, biopsy, surgical intervention) including pain due to swelling occurring after injury and/or pain due to healing,
   iii) pain associated with mechanical and/or metabolic injury to the nervous system, neuropathic pain, and/or tumor infiltration of nerves and/or nerve roots,
   iv) pain associated with mechanical injury (e.g., minor injuries) including pain due to swelling occurring after injury and/or pain due to healing,
   vi) fibromyalgia,
   vii) cancer pain, including pain due to exposure to chemotherapeutic agents and/or radiation therapy,
b) opioid overdoses such as poisoning due to opioids such as fentanyl, heroin, codeine, morphine and/or oxycodone,
c) addictions such as opioid addiction (such as addiction to fentanyl, heroin, codeine, morphine and/or oxycodone), nicotine addiction, pathological gambling, alcohol addiction, and/or sex addiction,
d) neuropsychiatric disorders such as compulsive behavior (e.g. obsessive compulsive disorder, trichotillomania, skin picking and/or compulsive behavior associated with neurodegenerative disorders such as Huntington's disease and/or Parkinson's disease), impulsive behavior (e.g., Tourette's syndrome, and/or kleptomania, and/or impulsive behavior associated with neurodegenerative disorders such as frontotemporal dementia and/or Alzheimer's disorder), depression (e.g., depressive disorders, anxiety disorder, panic disorder, dysphoria, and/or anhedonia), eating disorders (e.g. anorexia nervosa, bulimia nervosa, binge eating disorder, and/or obesity), sleep disorders (e.g. sleep disruption, in particular sleep disruption associated with a neurodegenerative disorder such as supranuclear palsy), epilepsy, schizophrenia, psychosis, bipolar disorder and/or autism spectrum disorder,
e) Prader-Willi Syndrome,
f) gastrointestinal disorders in particular impaired gastrointestinal motility such as constipation and/or diarrhea, or bowel disorders such as irritable bowel syndrome, inflammatory bowel disease, and/or Crohn's disease,
g) skin disorders such as itching and/or urticaria,
h) dyspnea,
j) temporomandibular joint dysfunction.

More preferably, the disease or condition includes various diseases and conditions such as
a) pain:
   The pain can be due to various causes such as
   i) pain associated with pulmonary edema, kidney stones, vaginitis, candidiasis, arthralgia (including osteoarthrosis such as lumbar spondylarthrosis), vascular disease (including intermittent claudication, Raynaud's disease, peripheral vascular disease), headaches (including migraine headaches, sinus headaches, tension headaches), dental pain (including gingivitis), periarteritis nodosa, Hodgkin's disease, rheumatic fever, type I diabetes (including diabetic peripheral neuropathy and diabetic amyotrophy), type II diabetes (including diabetic peripheral neuropathy and diabetic amyotrophy), multiple sclerosis, sarcoidosis, Behcet's syndrome, polymyositis, hypersensitivity (e.g. towards a substance or a stimulus), and/or myocardial ischemia,
   ii) pain associated with invasive procedures (e.g., lumbar puncture, biopsy, surgical intervention) including pain due to swelling occurring after injury and/or pain due to healing,
   iii) pain associated with mechanical and/or metabolic injury to the nervous system, neuropathic pain, and/or tumor infiltration of nerves and/or nerve roots,
   iv) pain associated with mechanical injury (e.g., minor injuries) including pain due to swelling occurring after injury and/or pain due to healing,
   v) fibromyalgia,
   vi) cancer pain, including pain due to exposure to chemotherapeutic agents and/or radiation therapy,
b) opioid overdoses such as poisoning due to opioids such as fentanyl, heroin, codeine, morphine and/or oxycodone,
c) addictions such as opioid addiction (such as addiction to fentanyl, heroin, codeine, morphine and/or oxycodone), nicotine addiction, pathological gambling, alcohol addiction, and/or sex addiction,
d) neuropsychiatric disorders such as compulsive behavior (e.g. obsessive compulsive disorder, trichotillomania, skin picking and/or compulsive behavior associated with neurodegenerative disorders such as Huntington's disease and/or Parkinson's disease), impulsive behavior (e.g., Tourette syndrome, and/or kleptomania, and/or impulsive behavior associated with neurodegenerative disorders such as frontotemporal dementia and/or Alzheimer's disorder), depression (e.g., depressive disorders, anxiety disorder, panic disorder, dysphoria, and/or anhedonia), eating disorders (e.g. anorexia nervosa, bulimia nervosa, binge eating disorder, and/or obesity), sleep disorders (e.g. sleep disruption, in particular sleep disruption associated with a neurodegenerative disorder such as supranuclear palsy), epilepsy, schizophrenia, psychosis, bipolar disorder and/or autism spectrum disorder,
e) gastrointestinal disorders such as constipation, diarrhea, irritable bowel syndrome, inflammatory bowel disease, and/or Crohn's disease,
f) skin disorders such as itching and/or urticaria,
g) dyspnea,
h) temporomandibular joint dysfunction.

Even more preferably, the disease or condition includes various diseases and conditions such as
a) pain:
   The pain can be due to various causes such as
   i) pain associated with pulmonary edema, kidney stones, vaginitis, candidiasis, arthralgia (including osteoarthrosis such as lumbar spondylarthrosis), vascular disease (including intermittent claudication, Raynaud's disease, peripheral vascular disease), headaches (including migraine headaches, sinus headaches, tension headaches), dental pain (including gingivitis), periarteritis nodosa, Hodgkin's disease, rheumatic fever, type I diabetes (including diabetic peripheral neuropathy and diabetic amyotrophy), type II diabetes (including diabetic peripheral neuropathy and diabetic amyotrophy), multiple sclerosis, sarcoidosis, Behcet's syndrome, polymyositis, hypersensitivity (e.g. towards a substance or a stimulus), and/or myocardial ischemia,
   ii) pain associated with invasive procedures (e.g., lumbar puncture, biopsy, surgical intervention) including pain due to swelling occurring after injury and/or pain due to healing,
   iii) pain associated with mechanical and/or metabolic injury to the nervous system, neuropathic pain, and/or tumor infiltration of nerves and/or nerve roots,
   iv) pain associated with mechanical injury (e.g., minor injuries) including pain due to swelling occurring after injury and/or pain due to healing,
   v) fibromyalgia,
   vi) cancer pain, including pain due to exposure to chemotherapeutic agents and/or radiation therapy,
c) addictions such as opioid addiction (such as addiction to fentanyl, heroin, codeine, morphine and/or oxycodone), nicotine addiction, pathological gambling, alcohol addiction, and/or sex addiction,
d) neuropsychiatric disorders such as compulsive behavior (e.g. obsessive compulsive disorder, trichotillomania, skin picking and/or compulsive behavior associated with neurodegenerative disorders such as Huntington's disease and/or Parkinson's disease), impulsive behavior (e.g., Tourette syndrome, and/or kleptomania, and/or impulsive behavior associated with neurodegenerative disorders such as frontotemporal dementia and/or Alzheimer's disorder), depression (e.g., depressive disorders, anxiety disorder, panic disorder, dysphoria, and/or anhedonia), eating disorders (e.g. anorexia nervosa, bulimia nervosa, binge eating disorder, and/or obesity), sleep disorders (e.g. sleep disruption, in particular sleep disruption associated with a neurodegenerative disorder such as supranuclear palsy), epilepsy, schizophrenia, psychosis, bipolar disorder and/or autism spectrum disorder,
e) gastrointestinal disorders such as constipation, diarrhea, irritable bowel syndrome, inflammatory bowel disease, and/or Crohn's disease,
f) skin disorders such as itching and/or urticaria.

It is to be understood that the neuropsychiatric disorders are preferably selected from obsessive compulsive disorder, Huntington's disease, Parkinson's disease, Tourette's syndrome, kleptomania, frontotemporal dementia, Alzheimer's disorder, depression including depressive disorders, anxiety disorder and panic disorder, anorexia nervosa, bulimia nervosa, binge eating disorder, obesity, sleep disruption associated with supranuclear palsy, epilepsy, schizophrenia, psychosis, bipolar disorder and autism spectrum disorder.

Still more preferably the disease or condition to be treated or prevented by the compounds of the present invention is pain. Thus, the disease or condition includes various diseases and conditions related to pain, which can be due to various causes such as
i) pain associated with pulmonary edema, kidney stones, vaginitis, candidiasis, arthralgia (including osteoarthrosis such as lumbar spondylarthrosis), vascular disease (including intermittent claudication, Raynaud's disease, peripheral vascular disease), headaches (including migraine headaches, sinus headaches, tension headaches), dental pain (including gingivitis), periarteritis nodosa, Hodgkin's disease, rheumatic fever, type I diabetes (including diabetic peripheral neuropathy and diabetic amyotrophy), type II diabetes (including diabetic peripheral neuropathy and diabetic amyotrophy), multiple sclerosis, sarcoidosis, Behcet's syndrome, polymyositis, hypersensitivity (e.g. towards a substance or a stimulus), and/or myocardial ischemia,
ii) pain associated with invasive procedures (e.g., lumbar puncture, biopsy, surgical intervention) including pain due to swelling occurring after injury and/or pain due to healing,
iii) pain associated with mechanical and/or metabolic injury to the nervous system, neuropathic pain, and/or tumor infiltration of nerves and/or nerve roots,
iv) pain associated with mechanical injury (e.g., minor injuries) including pain due to swelling occurring after injury and/or pain due to healing,
v) fibromyalgia,
vi) cancer pain, including pain due to exposure to chemotherapeutic agents and/or radiation therapy.

Regarding point a): The above examples of pain which can be treated include a number of diseases in which pain is commonly experienced by the subject in addition to the other effects of the disease. The duration of pain in each of these cases may be acute and/or chronic. The type of pain may be nociceptive, inflammatory, neuropathic, dysfunctional. In the present invention, this pain is to be treated by opioid receptor modulators. All of the conditions described here are known to be associated with pain, even though the subject suffering from these diseases may not always suffer from pain. However, for all of them there is evidence that pain may be a common or an occasional symptom. A painful symptom of diabetes can, e.g., be diabetic peripheral neuropathy or diabetic amyotrophy. These conditions may occur in both type I and II of diabetes as described in Diabetes Care 2005 Feb; 28(2): 485-487. Hypersensitivity typically refers to increased sensation or pain due to a certain stimulus. Hypersensitivity may be caused and/or treated by opioids (Anesthesiology 7 2019, Vol. 131, pages 132-147 and The Journal of Allergy and Clinical Immunology: In Practice; Vol. 5(6), 2017, pages 1601-1606 and Curr Pain Headache Rep. 2011 Apr; 15(2): 129-136.).
Regarding point b): Opioid overdose can be treated by opioid antagonists, which reverse the effects of opioid agonists. Novel antagonists for the G protein and β-arrestin-2 pathway are presented herein (see table for functional tests on G protein mediated signaling and β-arrestin-2 recruitment at the µOR).
Regarding point c): Opioid addiction, also termed opioid use disorder, can be treated by opioid substitution therapy. For the substitution partial agonists and antagonists for the G protein pathway are of interest. Benefits may arise from low intrinsic activity for β-arrestin-2 recruitment. Novel substances with such properties are presented herein (see table for functional tests on G protein mediated signaling and β-arrestin-2 recruitment at the µOR). In nicotine addiction, signaling of the opioid system is altered. Restoring normal opioid signaling may be a possible way to treat nicotine addiction or opioids may alleviate the withdrawal symptoms of nicotine addiction, as described in Arch Gen Psychiatry. 1999; 56(7), pages 663-668. Opioids have been shown to exhibit positive effects on behavioral addictions such as kleptomania and pathological gambling as reported in Expert Opin Pharmacother. 2016; 17(6):835-44. The opioid antagonist naltrexone is approved for the treatment of alcohol addiction by the FDA. Opioid antagonists may be useful as opioid antidotes and may therefore be suitable for the treatment of addictions such as opioid addiction.
Regarding point d): Neuropsychiatric disorders may be treated by opioid agonists, partial agonists and/or antagonists as clinical studies suggest. References for clinical studies that support the use of opioids in the treatment of neuropsychiatric disorders include the following:
   Opioids have been successfully used in the treatment of obsessive compulsive disorder as described in J Clin Psychiatry. 2005 Mar;66(3):353-9. It is furthermore known that δOR serves an antiparkinsonism and neuroprotective role in the brain. Example 1 described herein bound to this receptor with high affinity (see table "Radioligand binding experiments"). (CNS Neurosci Ther. 2018; 24: 1089-1099). Positive effects of opoids in patients suffering from Tourette's syndrome have been described in Lancet. 1994 Apr 30;343(8905):1107-8. Opioids showed positive effects on behavioral addictions such as kleptomania and gambling disorder as reported in Expert Opin Pharmacother. 2016;17(6):835-44. Amelioration of clinical symptoms in patients with advanced dementia by opoids are described in Int J Geriatr Psychiatry. 2003 Aug;18(8):700-5. The opioid receptor system is involved in the pathophysiology of Alzheimer's disease. Opioid treatment may be beneficial in ameliorating the clinical symptoms of the disorder as reported in J Neurosci Res. 2010 Nov 15;88(15):3215-21). Opioids showed improvement in patients with treatment resistant depression in clinical studies (J Clin Psychiatry. 2014 Aug;75(8):e785-93). Opioids have shown anti-depressant and anti-anxiety effects in clinical studies (Int J Neuropsychopharmacol. 2018 Feb 1;21(2): 120-127). Opioids furthermore showed panicolytic-like effects as reported in Neuroscience. 2017 Jun 23;354:178-195. The opioid system is known to be involved in feeding mechanisms and therefore implicated in anorexia nervosa and bulimia nervosa as reported in Med Hypotheses. 1995 Nov;45(5):491-7. Patients with eating disorder or morbid obesity have an impaired level of µOR availability in the brain as described in Psychiatry Res Neuroimaging. 2018 Jun 30;276:41-45. Opioids may be useful agents to restore normal µOR signaling. Opioids may furthermore be beneficial in ameliorating clinical symptoms of supranuclear palsy, schizophrenia and psychosis as described in Pharmac. Ther. Vol. 46, pp. 395-428, 1990. Opioid treatment can ameliorate the clinical symptoms of epilepsy, in particular seizures. (J Neurosci Res. 2010 Nov 15;88(15):3215-21). In the same publication, it was reported that opioid receptor system is involved in the pathophysiology of autism. Opioid treatment may therefore be beneficial in ameliorating the clinical symptoms of the disorder. In addition, it has been reported that opioids ameliorated the symptoms of patients with bipolar disorder (J Neuropsychiatry Clin Neurosci. Fall 2007;19(4):449-52).
Regarding point e): Constipation is a well-known side effect of opioid agonists that could be treated with novel opioid agonists with lower side effects or peripheral antagonists. Diarrhea can be treated by opioid agonists, preferably peripherally acting, via the otherwise undesired side effect of opioid agonists.
Regarding point f): Urticaria may be linked to mast-cell degranulation by opioids. Opioids can cause an itching sensation. Novel opioids may reduce the risk for itching.
Regarding point g): A common side effect of opioid agonists is respiratory depression. This side effect can be reversed by opioid antagonists, such as the G protein pathway antagonists described herein.

Terms such as "patient" or "subject in need thereof" refers to a living organism suffering from or prone to a disease or condition that can be treated by administration of a pharmaceutical composition as provided herein. Non-limiting examples include humans, other mammals, bovines, rats, mice, dogs, monkeys, goat, sheep, cows, deer, and other non-mammalian animals. The patient is preferably a human.

An "effective amount" is an amount sufficient for a compound to accomplish a stated purpose relative to the absence of the compound (e.g., achieve the effect for which it is administered, i.e. the reduction of pain). An example of an "effective amount" is an amount sufficient to contribute to the treatment, prevention, or reduction of pain, which could also be referred to as a "therapeutically effective amount".

The term "about" preferably refers to ±10% of the indicated numerical value, more preferably to ±5% of the indicated numerical value, and in particular to the exact numerical value indicated. For example, the expression "about 100" preferably refers to 100 ±10% (i.e., 90 to 110), more preferably to 100 ±5% (i.e., 95 to 105), and even more preferably to the specific value of 100. In connection with percentages, it is evident that 100 % can not be exceeded. If the term "about" is used in connection with the endpoints of a range, it preferably refers to the range from the lower endpoint 10% of its indicated numerical value to the upper endpoint +10% of its indicated numerical value, more preferably to the range from of the lower endpoint 5% to the upper endpoint +5%, and even more preferably to the range defined by the exact numerical values of the lower endpoint and the upper endpoint. Thus, the expression "about 10 to about 20" preferably refers to the range of 9 to 22, more preferably to the range of 9.5 to 21, and even more preferably to the range of 10 to 20. If the term "about" is used in connection with the endpoint of an open-ended range, it preferably refers to the corresponding range starting from the lower endpoint 10% or from the upper endpoint +10%, more preferably to the range starting from the lower endpoint 5% or from the upper endpoint +5%, and even more preferably to the open-ended range defined by the exact numerical value of the corresponding endpoint. For example, the expression "at least about 10%" preferably refers to at least 9%, more preferably to at least 9.5%, and even more preferably to at least 10%.

The term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

Any parameters referred to herein (including, e.g., any amounts/concentrations indicated in "mg/ml" or in "% (v/v)", and any pH values) are preferably to be determined at standard ambient temperature and pressure conditions, particularly at a temperature of 25°C (298.15 K) and at an absolute pressure of 1 atm (101.325 kPa).

It is to be understood that the present invention specifically relates to each and every combination of features and embodiments described herein, including any combination of general and/or preferred features/embodiments.

In this specification, a number of documents including patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### Pharmaceutically acceptable salts etc.

The scope of the invention embraces all pharmaceutically acceptable salt forms of the compounds of formula (I) and/or formula (II) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compounds of formula (I) and/or formula (II) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) and/or formula (II) is a hydrochloride salt. Accordingly, it is preferred that the compound of formula (I) and/or formula (II), including any one of the specific compounds of formula (I) and/or formula (II) described herein, is in the form of a hydrochloride salt, a hydrobromide salt, a mesylate salt, a sulfate salt, a tartrate salt, a fumarate salt, an acetate salt, a citrate salt, or a phosphate salt, and it is particularly preferred that the compound of formula (I) and/or formula (II) is in the form of a hydrochloride salt.

Furthermore, the compounds of formula (I) and/or formula (II) may exist in the form of different isomers, in particular stereoisomers (including, e.g., geometric isomers (or cis/trans isomers), enantiomers and diastereomers) or tautomers. All such isomers of the compounds of formula (I) and/or formula (II) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds provided herein.

The scope of the invention also embraces compounds of formula (I) and/or formula (II), in which one or more atoms are replaced by a specific isotope of the corresponding atom. For example, the invention encompasses compounds of formula (I) and/or formula (II), in which one or more hydrogen atoms (or, e.g., all hydrogen atoms) are replaced by deuterium atoms (i.e., ²H; also referred to as "D"). Accordingly, the invention also embraces compounds of formula (I) and/or formula (II) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) and/or formula (II) can be increased using deuteration techniques known in the art. For example, a compound of formula (I) and/or formula (II) or a reactant or precursor to be used in the synthesis of the compound of formula (I) and/or formula (II) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) and/or formula (II) is not enriched in deuterium. Accordingly, the presence of naturally occurring hydrogen atoms or ¹H hydrogen atoms in the compounds of formula (I) and/or formula (II) is preferred.

The present invention also embraces compounds of formula (I) and/or formula (II), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers or imaging probes in positron emission tomography (PET). The invention thus includes (i) compounds of formula (I) and/or formula (II), in which one or more fluorine atoms are replaced by ¹⁸F atoms, (ii) compounds of formula (I) and/or formula (II), in which one or more carbon atoms are replaced by ¹¹C atoms, (iii) compounds of formula (I) and/or formula (II), in which one or more nitrogen atoms are replaced by ¹³N atoms, (iv) compounds of formula (I) and/or formula (II), in which one or more oxygen atoms are replaced by ¹⁵O atoms, (v) compounds of formula (I) and/or formula (II), in which one or more bromine atoms are replaced by ⁷⁶Br atoms, (vi) compounds of formula (I) and/or formula (II), in which one or more bromine atoms are replaced by ⁷⁷Br atoms, (vii) compounds of formula (I) and/or formula (II), in which one or more iodine atoms are replaced by ¹²⁰I atoms, and (viii) compounds of formula (I) and/or formula (II), in which one or more iodine atoms are replaced by ¹²⁴I atoms.

### Formulation, dosage forms etc.

As detailed above, the compounds provided herein may be administered as compounds per se or may be formulated as medicaments, e.g. as pharmaceutical composition or combination. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers, or any combination thereof.

In particular, the pharmaceutical compositions may comprise one or more solubility enhancers, such as, e.g., poly(ethylene glycol), including poly(ethylene glycol) having a molecular weight in the range of about 200 to about 5,000 Da, ethylene glycol, propylene glycol, non-ionic surfactants, tyloxapol, polysorbate 80, macrogol-15-hydroxystearate, phospholipids, lecithin, dimyristoyl phosphatidylcholine, dipalmitoyl phosphatidylcholine, distearoyl phosphatidylcholine, cyclodextrins, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, carboxyalkyl thioethers, hydroxypropyl methylcellulose, hydroxypropylcellulose, polyvinylpyrrolidone, vinyl acetate copolymers, vinyl pyrrolidone, sodium lauryl sulfate, dioctyl sodium sulfosuccinate, or any combination thereof.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

The pharmaceutical compositions can be formulated by techniques known to the person skilled in the art, such as the techniques published in "Remington: The Science and Practice of Pharmacy", Pharmaceutical Press, 22nd edition. The pharmaceutical compositions can be formulated as dosage forms for oral, peroral, parenteral, such as intramuscular, intravenous, subcutaneous, intradermal, intraarterial, intracardial, intrathecal, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I) and/or formula (II) or the above described pharmaceutical compositions comprising a compound of formula (I) and/or formula (II) may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, and vaginal.

If said compounds or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

Alternatively, said compounds or pharmaceutical compositions can be administered in the form of a suppository or pessary, or it may be applied topically in the form of a gel, hydrogel, lotion, solution, cream, ointment or dusting powder. The compounds of the present invention may also be dermally or transdermally administered, for example, by the use of a skin patch.

Said compounds or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides (see, e.g., US 3,773,919), copolymers of L-glutamic acid and gamma-ethyl-L-glutamate (Sidman, U. et al., Biopolymers 22:547-556 (1983)), poly(2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP133988). Sustained-release pharmaceutical compositions also include liposomally entrapped compounds. Liposomes containing a compound of the present invention can be prepared by methods known in the art, such as, e.g., the methods described in any one of: DE3218121; Epstein et al., Proc. Natl. Acad. Sci. (USA) 82:3688-3692 (1985); Hwang et al., Proc. Natl. Acad. Sci. (USA) 77:4030-4034 (1980); EP0052322; EP0036676; EP088046; EP0143949; EP0142641; JP 83-118008; US 4,485,045; US 4,544,545; and EP0102324.

Said compounds or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the compounds of formula (I) and/or formula (II) for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to the emulsification/spray drying process disclosed in WO 99/16419 or WO 01/85136. Spray drying of solution formulations of the compounds of the present invention can be carried out, e.g., as described generally in the "Spray Drying Handbook", 5th ed., K. Masters, John Wiley & Sons, Inc., NY (1991), and in WO 97/41833 or WO 03/053411.

For topical application to the skin, said compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

The present invention thus relates to the compounds or the pharmaceutical compositions provided herein, wherein the corresponding compound or pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route. Particularly preferred routes of administration of the compounds or pharmaceutical compositions of the present invention are oral forms, preferably peroral forms, of administration.

Typically, a physician will determine the dosage which will be most suitable for an individual subject. The specific dose level and frequency of dosage for any particular individual subject may be varied and will depend upon a variety of factors including the age, body weight, general health, sex, diet, mode and time of administration, rate of excretion, drug combination, the severity of the particular condition, and the individual subject undergoing therapy.

A proposed, yet non-limiting dose of the compounds according to the invention for administration to a human (of approximately 70 kg body weight) may be 0.05 to 2000 mg, preferably 0.1 mg to 1000 mg, of the active ingredient per unit dose. The unit dose may be administered, e.g., 1, 2, 3 or more times per day. The unit dose may also be administered 1 to 7 times per week, e.g., with one, two or more administration(s) per day. It will be appreciated that it may be necessary to make routine variations to the dosage depending on the age and weight of the patient/subject as well as the severity of the condition to be treated. The precise dose and also the route of administration will ultimately be at the discretion of the attendant physician.

The compounds of formula (I) and/or formula (II) can be used in combination with other therapeutic agents, including in particular other anti-pain agents. When a compound of the invention is used in combination with a second therapeutic agent active against the same disease, the dose of each compound may differ from that when the compound is used alone. The combination of a compound of the present invention with a second therapeutic agent may comprise the administration of the second therapeutic agent simultaneously/concomitantly or sequentially/separately with the compound of the invention.

The following examples describe the invention further, without limiting it to the content of the examples.

### Examples

### A Experimental

### A-1 General information

### Solvents and chemicals:

The compounds described herein can be synthesized from commercially available precursors. Chemicals and solvents for synthesis were purchased from different manufacturers: Sigma Aldrich (via Merck, Darmstadt, Germany), Alfa Aesar (Rien, Germany), TCI (Eschborn, Germany), ACROS (via supplier Fisher Scientific, Niedderau, Germany or VWR International, Darmstadt, Germany). For preparative and analytical HPLC experiments, special HPLC-grade solvents were purchased from the above mentioned manufacturers.

### Preparative HPLC:

For HPLC purifications different solvent systems and methods were used on an AGILENT Series 1100 machine. Detailed conditions can be found in the compounds section. For separation, a Zorbax Eclipse XDB-C8 (21.2 mm x 150 mm, 5 µm) column was used.

### Microwave synthesis:

Microwave assisted reactions were carried out in sealed microwave vials in a BIOTAGE Initiator machine.

### Polarimetry:

Specific optical rotation measurement was performed in a JASCO P-2000 polarimeter in solution. Path length: 100 mm, volume: 1.2 mL.

### UHPLC-MS:

LC-MS analysis was carried out using a Dionex UltiMate 3000 UHPLC system with a RS diode array detector for detection at λ = 220 nm and 254 nm. A binary solvent gradient of either MeOH/H₂O or acetonitrile / H₂O was applied on a Kinetex 2.6 u C8 100A (75 mm x 2.1 mm, 2.6 µm) column or ZORBAX ECLIPSE XDB-C8 (3.0 x 100 mm, 3.5 µm, 0.4 mL/min flow). Masses were detected by electron spray ionization (ESI) in a Bruker Amazon SL mass spectrometer.

### High resolution mass spectrometry:

High resolution masses were measured on an AB Sciex Triple TOF660 SCiex or a Bruker maXix UHR-TOF with electron spray ionization.

### NMR spectra measurement:

NMR spectra were obtained on either a Bruker Avance 360 or Avance 600 and TMS was used for chemical shift referencing in the solvents indicated.

### Analytical HPLC:

HPLC analyses were performed on an Agilent 1200 series HPLC system with a DAD detector. Samples were separated on an Agilent Zorbax Eclipse XDB-C8 (4.6 mm x 150 mm, 5 µM) column. Different binary gradient solvent systems were applied.

| | |
|---|---|
| System 1: | eluent: MeOH/H₂O + 0.1 % HCOOH, flow: 0.5 mL/min. |
| | gradient-5 % MeOH for 3 min, 5 % to 95 % MeOH in 15 min, 95 % MeOH for 6 min, 95 % to 5 % MeOH in 3 min, 5 % MeOH for 3 min. |
| System 2: | eluent: ACN/H₂O + 0.1 % HCOOH, flow: 0.5 mL/min. |
| | gradient: 5 % ACN for 3 min, 5 % to 95 % ACN in 15 min, 95 % ACN for 6 min, 95 % to 5 % ACN in 3 min, 5 % ACN for 3 min. |
| System 3: | eluent: MeOH/H₂O + 0.3 % TFA, flow: 0.5 mL/min. |
| | gradient: 5 % MeOH for 3 min, 5 % to 95 % MeOH in 15 min, 95 % MeOH for 6 min, 95 % to 5 % MeOH in 3 min, 5 % MeOH for 3 min. |
| System 4: | eluent: ACN/H₂O + 0.3 % TFA, flow: 0.5 mL/min. |
| | gradient: 5 % ACN for 3 min, 5 % to 95 % ACN in 15 min, 95 % ACN for 6 min, 95 % to 5 % ACN in 3 min, 5 % ACN for 3 min. |

### Analytical chiral HPLC:

Chiral HPLC analyses were performed on an Agilent 1200 series HPLC system with a DAD detector. Samples were separated on a Daicel Chiralpack IC (IC00CE-SK041, 4.6 mmx 250 mm, 5 µm) column. Different binary isocratic solvent systems were applied:

| System | Hexane [vol. %] | Isopropanol + 0.1 % ethylenediamine [vol. %] | Ethanol + 0.1 % ethylenediamine [vol. %] | Flow [mL/min] | Temperature [°C] |
|---|---|---|---|---|---|
| A | 70 | 30 | 0 | 1 | R.t. |
| B | 80 | 20 | 0 | 1 | R.t. |
| C | 90 | 0 | 10 | 1 | R.t. |
| D | 95 | 0 | 5 | 1 | R.t. |
| E | 85 | 15 | 0 | 1 | R.t. |
| F | 85 | 15 | 0 | 1 | 20 |
| G | 90 | 10 | 0 | 1 | R.t. |
| H | 40 | 60 | 0 | 1 | 20 |
| I | 70 | 30 | 0 | 1 | 20 |
| J | 60 | 40 | 0 | 1 | 20 |
| K | 80 | 20 | 0 | 1 | 20 |
| L | 30 | 70 | 0 | 1 | 20 |
| M | 40 | 60 | 0 | 1 | 20 |
| N | 97.5 | 2.5 | 0 | 1 | 20 |

The following common abbreviations are used herein

| | |
|---|---|
| ACN | acetonitrile |
| BOP | (benzotriazol-1-yloxytris(dimethylamino)phosphonium hexafluorophosphate) |
| DAMGO | ((2*S*)-2-[[2-[[(2*R*)-2-[[(2*S*)-2-amino-3-(4-hydroxyphenyl)propanoyl]amino]propanoyl]amino]acetyl]-methylamino]-*N*-(2-hydroxyethyl)-3-phenylpropanamide) |
| DCM | dichloromethane |
| DMF | dimethylformamide |
| MeOD | deuterated methanol |
| MeOH | methanol |
| MTBE | methyl *tert*-butyl ether |
| PyBOP | benzotriazol-1-yl-oxytripyrrolidinophosphonium hexafluorophosphate |
| r.t. | room temperature |
| TEA | triethylamine |
| TFA | trifluoroacetic acid |
| THF | tetrahydrofuran |

### A-2 Syntheses of intermediates:

### Intermediate 1: Methyl (S)-2-amino-3-(4-hydroxyphenyl)-2-methylpropanoate hydrochloride

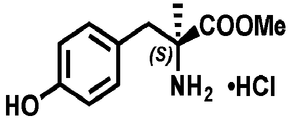

To a suspension of α-methyl-*L*-tyrosine (500 mg, 2.56 mmol, 1 eq.) in 5 mL of dry methanol was added thionyl chloride (0.56 mL, 7.7 mmol, 3 eq.) slowly at 0 °C. Remaining solids dissolved after completion of addition and the resulting solution was stirred for 23 h at reflux. The mixture was evaporated under vacuum and the remaining yellow oil was suspended in 15 mL of MTBE. The white solid was filtered and washed with 10 mL of MTBE to give the product as the hydrochloride salt (630 mg, 2.56 mmol, 100 %). No further purification was necessary.

| | |
|---|---|
| **Melting point** | 153 °C |
| **[α]_{D}²⁵** | + 5.9° (c = 0.738, MeOH) |
| **ESI (m/z)** | 209.94 [M+H]⁺ |
| **¹H NMR** | (400 MHz, DMSO-*d*₆) δ 9.53 (s, 1H), 8.67 (s, 3H), 6.99 - 6.93 (m, 2H), 6.77 - 6.70 (m, 2H), 3.72 (s, 3H), 3.09 - 2.98 (m, 2H), 1.49 (s, 3H). |
| **¹³C NMR** | (101 MHz, DMSO) δ 170.9, 156.8, 130.9, 123.3, 115.3, 60.0, 52.8, 41.7, 21.2. |

### Intermediate 2: Methyl dimethyl-L-tyrosinate

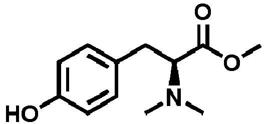

In a sealed microwave tube paraformaldehyde (308 mg, 10.2 mmol, 10 eq.) was refluxed for two hours to prepare a 35 wt. % solution in water. L-Tyrosine methyl ester (200 mg, 1.0 mmol, 1 eq.) was dissolved in a mixture of 5 mL acetonitrile and 1 mL distilled water and cooled to -10 °C in an acetone/ice bath. First, sodium triacetoxyborohydride (1.09 g, 5.12 mmol, 5 eq.), second, the formaldehyde solution was added. The reaction was quenched after 5 min by the addition of 5 mL saturated aqueous sodium bicarbonate solution and sodium carbonate to adjust to pH = 9. The slurry was diluted with 5 mL of dist. water. The aqueous phase was extracted five times with 10 mL of an organic solvent mixture of isopropanol: ethyl acetate 1 : 4. The combined organic phases were dried over sodium sulfate and evaporated to dryness. No further purification was necessary. The product was obtained as a light yellow syrup (228 mg, 1.0 mmol, 100 %).

| | |
|---|---|
| **[α]_{D}²⁶** | + 29.9° (c = 0.952, MeOH) |
| **ESI (m/z)** | 224.02 [M+H]⁺ |
| **¹H NMR** | (400 MHz, Methanol-*d*₄) δ 7.00 - 6.96 (m, 2H), 6.70 - 6.65 (m, 2H), 3.55 (s, 3H), 3.35 (dd, *J* = 9.3, 6.1 Hz, 1H), 2.91 - 2.83 (m, 2H), 2.36 (s, 6H). |
| **¹³C NMR** | (151 MHz, MeOD) δ 173.2, 157.2, 131.1, 129.4, 116.2, 71.4, 51.6, 42.3, 36.1. |

### Intermediate 3: (S)-4-(2-(Dimethylamino)-3-hydroxypropyl)phenol

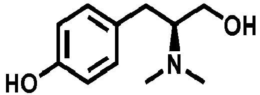

Intermediate 2 (209 mg, 0.94 mmol, 1 eq.) was suspended in 25 mL dry THF. Lithium aluminium hydride (0.7 mL, 2.81 mmol, 3 eq., 4 M solution in THF) was slowly added at 0°C. The reaction was stirred for 16 h at r.t. and quenched by the slow addition of 0.1 mL water at 0°C. Subsequently, 0.19 mL of a 2 N NaOH solution, 0.3 mL water and 0.5 g dry magnesium sulfate were added. The solids were filtered off and washed with 30 mL of a mixture of methanol + 0.1 % ammonia (25 %, aq.). The extracts were combined, dried over magnesium sulfate and evaporated under vacuum to yield a colorless oil. Purification by flash column chromatography (eluent: DCM . MeOH + 0.1 % ammonia (25 % aq.), 95:5 to 4:1) gave the product as a white solid (102 mg, 0.52 mmol, 56 %).

| | |
|---|---|
| **Melting point** | 119 - 121 °C |
| **[α]_{D}²⁶** | + 10.1° (c = 0.608, MeOH) |
| **ESI (m/z)** | 196.00 [M+H]⁺ |
| **¹H NMR** | (400 MHz, Methanol-*d*₄) δ 7.07 (d, *J* = 8.5 Hz, 2H), 6.79 - 6.69 (m, 2H), 3.60 (dd, *J* = 12.1, 4.2 Hz, 1H), 3.55 (dd, *J* = 12.1, 7.2 Hz, 1H), 3.17 - 3.05 (m, 1H), 2.93 (dd, *J* = 13.6, 4.3 Hz, 1H), 2.66 (s, 6H), 2.61 (dd, *J* = 13.6, 10.2 Hz, 1H). |
| **¹³C NMR** | (151 MHz, MeOD) δ 157.3, 131.2, 129.5, 116.5, 69.4, 59.9, 41.1, 31.5. |

### Intermediate 4: (S)-2-Amino-3-(4-hydroxyphenyl)-2-methylpropanamide

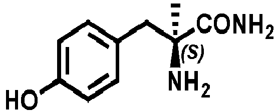

Intermediate 1 (300 mg, 1.22 mmol, 1 eq.) was dissolved in a minimum amount of a 5 % aqueous solution of sodium carbonate. The solution was extracted three times with 10 mL of an isopropanol: ethyl acetate mixture (1:4). The combined organic phases were dried over sodium sulfate and evaporated to dryness. The remaining solid was dissolved in 6 mL of dry methanol. The solution was cooled to -10°C using an acetone/ice bath and gaseous ammonia was bubbled through the solution for five minutes. The reaction was carried out in a sealed pressure tube at 60 °C. Ammonia addition was repeated every 8 to 24 h until the starting material was consumed (TLC control). After 5 d the solvent was evaporated to give the amide product as a light orange solid (230 mg, 1.18 mmol, 97 %). No further purification was necessary.

| | |
|---|---|
| **Melting point** | 279 °C |
| **[α]_{D}²³** | - 11.2° (c = 0.595, MeOH) |
| **ESI (m/z)** | 194.95 [M+H]⁺ |
| **¹H NMR** | (400 MHz, DMSO-*d*₆) δ 7.48 (s, 1H), 7.17 (s, 1H), 6.99 (d, *J* = 7.9 Hz, 2H), 6.67 (d, *J* = 7.9 Hz, 2H), 2.96 (d, *J* = 13.4 Hz, 1H), 2.66 (d, *J* = 13.5 Hz, 1H), 1.25 (s, 3H). |
| **¹³C NMR** | (101 MHz, DMSO) δ 176.4, 156.0, 131.0, 126.2, 114.7, 58.6, 43.8, 25.0. |

### Intermediate 5: (S)-2-(Dimethylamino)-3-(4-hydroxyphenyl)propanamide

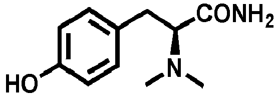

This compound has previously been described in the literature and was synthesized by adapting the respective procedure (Manglik, A., et al., Structure-based discovery of opioid analgesics with reduced side effects. Nature, 2016. 537(7619): p. 185-190). In a sealed microwave tube paraformaldehyde (4.30 g, 143 mmol, 10 eq.) was refluxed for two hours to prepare a 35 wt. % solution in water. L-Tyrosine amide (2.58 g, 14.3 mmol, 1 eq.) was dissolved in a mixture of 60 mL acetonitrile and 12 mL distilled water and cooled to -10 °C in an acetone/ice bath. First, sodium triacetoxyborohydride (15.2 g, 71.5 mmol, 5 eq.), second, the formaldehyde solution was added. The reaction was quenched after 10 min by the addition of 40 mL saturated sodium bicarbonate solution and sodium carbonate to adjust to pH = 9. The slurry was diluted with 40 mL of dist. water. The aqueous phase was extracted five times with 100 mL of an organic solvent mixture of isopropanol: ethyl acetate 1 : 4. The combined organic phases were dried over sodium sulfate and evaporated to dryness. No further purification was necessary. The product was obtained as a white solid (2.98 g, 14.3 mmol, 100 %).

| | |
|---|---|
| **Melting point** | 105 °C |
| **[α]_{D}²⁴** | + 35.6° (c = 0.44, MeOH) |
| **ESI (m/z)** | 208.98 [M+H]⁺ |
| **¹H NMR** | (400 MHz, DMSO-*d*₆) δ 7.15 (d, *J* = 2.6 Hz, 1H), 6.99 - 6.94 (m, 2H), 6.84 (d, *J* = 2.5 Hz, 1H), 6.65 - 6.60 (m, 2H), 4.55 (s, 1H), 3.07 (dd, *J* = 9.1, 5.3 Hz, 1 H)NH₂), 2.80 (dd, *J* = 13.4, 9.1 Hz, 1H), 2.60 (dd, *J* = 13.4, 5.3 Hz, 1H), 2.23 (s, 6H). |
| **¹³C NMR** | (151 MHz, DMSO) δ 171.8, 155.4, 129.7, 129.2, 114.7, 69.0, 41.5, 33.3. |

### Intermediate 6: (S)-4-(3-Amino-2-(dimethylamino)propyl)phenol

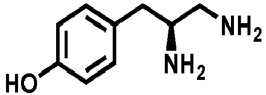

This compound has previously been described in the literature and was synthesized by adapting the respective procedure (Manglik, A., et al., Structure-based discovery of opioid analgesics with reduced side effects. Nature, 2016. 537(7619): p. 185-190). To an ice-cooled solution of Intermediate 5 (3.00 g, 14.4 mmol, 1 eq.) in 60 mL of dry THF, borane-THF complex (86.4 ml, 86.4 mmol, 1M in THF, 6 eq.) was added dropwise at 0°C. After addition, the mixture was refluxed for six hours. The reaction was quenched by the slow addition of 20 mL dry methanol at reflux temperature in order to cleave the borane-amine complex. The mixture was refluxed for additional 30 minutes. The methanol addition-evaporation sequence was repeated twice. The remaining residue was subjected to flash column chromatography (eluent: DCM : MeOH + 0.1 % ammonia (25 % aq.), 10: 1 to 4: 1) which gave the product as a white solid (1.20 g, 6.18 mmol, 43 %).

| | |
|---|---|
| **Melting point** | 179 °C |
| **[α]_{D}²⁵** | + 15.5° (c = 0.50, MeOH) |
| **ESI (m/z)** | 195.00 [M+H]⁺ |
| **¹H NMR** | (400 MHz, DMSO-*d*₆) δ 6.97 - 6.91 (m, 2H), 6.68 - 6.62 (m, 2H), 2.67 (dd, *J* = 13.3, 3.8 Hz, 1H), 2.50 - 2.36 (m, 2H), 2.33 (dd, *J* = 11.4, 3.2 Hz, 1H), 2.23 (s, 6H), 2.16 (dd, *J* = 13.3, 8.7 Hz, 1H). |
| **¹³C NMR** | (151 MHz, DMSO) δ 155.1, 130.5, 129.7, 114.9, 68.2.0, 40.1, 30.3. |

Based on the procedure described literature (Manglik, A., et al., Structure-based discovery of opioid analgesics with reduced side effects. Nature, 2016. 537(7619): p. 185-190) a portion of the pure product was converted to the dihydrochloride salt (Intermediate 6 dihydrochloride) for referencing. The analytical data were identical.

### Intermediate 7: (S)-2-(Dimethylamino)-3-(4-hydroxyphenyl)-2-methylpropanamide

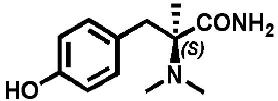

In a sealed microwave tube paraformaldehyde (340 mg, 11.3 mmol, 10 eq.) was refluxed for two hours to prepare a 35 wt. % solution in water. Intermediate 4 (220 mg, 1.13 mmol, 1 eq.) was dissolved in a mixture of 15 mL acetonitrile and 3 mL distilled water and cooled to -10 °C in an acetone/ice bath. First, sodium triacetoxyborohydride (15.2 g, 71.5 mmol, 5 eq.), second, the formaldehyde solution was added. The reaction was quenched after 10 min by the addition of 5 mL saturated sodium bicarbonate solution and sodium carbonate to adjust to pH = 9. The slurry was diluted with 5 mL of dist. water. The aqueous phase was extracted five times with 10 mL of an organic solvent mixture of isopropanol: ethyl acetate 1 : 4. The combined organic phases were dried over sodium sulfate and evaporated to dryness. Purification by flash column chromatography (eluent: DCM : MeOH + 0.1 % ammonia (25 % aq.), 20: 1) gave the product as a white solid (168 mg, 756 µmol, 67 %).

| | |
|---|---|
| **Melting point** | 122 °C |
| **[α]_{D}²³** | + 28.6° (c = 0.359, MeOH) |
| **ESI (m/z)** | 222.98 [M+H]⁺ |
| **¹H NMR** | (400 MHz, DMSO-*d*₆) δ 9.12 (s, 1H), 7.12 (d, *J* = 2.8 Hz, 1H), 7.01 - 6.93 (m, 3H), 6.64 - 6.54 (m, 2H), 2.80 (d, *J* = 13.0 Hz, 1H), 2.66 (d, *J* = 13.0 Hz, 1H), 2.18 (s, 6H), 0.88 (s, 3H). |
| **¹³C NMR** | (101 MHz, DMSO) δ 175.7, 155.5, 131.0, 127.8, 114.3, 66.6, 41.6, 38.9, 12.4. |

### Intermediate 8: (S)-4-(3-Amino-2-(dimethylamino)-2-methylpropyl)phenol

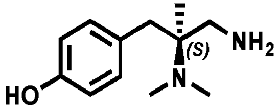

To an ice-cooled solution of Intermediate 7 (103 mg, 463 µmol, 1 eq.) in 7 mL of dry THF, borane-THF complex (2.78 ml, 2.78 mmol, 1M in THF, 6 eq.) was added dropwise at 0°C. After addition, the mixture was refluxed for seven hours. The reaction was quenched by the slow addition of 10 mL dry methanol at reflux temperature in order to cleave the borane-amine complex. The mixture was refluxed overnight. The remaining residue was subjected to flash column chromatography (eluent: DCM : MeOH + 0.1 % ammonia (25 % aq.), 10: 1 to 4: 1) and gave the product as a white solid (58 mg, 0.28 mmol, 70 %).

| | |
|---|---|
| **Melting point** | 129 °C |
| **[α]_{D}²⁵** | + 11.8° (c = 0.446, MeOH) |
| **ESI (m/z)** | 208.95 [M+H]⁺ |
| **¹H NMR** | (400 MHz, DMSO-*d*₆) δ 7.10 - 6.88 (m, 2H), 6.65 - 6.61 (m, 2H), 3.33 (s, 2H), 2.53 - 2.51 (m, 2H), 2.48 - 2.43 (m, 1H), 2.27 - 2.16 (m, 7H), 0.77 (s, 3H). |
| **¹³C NMR** | (101 MHz, DMSO) δ 155.2, 131.2, 128.8, 114.4, 59.1, 46.0, 37.8, 36.8, 17.5. |

### Intermediate 9: (S)-3-(4-Hydroxy-2,6-dimethylphenyl)-2-(tritylamino)propanamide

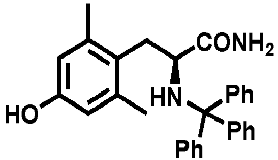

Intermediate 11 (218 mg, 1.05 mmol, 1 eq.), tritylchloride (379 mg, 1.36 mmol, 1.3 eq.), tetrabutylammonium bromide (101 mg, 314 µmol, 0.3 eq.) and triethylamine (190 µL, 1.36 mmol, 1.3 eq.) were dissolved in 1 mL of dry THF under an inert gas atmosphere. The reaction was stirred at 80 °C in a pressure tube for 3 h. The solvent was evaporated and the crude was purified by flash column chromatography (eluent: DCM : MeOH, 99 : 1) to give the product as a white solid (405 mg, 899 µmol, 86 %).

| | |
|---|---|
| **Melting point** | 56 °C |
| **[α]_{D}²⁵** | + 47.3° (c = 0.92, MeOH) |
| **ESI (m/z)** | 473.02 [M+H]⁺ |
| **¹H NMR** | (600 MHz, Chloroform-d) δ 7.20 - 7.14 (m, 15H), 6.55 (s, 2H), 5.38 - 5.32 (m, 1H), 4.98 (s, 1H), 4.43 (s, 1H), 3.44 (ddd, *J* = 9.5, 5.7, 3.7 Hz, 1H), 3.11 - 3.05 (m, 2H), 3.01 (dd, *J* = 14.5, 9.7 Hz, 1H), 2.28 (s, 6H). |
| **¹³C NMR** | (151 MHz, CDCl₃) δ 178.3, 154.2, 145.4, 139.4, 129.1, 127.9, 126.8, 126.3, 115.4, 71.2, 57.7, 34.9, 20.7. |

### Intermediate 10: Methyl (S)-2-Amino-3-(4-hydroxy-2,6-dimethylphenyl)propanoate hydrochoride

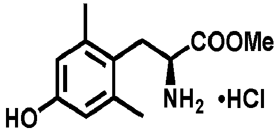

To a suspension of 2,6-dimethyl-L-tyrosine (359 mg, 1.72 mmol, 1 eq.) in 6 mL of dry methanol was added thionyl chloride (0.50 mL, 6.9 mmol, 4 eq.) slowly at 0 °C. Remaining solids dissolved after completion of addition and the resulting solution was stirred for 24 h at reflux. The mixture was evaporated under vacuum and the remaining yellow oil was suspended in 15 mL of MTBE. The white solid was filtered and washed with 10 mL of MTBE to give product as the hydrochloride salt (448 mg, 1.72 mmol, 100 %). No further purification was necessary.

| | |
|---|---|
| **Melting point** | 211 °C |
| **[α]_{D}²⁵** | + 79.5° (c = 1.51, MeOH) |
| **ESI (m/z)** | 206.85 [M+H]⁺ |
| **¹H NMR** | (600 MHz, DMSO-*d*₆) δ 9.14 (s, 1H), 8.63 (s, 3H), 6.43 (s, 2H), 3.92 (dd, J = 9.0, 7.4 Hz, 1H), 3.54 (s, 3H), 3.06 - 3.03 (m, 2H), 2.15 (s, 6H). |
| **¹³C NMR** | (101 MHz, DMSO) δ 170.0, 155.7, 137.8, 121.7, 114.9, 52.4, 51.5, 29.9, 19.6. |

### Intermediate 11: (S)-2-Amino-3-(4-hydroxy-2,6-dimethylphenyl)propanamide

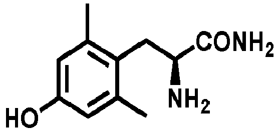

Intermediate 10 (360 mg, 1.39 mmol, 1 eq.) was dissolved in a minimum amount of a 5 % aqueous solution of sodium carbonate. The solution was extracted three times with 10 mL of an isopropanol: ethyl acetate mixture (1:4). The combined organic phases were dried over sodium sulfate and evaporated to dryness. The remaining solid was dissolved in 6 mL of dry methanol. The solution was cooled to -10°C using an acetone/ice bath and gaseous ammonia was bubbled through the solution for five minutes. The reaction flask was sealed and stirred at room temperature. Ammonia addition was repeated every 8 to 24 h until the starting material was consumed (TLC control). After 7 d the solvent was evaporated to give the amide product as a white solid (280 mg, 1.34 mmol, 97 %). No further purification was necessary.

| | |
|---|---|
| **Melting point** | 199 °C |
| **[α]_{D}²⁵** | + 66.8° (c = 0.89, MeOH) |
| **ESI (m/z)** | 208.89 [M+H]⁺ |
| **¹H NMR** | (400 MHz, DMSO-*d*₆) δ 8.92 (s, 1H), 7.18 (d, *J* = 2.7 Hz, 1H), 6.91 (d, *J =* 2.6 Hz, 1H), 6.38 (s, 2H), 3.23 (dd, *J* = 8.6, 5.8 Hz, 1H), 2.79 (dd, *J* = 13.9, 5.8 Hz, 1H), 2.57 - 2.53 (m, 1H), 2.20 (s, 6H), 1.65 (s, 2H). |
| **¹³C NMR** | (151 MHz, DMSO) δ 177.2, 154.7, 137.7, 125.9, 114.7, 55.3, 34.5, 20.2. |

### Intermediate 12: (S)-2-(Benzylamino)-3-(4-hydroxy-2,6-dimethylphenyl)propanamide

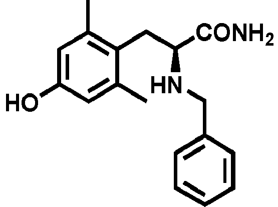

To a solution of Intermediate 11 (258 mg, 1.24 mmol, 1 eq.) and acetic acid (127 µL, 2.23 mmol, 1.8 eq.) in 5 mL of acetonitrile and 0.5 mL of dist. water was subsequently added sodium triacetoxyborohydride (788 mg, 3.72 mmol, 3 eq.) and then benzaldehyde (125 µL, 1.24 mmol, 1 eq.) at 0 °C. After stirring for 2h at r.t., an additional amount of sodium triacetoxyborohydride (262 mg, 1.24 mmol, 1 eq.) was added. The reaction was stirred for 30 minutes and 5 mL of a saturated sodium bicarbonate solution were added. Sodium carbonate (5 % aqueous solution) was added until pH = 9 was reached. The aqueous phase was extracted three times with 10 mL dichloromethane. The combined organic layers were dried over sodium sulfate and evaporated to dryness. Purification by flash column chromatography (eluent: DCM: MeOH + 0.1 % ammonia (25 % aq.), 99: 1 to 98: 2) gave the product as a colorless oil (238 mg, 798 µmol, 64 %).

| | |
|---|---|
| **Melting point** | 54 °C |
| **[α]_{D}²³** | - 14.7° (c = 0.609, MeOH) |
| **ESI (m/z)** | 299.03 [M+H]⁺ |
| **¹H NMR** | (600 MHz, Chloroform-d) δ 7.24 - 7.18 (m, 3H), 7.09 (d, *J* = 4.8 Hz, 1H), 6.99 (d, *J* = 7.0 Hz, 2H), 6.51 (s, 2H), 5.72 (d, *J* = 5.1 Hz) 3.75 (d, *J* = 13.9 Hz, 1H), 3.47 (d, *J* = 13.9 Hz, 1H), 3.32 (dd, *J* = 10.2, 5.4 Hz, 1H), 3.11 (dd, *J =* 14.4, 5.4 Hz, 1H), 2.82 (dd, *J* = 14.4, 10.2 Hz, 1H), 2.21 (s, 6H). |
| **¹³C NMR** | (151 MHz, CDCl₃) δ 178.0, 154.5, 139.1, 138.8, 128.5, 127.6, 127.1, 125.4, 115.5, 61.1, 52.4, 32.8, 20.6. |

### Intermediate 13: (S)-2-(Benzyl(methyl)amino)-3-(4-hydroxy-2,6-dimethylphenyl)propanamide

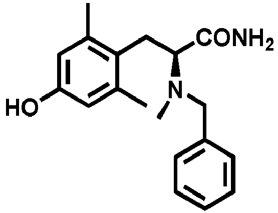

In a sealed microwave tube paraformaldehyde (333 mg, 7.24 mmol, 10 eq.) was refluxed for two hours to prepare a 35 wt. % solution in water. Intermediate 12 (216 mg, 724 µmol, 1 eq.) was dissolved in 5 mL of acetonitrile and 0.5 mL of distilled water and cooled to -10 °C in an acetone/ice bath. First, sodium triacetoxyborohydride (767 mg, 3.62 mmol, 5 eq.), second, the formaldehyde solution was added. The reaction was quenched after 5 min by the addition of 10 mL saturated sodium bicarbonate solution and sodium carbonate to adjust to pH = 9. The aqueous phase was extracted three times with 10 mL dichloromethane. The combined organic phases were dried over sodium sulfate and evaporated to dryness. No further purification was necessary. The product was obtained as a white solid (165 mg, 528 µmol, 73 %).

| | |
|---|---|
| **Melting point** | 137 °C |
| **[α]_{D}²⁵** | - 1.0° (c = 0.745, MeOH) |
| **ESI (m/z)** | 313.06 [M+H]⁺ |
| **¹H NMR** | (600 MHz, Chloroform-d) δ 7.31 - 7.27 (m, 2H), 7.26 - 7.20 (m, 3H), 6.43 (s, 2H), 6.19 (s, 1H), 5.49 (s, 1H), 3.73 (d, *J* = 13.5 Hz, 1H), 3.61 (d, *J =* 13.5 Hz, 1H), 3.28 (dd, *J* = 8.7, 5.3 Hz, 1H), 3.21 (dd, *J* = 14.0, 8.7 Hz, 1H), 2.96 (dd, *J* = 14.0, 5.3 Hz, 1H), 2.44 (s, 3H), 2.25 (s, 6H). |
| **¹³C NMR** | (151 MHz, CDCl₃) δ 175.6, 153.8, 138.9, 138.3, 128.6, 128.4, 127.5, 127.2, 115.2, 66.1, 59.4, 38.4, 28.4, 20.5. |

### Intermediate 14: (S)-4-(3-Amino-2-(benzyl(methyl)amino)propyl)-3,5-dimethylphenol

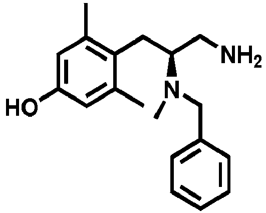

To an ice-cooled solution of Intermediate 13 (145 mg, 464 µmol, 1 eq.) in 3.0 mL of dry THF, borane-THF complex (2.78 ml, 2.78 mmol, 1M in THF, 6 eq.) was added dropwise at 0°C. After addition, the mixture was refluxed for six hours. The reaction was quenched by the slow addition of 0.4 mL of 2 N HCl at r.t. in order to cleave the borane-amine complex. The mixture was diluted with 5 mL dist. water and ammonia (25 % aq.) was added to adjust to pH = 9. The aqueous phase was extracted four times with 10 mL of dichloromethane. The combined organic phases were dried over sodium sulfate and evaporated to dryness. The remaining residue was subjected to flash column chromatography (eluent: DCM : MeOH + 0.1 % ammonia (25 % aq.), 100: 0 to 98: 2) which gave the product as a white solid (121 mg, 405 µmol, 87 %).

### Melting point 165 °C

| | |
|---|---|
| **[α]_{D}²⁴** | - 8.0° (c = 0.51, MeOH) |
| **ESI (m/z)** | 299.06 [M+H]⁺ |
| **¹H NMR** | (600 MHz, Chloroform-d) δ 7.37 - 7.30 (m, 4H), 7.28 - 7.23 (m, 1H), 6.36 (s, 2H), 3.87 (d, *J* = 13.3 Hz, 1H), 3.67 (d, *J* = 13.3 Hz, 1H), 2.91 - 2.85 (m, 2H), 2.76 (dd, *J* = 12.6, 10.6 Hz, 1H), 2.55 (dd, *J* = 14.3, 12.0 Hz, 1H), 2.41 (dd, *J* = 12.6, 3.3 Hz, 1H), 2.32 (s, 3H), 2.18 (s, 6H). |
| **¹³C NMR** | (151 MHz, CDCl₃) δ 154.7, 139.9, 137.6, 128.6, 128.6, 127.0, 126.7, 115.7, 65.3, 57.8, 40.8, 36.1, 24.1, 20.6. |

### Intermediate 15: (S)-4-(3-Amino-2-(methylamino)propyl)-3,5-dimethylphenol

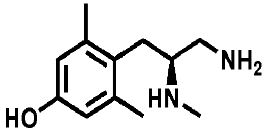

Intermediate 14 (110 mg, 269 µmol, 1 eq.), 1,1,2-trichloroethane (75 µL, 0.81 mmol, 2.2 eq.) and palladium (39 mg, 37 µmol, 0.1 eq., 10% on charcoal) were mixed in 8 mL of dry methanol. Hydrogen gas was purged into the flask and the reaction was left to stir at room temperature. Hydrogen addition was repeated every 8h to 16 h. After 48 h palladium hydroxide (52 mg, 36 µmol, 0.1 eq., 10 % on charcoal) was added and the suspension was stirred for 1 h. The mixture was filtered through a pad of celite and the solvent was evaporated. Purification by flash column chromatography (eluent: DCM: MeOH + 0.1 % ammonia (25 %, aq.), 20: 1 to 5: 1) gave the product as a white solid (49 mg, 0.24 mmol, 64 %).

| | |
|---|---|
| **Melting point** | 59 °C |
| **[α]_{D}²⁴** | + 18.7° (c = 0.41, MeOH) |
| **ESI (m/z)** | 208.94 [M+H]⁺ |
| **¹H NMR** | (600 MHz, DMSO-*d*₆) δ 6.42 (s, 2H), 2.79 (dd, *J* = 14.0, 5.5 Hz, 1H), 2.65-2.59 (m, 1H), 2.53 - 2.51 (m, 2H), 2.45 (dd, *J* = 13.9, 8.7 Hz, 1H), 2.34 (s, 3H), 2.19 (s, 6H). |
| **¹³C NMR** | (151 MHz, DMSO) δ 154.8, 137.3, 125.8, 114.9, 59.3, 42.1, 33.0, 30.5, 20.2. |

### Intermediate 16: (9H-Fluoren-9-yl)methyl (S)-(1-(4-carbamoylphenyl)-3-hydroxypropan-2-yl)carbamate

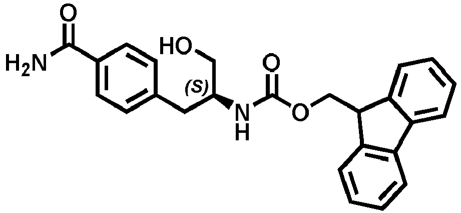

N-Fmoc-L-4-Carbamoylphenylalanine (1.00 g, 2.32 mmol, 1 eq.) was suspended in 20 mL of dry THF under nitrogen atmosphere. Borane-THF complex (1 M, 23.23 mL, 23.23 mmol) was slowly added at 0°C. The white suspension turned into a clear solution upon completion of addition. After stirring at 0°C for 24 h, 3 mL of methanol were slowly added in order to quench the reaction. Celite (1 g) was added to the solution and the solvent was evaporated. The crude was purified on silica gel, eluting with a gradient of DCM : MeOH 100 : 0 to 10 : 1.

Remaining starting material was recovered (63 mg, 0.15 mmol). The product was obtained as a white solid (550 mg, 1.32 mmol, respective yield 61 %.).

| | |
|---|---|
| **Melting point** | 142 - 144 °C |
| **[α]_{D}²⁵** | - 22.0° (c = 1.025, DMSO) |
| **ESI (m/z)** | 417.15 [M+H]⁺ |
| **HRMS (m/z)** | [M+Na]⁺ calculated for C₂₅H₂₄N₂O₄: 439.1628, found: 439.1622. |
| **NMR** | NMR spectra were measured at 298 K and 318 K. At 298 K two sets of signals were observed in the DEPTQ experiment. At 318 K the signal sets merged to one single signal set. This indicates that at 298 K the molecule adopts two different conformations, e.g. due to a rotational barrier or an inter- or intramolecular hydrogen bond, which is broken at 318 K. |
| **¹H NMR** | (600 MHz, DMSO-*d*₆, T = 318 K) δ 7.87 (dt, *J* = 7.5, 1.0 Hz, 2H), 7.83 (dt, *J* = 7.5, 0.9 Hz, 2H), 7.79 - 7.77 (m, 3H), 7.41 (td, *J* = 7.4, 1.1 Hz, 2H), 7.34 (td, *J* = 7.4, 1.1 Hz, 2H), 7.27 (d, *J* = 8.1 Hz, 2H), 7.12 (s, 1H), 6.26 (s, 2H), 3.41 - 3.37 (m, 1H), 3.28 (dd, *J* = 10.4, 4.9 Hz, 1H), 3.19 (dd, *J* = 10.4, 6.4 Hz, 1H), 2.92 - 2.86 (m, 1H), 2.73 (dd, *J* = 13.3, 5.5 Hz, 1H), 2.49 - 2.45 (m, 1H), 1.46 - 1.42 (m, 1H). |
| **¹³C NMR** | (151 MHz, DMSO, T = 318 K) δ 167.8, 143.4, 142.6, 139.4, 137.4, 131.9, 128.9, 128.8, 127.2, 127.2, 121.2, 119.9, 109.4, 65.9, 60.7, 54.2, 40.0, 29.2. |
| **Chiral HPLC** | system K: t_{R} = 34.8 min, purity (λ = 254 nm): ≥ 99 % |

### Intermediate 17: (9H-Fluoren-9-yl)methyl (S)-(1-(4-carbamoylphenyl)-3-oxopropan-2-yl)carbamate

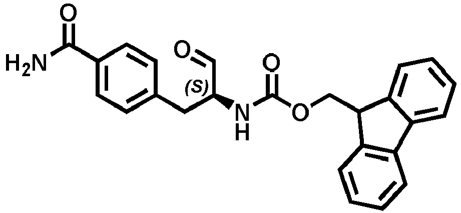

Intermediate 16 (403 mg, 0.97 mmol, 1 eq.) was dissolved in 100 mL dry THF. The clear solution was cooled with an acetone/ice bath and Dess-Martin periodinane (616 mg, 1.45 mmol, 1.5 eq.) was added in small portions. The white suspension was stirred at 0 °C for 6 h. The solvent was evaporated in a nitrogen stream to avoid heating. Celite and 10 mL of water were added for lyophilizing overnight. Purification via a Biotage system (10 g, silica column) eluting with a ratio of 100: 0 to 95: 5 (DCM: MeOH) gave the product as a white solid (320 mg, 0.77 mmol, 80 %). Due to instability, the substance was directly used for the next step without detailed characterization.

| | |
|---|---|
| **ESI (m/z)** | 415.06 [M+H]⁺ |
| **HRMS (m/z)** | [2M+H]+ calculated for C₂₅H₂₂N₂O₄: 829.3232, found: 829.3242 |

### Intermediate 18: (9H-Fluoren-9-yl)methyl (S)-(1-amino-3-(4-carbamoylphenyl)propan-2-yl)carbamate formiate

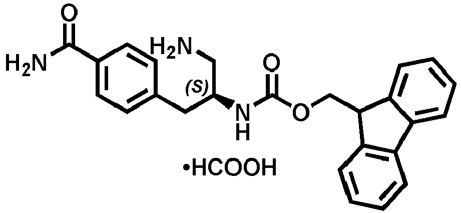

Intermediate 17 (310 mg, 0.76 mmol, 1 eq.) was dissolved in 60 mL of dry THF in an argon atmosphere. Dry molecular sieves were added and the solution was cooled to - 50 °C. Titanium tetraisopropoxide (443 µL, 1.50 mmol, 2 eq.) was slowly added and the mixture was stirred for 15 min. Ammonium trifluoroacetate (980 mg, 1.48 mmol, 10 eq.) and sodium cyanoborohydride (141 mg, 2.24 mmol, 3 eq.) in 20 mL dry THF were slowly added. The mixture was stirred at - 50 °C for two hours and then at -20 °C for three hours. Molecular sieves were filtered off and washed with 20 mL of methanol. The combined organic solvent fractions were evaporated in a nitrogen stream. Purification was performed on a Biotage system. The product was obtained as a white solid (90 mg, 0.20 µmol, 37 %, 60 mg of the respective alcohol were recovered, overall yield: 51 %).

| | |
|---|---|
| **Melting point** | 158 °C |
| **ESI (m/z)** | 416.08 [M+H]⁺ |
| **¹H NMR** | (600 MHz, DMSO-*d*₆) δ 8.24 (s, 1H), 7.90 - 7.82 (m, 4H), 7.81 - 7.79 (m, 2H), 7.63 - 7.60 (m, 2H), 7.44 - 7.39 (m, 2H), 7.37 - 7.28 (m, 4H), 7.26 (d, *J* = 8.0 Hz, 2H), 6.28 (s, 1H), 4.32 (dd, *J* = 9.9, 6.3 Hz, 1H)), 4.22 - 4.14 (m, 2H)), 3.87 - 3.80 (m, 1H), 2.88 - 2.80 (m, 2H), 2.78 - 2.69 (m, 2H). |
| **¹³C NMR** | (151 MHz, DMSO) δ 167.5, 163.8, 155.7, 143.7, 142.5, 141.4, 140.6, 139.3, 137.3, 132.2, 128.9, 128.8, 127.5, 127.4, 127.2, 126.9, 125.0, 124.9, 121.3, 120.0, 109.6, 65.3, 51.4, 46.6, 42.7, 37.3. |

### A-3 Syntheses of inventive compounds:

### Example 1: (S)-N-(2-(Dimethylamino)-3-(4-hydroxyphenyl)propyl)cinnamamide

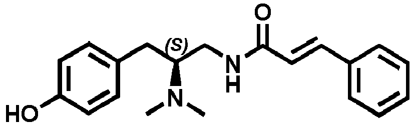

To a solution of Intermediate 6 (13 mg, 67 µmol, 1 eq.) in 0.5 mL dry DMF were added (*E*)-cinnamic acid (12 mg, 80 µmol, 1.2 eq.), BOP (35 mg, 80 µmol, 1.2 eq.) and triethylamine (65 µL, 0.47 mmol, 7 eq.) in 0.5 mL dry DMF. After stirring for 72 h at room temperature the solvent was removed under vacuum. The crude oil was dissolved in 5 mL of dichloromethane and extracted three times with 5 mL of saturated sodium bicarbonate solution. The organic phase was dried over sodium sulfate and evaporated to dryness. The crude was purified by flash column chromatography (solvent system: DCM : MeOH + 0.1 % ammonia (25 % aq.), 20: 1). The product was obtained as a white solid (15 mg, 46 µmol, 69 %).

| | |
|---|---|
| **Melting point** | 164 °C |
| **[α]_{D}²⁶** | + 92.0° (c = 0.486, CHCl₃) |
| **ESI (m/z)** | 325.21 [M+H]⁺ |
| **HRMS (m/z)** | [M+H]⁺ calculated for C₂₀H₂₄N₂O₂: 325.1911, found: 325.1908. |
| **¹H NMR** | (400 MHz, Chloroform-*d*) δ 7.59 (d, *J* = 15.6 Hz, 1H), 7.52 - 7.46 (m, 2H), 7.38 - 7.32 (m, 3H), 7.00 - 6.96 (m, 2H), 6.82 - 6.78 (m, 2H), 6.65 - 6.60 (m, 1H), 6.40 (d, *J* = 15.6 Hz, 1H), 3.60 (ddd, *J* = 14.0, 6.7, 4.5 Hz, 1H), 3.14 - 3.00 (m, 1H), 2.93 (dd, *J* = 13.3, 3.3 Hz, 1H), 2.81 - 2.71 (m, 1H), 2.38 (s, 6H), 2.29 (dd, *J* = 13.3, 10.5 Hz, 1H). |
| **¹³C NMR** | (151 MHz, CDCl₃) δ 166.3, 155.4, 141.2, 134.8, 129.9, 129.6, 129.4, 128.7, 127.9, 120.5, 115.7, 65.0, 40.2, 39.7, 30.5. |
| **HPLC** | system 1: t_{R} = 14.8 min, purity (λ = 254 nm): 97 %, (λ = 280 nm): 98 % system 2: t_{R} = 13.4 min, purity (λ = 254 nm): 96 %, (λ = 280 nm): 97 % |
| **Chiral HPLC** | system B: t_{R} = 19.7 min, purity (λ = 254 nm): ≥ 99 % |

### Example 2: (S)-N-(2-(Dimethylamino)-3-(4-hydroxyphenyl)propyl)-3-phenylpropiolamide

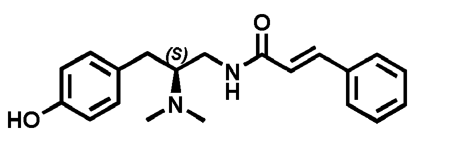

To a solution of Intermediate 6 (18 mg, 67 µmol) in 0.5 mL dry DMF were added 3-phenylpropiolic acid (12 mg, 80 µmol, 1.2 eq.), BOP (36 mg, 80 µmol, 1.2 eq.) and triethylamine (65 µL, 0.47 mmol, 7 eq.) in 0.5 mL dry DMF. After stirring for 23 h at room temperature the solvent was removed under vacuum. The crude oil was dissolved in 5 mL of dichloromethane and extracted three times with 5 mL of saturated sodium bicarbonate solution. The organic phase was dried over sodium sulfate and evaporated to dryness. The crude was first passed through a silica plug (eluent: ethyl acetate + 0.1 % TEA) and then purified by flash column chromatography (solvent system: DCM : MeOH + 0.1 % ammonia (25 % aq.), 20: 1). The product was obtained as a white solid (16 mg, 50 µmol, 74 %).

| | |
|---|---|
| **Melting point** | 152 - 153 °C |
| **[α]_{D}²⁶** | + 10.5° (c = 0.523, CHCl₃) |
| **ESI (m/z)** | 323.16 [M+H]⁺ |
| **HRMS (m/z)** | [M+H]⁺ calculated for C₂₀H₂₂N₂O₂: 323.1754, found: 323.1753. |
| **¹H NMR** | (400 MHz, Chloroform-d) δ 7.55 - 7.51 (m, 2H), 7.43 - 7.32 (m, 3H), 7.01 - 6.96 (m, 2H), 6.79 - 6.74 (m, 3H), 3.51 (ddd, *J* = 13.9, 6.8, 4.6 Hz, 1H), 3.05 - 2.97 (m, 1H), 2.93 (dd, *J* = 13.4, 3.5 Hz, 1H), 2.74 (m, 1H), 2.37 (s, 6H), 2.27 (dd, *J* = 13.4, 10.3 Hz, 1H). |
| **¹³C NMR** | (101 MHz, CDCl₃) δ 154.7, 153.5, 132.5, 130.1, 130.1, 130.0, 128.5, 120.3, 115.6, 84.7, 83.1, 64.6, 40.1, 39.9, 30.5. |
| **HPLC** | system 1: t_{R} = 14.7 min, purity (λ = 254 nm): 96 %, (λ = 280 nm): 96 % |
| | system 2: t_{R} = 13.4 min, purity (λ = 254 nm): 96 %, (λ = 280 nm): 96 % |
| **Chiral HPLC** | system B: t_{R} = 19.9 min, purity (λ = 254 nm): ≥ 99 % |

### Example 3: (S,E)-N-(2-(Dimethylamino)-3-(4-hydroxyphenyl)propyl)-3-phenylbut-2-enamide

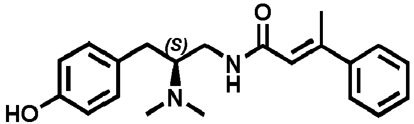

To a solution of Intermediate 6 (15 mg, 77 µmol, 1 eq.) in 0.5 mL dry DMF were added (*E*)-3-methyl-3-phenylacrylic acid (15 mg, 93 µmol, 1.2 eq.), PyBOP (48 mg, 93 µmol, 1.2 eq.) and triethylamine (54 µL, 0.39 mmol, 5 eq.) in 0.5 mL dry DMF. After stirring for 16 h at room temperature the solvent was removed under vacuum. The crude was first passed through a silica plug (eluent: ethyl acetate + 0.1 % TEA) and then purified by flash column chromatography (solvent system: DCM : MeOH + 0.1 % ammonia (25 % aq.), 95 : 5). The product was obtained as a white solid (14 mg,41 µmol, 54 %).

| | |
|---|---|
| **Melting point** | 151 °C |
| **[α]_{D}²⁴** | + 11.4° (c = 0.114, MeOH) |
| **ESI (m/z)** | 339.14 [M+H]⁺ |
| **HRMS (m/z)** | [M+H]⁺ calculated for C₂₁H₂₆N₂O₂: 339.2067, found: 339.2066. |
| **¹H NMR** | (600 MHz, Methanol-*d*₄) δ 7.48 - 7.43 (m, 2H), 7.36 - 7.33 (m, 2H), 7.33 - 7.29 (m, 1H), 7.05 - 7.02 (m, 2H), 6.73 - 6.70 (m, 2H), 6.10 (q, *J* = 1.4 Hz, 1H), 3.32 - 3.29 (m, 2H), 2.94 - 2.87 (m, 2H), 2.45 (d, *J* = 1.4 Hz, 3H), 2.43 - 2.38 (m, 1H), 2.37 (s, 6H). |
| **¹³C NMR** | (151 MHz, MeOD) δ 169.5 156.9, 151.2, 144.0, 131.7, 131.1, 129.6, 129.5, 127.2, 121.1, 116.4, 66.5, 40.9, 40.4, 33.2, 17.8. |
| **HPLC** | system 1: t_{R} = 15.7 min, purity (λ = 254 nm): ≥ 99 %, (λ = 280 nm): ≥ 99 % |
| | system 2: t_{R} = 13.9 min, purity (λ = 254 nm): ≥ 99 %, (λ = 280 nm): ≥ 99 % |
| **Chiral HPLC** | system B: t_{R} = 12.3 min, purity (λ = 254 nm): ≥ 99 % |

### Example 4: (S,E)-N-(2-(Dimethylamino)-3-(4-hydroxyphenyl)propyl)-2-methyl-3-phenylacrylamide

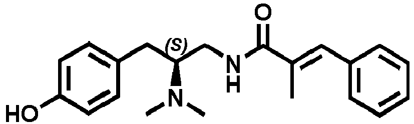

To a solution of Intermediate 6 (15 mg, 77 µmol, 1 eq.) in 0.5 mL dry DMF was added (*E*)-2-methyl-3-phenylacrylic acid (15 mg, 93 µmol, 1.2 eq.), PyBOP (48 mg, 93 µmol, 1.2 eq.) and triethylamine (54 µL, 0.39 mmol, 5 eq.) in 0.5 mL dry DMF. After stirring for 24 h at room temperature the solvent was removed under vacuum. The crude was first passed through a silica plug (eluent: ethyl acetate + 0.1 % TEA) and then purified by flash column chromatography (solvent system: DCM : MeOH + 0.1 % ammonia (25 % aq.), 95 : 5). The product was obtained as a white solid (18 mg, 53 µmol, 69 %).

| | |
|---|---|
| **Melting point** | 187 °C |
| **[α]_{D}²⁴** | + 0.05° (c = 1.07, MeOH) |
| **ESI (m/z)** | 339.13 [M+H]⁺ |
| **HRMS (m/z)** | [M+H]⁺ calculated for C₂₁H₂₆N₂O₂: 339.2067, found: 339.2070. |
| **¹H NMR** | (400 MHz, Chloroform-d) δ 7.40 - 7.28 (m, 6H), 7.02 - 6.98 (m, 2H), 6.87 (d, *J* = 6.2 Hz, 1H), 6.81 - 6.77 (m, 2H), 3.56 (ddd, *J* = 13.9, 6.5, 4.6 Hz, 1H), 3.11 (ddd, *J* = 13.7, 10.6, 2.4 Hz, 1H), 2.95 (dd, *J* = 13.3, 3.4 Hz, 1H), 2.86 - 2.78 (m, 1H), 2.41 (s, 6H), 2.33 (dd, *J* = 13.2, 10.3 Hz, 1H), 2.06 (d, *J* = 1.4 Hz, 3H). |
| **¹³C NMR** | (151 MHz, CDCl₃) δ 169.9, 155.2, 136.2, 134.3, 131.6, 130.0, 129.7, 129.4, 128.3, 127.8, 115.7, 65.1, 40.2, 40.0, 30.8, 14.1. |
| **HPLC** | system 1: t_{R} = 15.4 min, purity (λ = 254 nm): 98 %, (λ = 280 nm): 98 % |
| | system 2: t_{R} = 13.8 min, purity (λ = 254 nm): 98 %, (λ = 280 nm): 98 % |
| **Chiral HPLC** | system B: t_{R} = 16.6 min, purity (λ = 254 nm): ≥ 99 % |

### Example 5: (S)-N-(2-(Dimethylamino)-3-(4-hydroxyphenyl)-2-methylpropyl)cinnamamide

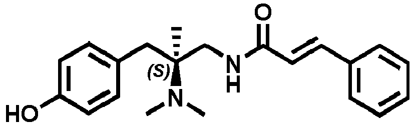

To a solution of Intermediate 8 (12 mg, 57 µmol, 1 eq.) in 0.5 mL dry DMF were added (*E*)-cinnamic acid (10 mg, 69 µmol, 1.2 eq.), PyBOP (36 mg, 69 µmol, 1.2 eq.) and triethylamine (40 µL, 0.29 mmol, 5 eq.) in 0.5 mL dry DMF. After stirring for 24 h at r.t. the solvent was removed under vacuum. The crude was first passed through a silica plug (eluent: ethyl acetate + 0.1 % TEA) and then purified by flash column chromatography (solvent system: DCM : MeOH + 0.1 % ammonia (25 % aq.), 95 : 5). The product was obtained as a clear oil (7.0 mg, 20 µmol, 36 %).

| | |
|---|---|
| **[α]_{D}²⁴** | + 63.5° (c = 0.207, MeOH) |
| **ESI (m/z)** | 339.13 [M+H]⁺ |
| **HRMS (m/z)** | [M+H]⁺ calculated for C₂₁H₂₆N₂O₂: 339.2067, found: 339.2067. |
| **¹H NMR** | (600 MHz, Chloroform-d) δ 7.62 (d, *J* = 15.6 Hz, 1H), 7.55 - 7.50 (m, 2H), 7.39 - 7.32 (m, 3H), 7.01 - 6.95 (m, 2H), 6.82 - 6.78 (m, 2H), 6.66 (s, 1H), 6.48 (d, *J* = 15.6 Hz, 1H), 3.34 (dd, *J* = 14.0, 4.0 Hz, 1H), 3.21 (dd, *J* = 14.0, 4.6 Hz, 1H), 2.68 (s, 2H), 2.35 (s, 6H), 0.94 (s, 3H). |
| **¹³C NMR** | (151 MHz, CDCl₃) δ 166.4, 155.5, 141.1, 134.9, 131.3, 129.6, 128.8, 128.2, 127.9, 120.6, 115.3, 58.7, 44.8, 38.1, 38.0, 17.9. |
| **HPLC** | system 1: t_{R} = 15.0 min, purity (λ = 254 nm): ≥ 99 %, (λ = 280 nm): ≥ 99 % |
| | system 2: t_{R} = 13.6 min, purity (λ = 254 nm): ≥ 99 %, (λ = 280 nm): ≥ 99 % |
| **Chiral HPLC** | system B: t_{R} = 13.2 min, purity (λ = 254 nm): ≥ 99 % |

### Example 6: (S,E)-N-(2-(Dimethylamino)-3-(4-hydroxyphenyl)propyl)-3-(naphthalen-1-yl)acrylamide

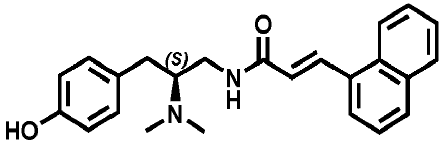

To a solution of Intermediate 6 (15 mg, 77 µmol, 1 eq.) in 0.5 mL dry DMF were added (*E*)-3-(1-naphtyl) acrylic acid (15 mg, 77 µmol, 1 eq.), PyBOP (40 mg, 77 µmol, 1 eq.) and triethylamine (22 µL, 0.15 mmol, 2 eq.) in 0.5 mL dry DMF. After stirring for 2h at room temperature the solvent was removed under vacuum. The crude was purified by flash column chromatography (solvent system: DCM: ethyl acetate: MeOH + 0.1 % ammonia (25 % aq.), 90: 5: 5). The product was obtained as a colorless crystalline solid (26 mg, 69 µmol, 90 %).

| | |
|---|---|
| **Melting point** | 90 °C |
| **[α]_{D}²⁵** | + 30.0° (c = 0.364, MeOH) |
| **ESI (m/z)** | 375.08 [M+H]⁺ |
| **HRMS (m/z)** | [M+H]⁺ calculated for C₂₄H₂₆N₂O₂: 375.2067, found: 375.2066. |
| **¹H NMR** | (400 MHz, Chloroform-*d*) δ 8.44 (d, *J* = 15.3 Hz, 1H), 8.25 - 8.20 (m, 1H), 7.87 - 7.83 (m, 2H), 7.69 (dt, *J* = 7.2, 0.9 Hz, 1H), 7.58 - 7.48 (m, 2H), 7.47 - 7.42 (m, 1H), 7.04 - 7.00 (m, 2H), 6.86 - 6.81 (m, 2H), 6.66 - 6.59 (d, *J* = 5.2 Hz, 1H), 6.47 (d, *J* = 15.3 Hz, 1H), 3.66 (ddd, *J* = 14.0, 6.8, 4.5 Hz, 1H), 3.11 (ddd, *J* = 14.0, 10.4, 4.5 Hz, 1H), 2.95 (dd, *J* = 13.3, 3.3 Hz, 1H), 2.81 -2.73 (m, 1H), 2.38 (s, 6H), 2.31 (dd, *J* = 13.3, 10.5 Hz, 1H). |
| **¹³C NMR** | (151 MHz, CDCl₃) δ 166.2, 155.4, 138.4, 133.6, 132.5, 131.5, 130.0, 129.9, 129.7, 128.5, 126.7, 126.1, 125.3, 124.6, 123.8, 123.4, 115.7, 65.1, 40.2, 39.8, 30.5. |
| **HPLC** | system 1: t_{R} = 16.7 min, purity (λ = 254 nm): ≥ 99 %, (λ = 230 nm): 97 % |
| | system 2: t_{R} = 14.6 min, purity (λ = 254 nm): 97 %, (λ = 230 nm): 97 % |
| **Chiral HPLC** | system B: t_{R} = 20.5 min, purity (λ = 254 nm): ≥ 99 % |

### Example 7: (S,E)-N-(2-(Dimethylamino)-3-(4-hydroxyphenyl)propyl)-3-(2-(trifluoromethyl)phenyl)acrylamide

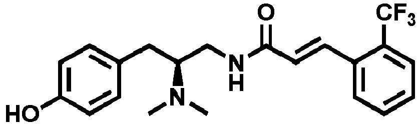

To a solution of Intermediate 6 (15 mg, 77 µmol, 1 eq.) in 0.5 mL dry DMF were added (*E*)-*o-*(trifluoromethyl)cinnamic acid (17 mg, 77 µmol, 1 eq.), PyBOP (40 mg, 77 µmol, 1 eq.) and triethylamine (22 µL, 0.15 mmol, 2 eq.) in 0.5 mL dry DMF. After stirring for 30 min at room temperature the solvent was removed under vacuum. The crude was passed through a silica plug (eluent: ethyl acetate + 0.1 % TEA) and then purified flash chromatography (solvent system: DCM : MeOH + 0.1 % ammonia (25 % aq.), 97: 3). The product was obtained as a colorless crystalline solid (26 mg, 66 µmol, 86 %).

| | |
|---|---|
| **Melting point** | 57 -59 °C |
| **[α]_{D}²⁵** | + 39.3° (c = 0.603, MeOH) |
| **ESI (m/z)** | 393.01 [M+H]⁺ |
| **HRMS (m/z)** | [M+H]⁺ calculated for C₂₁H₂₃F₃N₂O₂: 393.1784, found: 393.1783. |
| **¹H NMR** | (400 MHz, Methanol-*d*₄) δ 7.92 (dd, *J* = 15.5, 2.4 Hz, 1H), 7.81 (d, *J* = 7.8 Hz, 1H), 7.74 (d, *J* = 7.8 Hz, 1H), 7.65 (pseudo-t, *J* = 7.6 Hz, 1H), 7.55 (pseudo-t, *J* = 7.6 Hz, 1H), 7.15 - 7.09 (m, 2H), 6.80 - 6.72 (m, 2H), 6.57 (d, *J* = 15.5 Hz, 1H), 3.61 (dd, *J* = 15.1, 8.7 Hz, 1H), 3.42 - 3.34 (m, 2H), 3.05 (dd, *J* = 13.8, 4.1 Hz, 1H), 2.75 (s, 6H), 2.63 (dd, *J* = 13.7, 9.9 Hz, 1H). |
| **¹³CNMR** | (101 MHz, Methanol-*d*₄) δ 168.6, 157.6, 137.7, 135.0, 133.7, 131.3, 130.8 130.8, 129.5 (q, *J* = 30.17 Hz), 129.1, 127.2 (q, *J* = 5.7 Hz), 125.7, 125.6 (q, *J* = 272.7 Hz, CF₃), 116.7, 68.2, 40.7, 40.1, 32.5. |
| **HPLC** | system 1: t_{R} = 16.2 min, purity (λ = 254 nm): ≥ 99 %, (λ = 280 nm): ≥ 99 % |
| | system 2: t_{R} = 14.3 min, purity (λ = 254 nm): ≥ 99 %, (λ = 280 nm): ≥ 99 % |
| **Chiral HPLC** | system A: t_{R} = 32.7 min, purity (λ = 254 nm): ≥ 99 % |

### Example 8: (S,E)-N-(2-(Dimethylamino)-3-(4-hydroxyphenyl)propyl)-3-(3-(trifluoromethyl)phenyl)acrylamide formiate

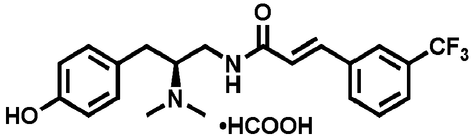

To a solution of Intermediate 6 (15 mg, 77 µmol, 1 eq.) in 0.5 mL dry DMF were added (*E*)-m-(trifluoromethyl)cinnamic acid (17 mg, 77 µmol, 1 eq.), PyBOP (40 mg, 77 µmol, 1 eq.) and triethylamine (22 µL, 0.15 mmol, 2 eq.) in 0.5 mL dry DMF. After stirring for 30 min at room temperature the solvent was removed under vacuum. The crude was passed through a silica plug (eluent: ethyl acetate + 0.1 % TEA) and then purified by preparative HPLC (RP-C8, flow: 10 mL/min, solvent system: ACN/H₂O + 0.1 % formic acid, 10 % ACN for 3 min, 10 % to 65 % ACN in 9 min, 65 % to 100 % ACN in 1 min, t_{R} = 10.4 min). The product was obtained as a white solid (30 mg, 68 µmol, 89 %).

| | |
|---|---|
| **Melting point** | 67 -69 °C |
| **[α]_{D}²⁴** | + 13.1° (c = 0.865, MeOH) |
| **ESI (m/z)** | 393.00 [M+H]⁺ |
| **HRMS** (m/z) | [M+H]⁺ calculated for C₂₁H₂₃F₃N₂O₂: 393.1784, found: 393.1785. |
| **¹H NMR** | (600 MHz, Methanol-*d*₄) δ 8.49 (s, 1H), 7.83 (s, 1H), 7.81 (d, *J* = 7.9 Hz, 1H), 7.67 (d, *J* = 7.7 Hz, 1H), 7.60 (pseudo-t, *J* = 7.8 Hz, 1H), 7.56 (d, *J* = 15.8 Hz, 1H), 7.14 - 7.10 (m, 2H), 6.79 - 6.75 (m, 2H), 6.67 (d, *J* = 15.8 |
| | Hz, 1H), 3.60 (dd, *J* = 14.7, 8.1 Hz, 1H), 3.44 - 3.35 (m, 2H), 3.06 (dd, *J* = 13.8, 4.2 Hz, 1H), 2.75 (s, 6H), 2.65 (dd, *J* = 13.8, 9.8 Hz, 1H). |
| **¹³C NMR** | (151 MHz, MeOD) δ 169.6, 169.0, 157.7, 140.5, 137.3, 132.4 (q, *J* = 32.4, CCF₃), 132.4, 131.3, 131.0, 128.9, 127.3 (q, *J* = 3.6 Hz), 125.4 (q, *J* = 3.8 Hz), 125.5 (q, *J* = 271.5 Hz, CF₃), 123.5, 116.8, 68.2, 40.8, 40.0, 32.7. |
| **HPLC** | system 1: t_{R} = 16.5 min, purity (λ = 254 nm): ≥ 99 %, (λ = 280 nm): ≥ 99 % system 2: t_{R} = 14.6 min, purity (λ = 254 nm): ≥ 99 %, (λ = 280 nm): ≥ 99 % |
| **Chiral HPLC** | system A: t_{R} = 32.8 min, purity (λ = 254 nm): ≥ 99 % |

### Example 9: (S,E)-N-(2-(Dimethylamino)-3-(4-hydroxyphenyl)propyl)-3-(4-(trifluoromethyl)phenyl)acrylamide

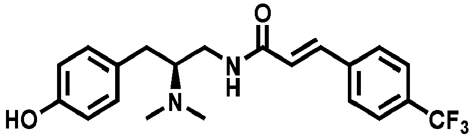

To a solution of Intermediate 6 (15 mg, 77 µmol, 1 eq.) in 0.5 mL dry DMF were added (*E*)-*p-*(trifluoromethyl)cinnamic acid (17 mg, 77 µmol, 1 eq.), PyBOP (40 mg, 77 µmol, 1 eq.) and triethylamine (22 µL, 0.15 mmol, 2 eq.) in 0.5 mL dry DMF. After stirring for 30 min at room temperature the solvent was removed under vacuum. The crude was passed through a silica plug (eluent: ethyl acetate + 0.1 % TEA) and then purified flash chromatography (solvent system: DCM : MeOH + 0.1 % ammonia (25 % aq.), 97: 3). The product was obtained as a colorless crystalline solid (26 mg, 61 µmol, 79 %).

| | |
|---|---|
| **Melting point** | 83 °C |
| **[α]_{D}²⁴** | + 7.2° (c = 0.607, MeOH) |
| **ESI (m/z)** | 393.01 [M+H]⁺ |
| **HRMS (m/z)** | [M+H]⁺ calculated for C₂₁H₂₃F₃N₂O₂: 393.1784, found: 393.1784. |
| **¹H NMR** | (600 MHz, Methanol-*d*₄) δ 7.71 (d, *J* = 8.3 Hz, 2H), 7.67 (d, *J* = 8.3 Hz, 2H), 7.50 (d, *J* = 15.8 Hz, 1H), 7.07 - 7.05 (m, 2H), 6.74 - 6.71 (m, 2H), 6.68 (d, *J* = 15.8 Hz, 1H), 3.41 (dd, *J* = 14.3, 8.3 Hz, 1H), 3.37 (dd, *J* = 14.3, 5.2 Hz, 1H), 3.08 - 3.02 (m, 1H)), 2.95 (dd, *J* = 13.7, 4.3 Hz, 1H), 2.52 - 2.44 (m, 7H). |
| **¹³CNMR** | (151 MHz, Methanol-*d*₄) δ 168.2, 157.1, 140.2, 139.9, 132.2 (q, *J* = 32.4 Hz), 131.1, 130.8, 129.3, 126.8 (q, *J* = 3.8 Hz), 125.5 (q, *J* = 271.0 Hz), 124.7, 116.5, 67.1, 40.8, 40.6, 32.9. |
| **HPLC** | system 1: t_{R} = 16.8 min, purity (λ = 254 nm): ≥ 99 %, (λ = 280 nm): ≥ 99 % |
| | system 2: t_{R} = 14.7 min, purity (λ = 254 nm): ≥ 99 %, (λ = 280 nm): ≥ 99 % |
| **Chiral HPLC** | system A: t_{R} = 35.8 min, purity (λ = 254 nm): ≥ 99 % |

### Example 10: (S)-N-(2-Amino-3-(4-hydroxy-2,6-dimethylphenyl)propyl)cinnamamide formiate

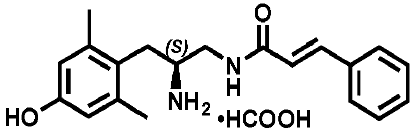

Intermediate 9 (42 mg, 93 µmol, 1 eq.) was dissolved in 1 mL dry THF in a sealed tube under an argon atmosphere. Borane-THF (1M in THF, 0.56 mL, 0.56 mmol, 6 eq.) was added dropwise at 0°C. The mixture was heated at 85 °C for 7 h until completion. The reaction was quenched by the slow addition of 1 mL of dry methanol at r.t. in order to cleave the aminoborane complex. After stirring for 30 minutes, the solvent was evaporated to give a colorless oil. Due to instability, the intermediate was not further characterized.

The colorless oil and triethylamine (26 µL, 186 µmol, 2 eq.) in 0.5 mL dry DMF were added to a mixture of *trans*-cinnamic acid (14 mg, 93 µmol, 1 eq.) and PyBOP (28 mg, 93 µmol, 1 eq.) in 0.5 mL dry DMF at room temperature. After stirring for 0.5 h, the solvent was evaporated under vacuum to give a clear foamy residue. Due to instability, the intermediate was not further characterized.

The foamy residue was dissolved in 3 mL of dry dichloromethane and TFA (36 µL, 0.46 mmol, 5 eq.) was added at 0°C. The solution turned from colorless to bright yellow. The reaction was ended after stirring for 0.5 h at r.t. by dilution with 5 mL of dichloromethane. The organic phase was extracted five times with 5 mL of HCI (1M). The combined aqueous phases were basified with ammonia (25 % aq.) until pH = 9 - 10 was reached; the clear solution turned turbid. The aqueous phase was extracted five times with 5 mL of dichloromethane. The combined organic phases were dried over sodium sulfate and evaporated to dryness to yield a white solid. The product was purified by preparative HPLC (RP-C8, flow = 10 mL/min, solvent system: ACN/H₂O + 0.1 % formic acid, 15 % ACN for 3 min, 15 % to 35 % ACN in 32 min, t_{R} = 11.5 min). The product was obtained as a white solid (16.1 mg, 44 µmol, 47 %).

| | |
|---|---|
| **Melting point** | 116 °C |
| **[α]_{D}²⁵** | + 5.3° (c = 0.37, MeOH) |
| **ESI (m/z)** | 325.08 [M+H]⁺ |
| **HRMS (m/z)** | [M+H]⁺ calculated for C₂₀H₂₄N₂O₂: 325.1911, found: 325.1909. |
| **¹H NMR** | (600 MHz, DMSO-*d*₆) δ 8.98 (s, 1H), 8.19 (t, *J* = 5.7 Hz, 1H), 7.58 - 7.54 (m, 2H), 7.44 - 7.35 (m, 4H), 6.64 (d, *J* = 15.8 Hz, 1H), 6.42 (s, 2H), 3.22 - 3.13 (m, 2H), 3.09 - 3.04 (m, 1H), 2.66 (dd, *J* = 14.2, 6.8 Hz, 1H), 2.62 - 2.55 (m, 1H), 2.20 (s, 6H). |
| **¹³C NMR** | (151 MHz, DMSO) δ 165.2, 163.6, 154.9, 138.5, 137.5, 134.8, 129.3, 128.8, 127.4, 125.4, 122.1, 114.9, 51.6, 43.9, 32.9, 20.2. |
| **HPLC** | system 1: t_{R} = 15.1 min, purity (λ = 254 nm): ≥ 99 %, (λ = 280 nm): ≥ 99 % |
| | system 2: t_{R} = 13.1 min, purity (λ = 254 nm): ≥ 99 %, (λ = 280 nm): ≥ 99 % |
| **Chiral HPLC** | system B: t_{R} = 34.4 min, purity (λ = 280 nm): ≥ 99 % |

### Example 11: (S,E)-N-(2-Amino-3-(4-hydroxy-2,6-dimethylphenyl)propyl)-3-(3-(trifluoromethyl)phenyl)acrylamide formiate

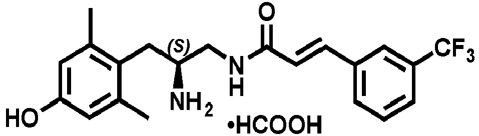

Intermediate 9 (40 mg, 89 µmol, 1 eq.) was dissolved in 2 mL dry THF in a sealed tube under an argon atmosphere. Borane-THF (1M in THF, 0.80 mL, 0.80 mmol, 6 eq.) was added dropwise at 0°C. The mixture was heated at 85 °C for 7 h until completion. The reaction was quenched by the slow addition of 1 mL of dry methanol at r.t. in order to cleave the aminoborane complex. After stirring for 30 minutes, the solvent was evaporated to give a colorless oil. Due to instability, the intermediate was not further characterized.

The colorless oil and triethylamine (25 µL, 0.18 mmol, 2 eq.) in 0.5 mL dry DMF was added to a mixture of (*E*)-3-trifluormethyl cinnamic acid (19 mg, 89 µmol, 1 eq.) and pyBOP (46 mg, 89 µmol, 1 eq.) in 0.5 mL dry DMF at room temperature. After stirring for 0.5 h, the solvent was evaporated under vacuum to give a clear foamy residue. Due to instability, the intermediate was not further characterized.

The foamy residue was dissolved in 3 mL of dry dichloromethane and TFA (34 µL, 0.44 mmol, 5 eq.) was added at 0°C. The solution turned from colorless to bright yellow. The reaction was ended after stirring for 0.5 h at r.t. by dilution with 5 mL of dichloromethane. The organic phase was extracted five times with 5 mL of HCI (1M). The combined aqueous phases were basified with ammonia (25 % aq.) until pH = 9 - 10 was reached; the clear solution turned turbid. The suspension was extracted five times with 5 mL of dichloromethane. The combined organic phases were dried over sodium sulfate and evaporated to dryness to yield a white solid. The product was purified by preparative HPLC (RP-C8, flow = 10 mL/min, solvent system: ACN/H₂O + 0.1 % formic acid, 15 % ACN for 3 min, 15 % to 35 % ACN in 32 min, t_{R} = 21.2 min). The product was obtained as a white solid, 15.32 mg (35 µmol, 39 %).

| | |
|---|---|
| **Melting point** | 98 °C |
| **[α]_{D}²⁴** | + 17.1° (c = 0.13, DMSO) |
| **ESI (m/z)** | 393.17 [M+H]⁺ |
| **HRMS (m/z)** | [M+H]⁺ calculated for C₂₁H₂₃F₃N₂O₂: 393.1784, found: 393.1784. |
| **¹H NMR** | (600 MHz, DMSO-*d*₆) δ 8.54 (t, *J* = 5.6 Hz, 1H), 8.41 (s, 1H), 7.91 (s, 1H), 7.87 (d, *J* = 7.8 Hz, 1H), 7.72 (d, *J* = 7.8 Hz, 1H), 7.65 (t, *J* = 7.8 Hz, 1H), 7.50 (d, *J* = 15.8 Hz, 1H), 6.82 (d, *J* = 15.9 Hz, 1H), 6.43 (s, 2H), 3.25 - 3.18 (m, 2H), 3.17 - 3.10 (m, 1H), 2.71 (dd, *J* = 14.2, 7.3 Hz, 1H), 2.66 (dd, *J* = 14.2, 7.0 Hz, 1H), 2.20 (s, 6H). |
| **¹³C NMR** | (151 MHz, DMSO) δ 165.4, 164.9, 155.1, 137.5, 136.8, 136.0, 131.1, 130.0, 129.5 (q, *J* = 31.8 Hz), 125.6 (q, *J* = 3.8 Hz), 124.8, 124.3, 123.9 (q, *J* = 272.4 Hz), 123.8 (q, *J* = 3.8 Hz), 115.0, 51.3, 43.2, 32.4, 20.2. |
| **HPLC** | system 1: t_{R} = 16.8 min, purity (λ = 254 nm): ≥ 99 %, (λ = 280 nm): ≥ 99 % |
| | system 2: t_{R} = 14.2 min, purity (λ = 254 nm): ≥ 99 %, (λ = 280 nm): ≥ 99 % |
| **Chiral HPLC** | system F: t_{R} = 27.6 min, purity (λ = 280 nm): ≥ 99 % |

### Example 12: (S,E)-N-(3-(4-Hydroxy-2,6-dimethylphenyl)-2-(methylamino)propyl)-3-(3-(trifluoromethyl)phenyl)acrylamide formiate

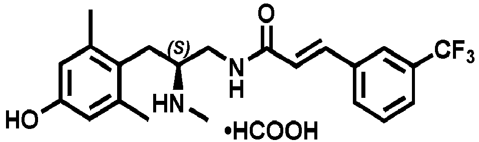

To a solution of Intermediate 15 (15 mg, 72 µmol, 1 eq.) in 0.5 mL dry DMF were added (*E*)-3-trifluoromethyl cinnamic acid (16 mg, 72 µmol, 1 eq.), PyBOP (38 mg, 72 µmol, 1 eq.) and triethylamine (20 µL, 0.14 mmol, 2 eq.). After stirring for 30 min at room temperature the solvent was evaporated under vacuum. The oily residue was dissolved in 5 mL of dichloromethane and extracted four times with 5 mL of 1 N HCl. The combined aqueous phases were basified with ammonia (25 % aq., pH = 9) and a white precipitate formed. The suspension was extracted four times with 5 mL dichloromethane. The combined organic phases were dried over sodium sulfate and evaporated to dryness. Purification by preparative HPLC (RP-C18, flow: 5 mL/min, solvent system: ACN/H₂O + 0.1 % formic acid, 15 % ACN for 3 min, 15 % to 45 % ACN in 32 min,45 % ACN to 95 % ACN in 1 min, t_{R} = 26.1 min) gave the product as a white solid (8.0 mg, 18 µmol, 25 %).

| | |
|---|---|
| **Melting point** | 104 °C |
| **[α]_{D}²⁴** | + 2.7° (c = 0.10, MeOH) |
| **ESI (m/z)** | 407.14 [M+H]⁺ |
| **HRMS (m/z)** | [M+H]⁺ calculated for C₂₂H₂₅F₃N₂O₂: 407.1941, found: 407.1941. |
| **¹H NMR** | (600 MHz, DMSO-*d*₆) δ 8.95 (s, 1H), 8.33 (s, 1H), 8.00 (t, *J* = 5.5 Hz, 1H), 7.92 (s, 1H), 7.87 (d, *J* = 7.8 Hz, 1H), 7.72 (d, *J* = 7.8 Hz, 1H), 7.65 (t, *J* = 7.8 Hz, 1H), 7.48 (d, *J* = 15.8 Hz, 1H), 6.89 (d, *J* = 15.8 Hz, 1H), 6.40 (s, |
| | 2H), 3.22 - 3.17 (m, 1H), 3.14 - 3.09 (m, 1H), 2.71 - 2.65 (m, 2H), 2.55 - 2.52 (m, 1H), 2.30 (s, 3H), 2.19 (s, 6H). |
| **¹³C NMR** | (151 MHz, DMSO-*d*₆) δ 164.6, 154.7, 137.4, 136.6, 136.1, 131.3, 129.9, 129.6 (q, *J* = 31.9 Hz), 126.3, 125.5 (q, *J* = 3.6 Hz), 123.9 (q, *J* = 272.7 Hz), 124.5, 123.6 (q, *J* = 3.8 Hz), 114.8, 59.3, 41.3, 33.6, 31.4, 20.2. |
| **HPLC** | system 1: t_{R} = 16.7 min, purity (λ = 254 nm): ≥ 99 %, (λ = 280 nm): ≥ 99 % |
| | system 2: t_{R} = 14.4 min, purity (λ = 254 nm): 95 %, (λ = 280 nm): 95 % |
| **Chiral HPLC** | system B: t_{R} = 15.5 min, purity (λ = 280 nm): ≥ 99 % |

### Example 13: (S,E)-N-(2-(Dimethylamino)-3-(4-hydroxyphenyl)propyl)-3-(m-tolyl)acrylamide

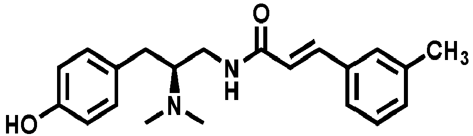

To a solution of Intermediate 6 (15 mg, 77 µmol, 1 eq.) in 0.5 mL dry DMF were added (*E*)-m-methyl-cinnamic acid (13 mg, 77 µmol, 1 eq.), PyBOP (40 mg, 77 µmol, 1 eq.) and triethylamine (22 µL, 0.15 mmol, 2 eq.). After stirring for 30 min at room temperature the solvent was removed under vacuum. The crude was passed through a silica plug (eluent: ethyl acetate + 0.1 % TEA) and then purified by flash column chromatography (eluent: DCM : MeOH + 0.1 % ammonia (25 % aq.), 100 %: 0 % to 97 %: 3 %). The product was obtained as a white solid (15 mg, 44 µmol, 57 %).

| | |
|---|---|
| **Melting point** | 74 - 75 °C |
| **[α]_{D}²⁶** | + 2.3° (c = 0.42, MeOH) |
| **ESI (m/z)** | 339.07 [M+H]⁺ |
| **HRMS (m/z)** | [M+H]⁺ calculated for C₂₁H₂₆N₂O₂: 339.2067, found: 339.2068. |
| **¹H NMR** | (600 MHz, Methanol-*d*₄) δ 7.46 (d, *J* = 15.8 Hz, 1H), 7.35 (s, 1H), 7.33 (d, *J* = 7.8 Hz, 1H), 7.26 (t, *J* = 7.6 Hz, 1H), 7.18 (d, *J* = 7.6 Hz, 1H), 7.11 - 7.08 (m, 2H), 6.77 - 6.74 (m, 2H), 6.54 (d, *J* = 15.8 Hz, 1H), 3.52 (dd, *J* = 14.6, 8.4 Hz, 1H), 3.38 (dd, *J* = 14.6, 4.5 Hz, 1H), 3.28 - 3.22 (m, 1H), 3.01 (dd, *J* = 13.8, 4.4 Hz, 1H), 2.65 (s, 6H), 2.57 (dd, *J* = 13.8, 9.8 Hz, 1H), 2.34 (s, 3H). |
| **¹³C NMR** | (151 MHz, MeOD) δ 169.5, 157.4, 142.4, 139.8, 136.1, 131.7, 131.2, 129.9, 129.7, 129.5, 126.1, 121.2, 116.6, 67.8, 40.8, 40.2, 32.7, 21.3. |
| **HPLC** | system 1: t_{R} = 15.5 min, purity (λ = 254 nm): 96 %, (λ = 280 nm): 96 % |
| | system 2: t_{R} = 13.6 min, purity (λ = 254 nm): 96 %, (λ = 280 nm): 96 % |
| **Chiral HPLC** | system K: t_{R} = 19.0 min, purity (λ = 280 nm): ≥ 99 % |

### Example 14: (S,E)-4-(2-Amino-3-(3-(3-(trifluoromethyl)phenyl)acrylamido)propyl)benzamide formiate

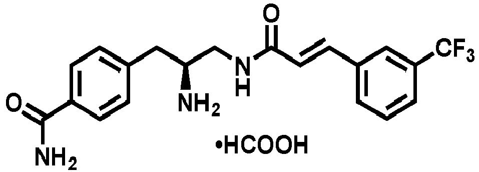

Intermediate 18 (40 mg, 87 µmol, 1 eq.) was dissolved in 1.5 mL dry DMF. PyBOP (45 mg, 87 µmol, 1 eq.), E-3-trifluormethyl cinnamic acid (19 mg, 87 µmol, 1 eq.) and TEA (24 µL, 0.17 mmol, 2 eq.) were dissolved in 1.5 mL dry DMF and were slowly added at 0 °C. The colorless solution was stirred at r.t. for 1 h and the solution turned slightly yellow. Piperidine (0.75 mL, 20 vol. % in DMF) was added and the mixture was allowed to stir for 1 h. The solvent was evaporated under vacuum and the crude was purified on a silica column eluting with a gradient of DCM : MeOH + 0.1 % ammonia (25 % aq.) 99: 1 to 94: 6. The oily product was further purified by preparative HPLC (RP-C8, flow: 10 mL/min, solvent system: ACN/H₂O + 0.1 % formic acid, 10 % ACN for 3 min, 10 % to 50 % ACN in 12 min, 50 % to 100 % in 1 min, t_{R} = 12.4 min) to give a white solid (18 mg, 41 µmol, 48 %).

| | |
|---|---|
| **Melting point** | 139 - 141 °C |
| **[α]_{D}²⁴** | + 18.3° (c = 0.20, MeOH) |
| **ESI (m/z)** | 392.04 [M+H]⁺ |
| **HRMS (m/z)** | [M+H]⁺ calculated for C₂₀H₂₀F₃N₃O₂: 392.1580, found: 392.1581. |
| **¹H NMR** | (400 MHz, DMSO-*d*₆) δ 8.40 - 8.33 (m, 1H), 8.30 (s, 1H), 7.93 (s, 1H), 7.89 (d, *J* = 7.8 Hz, 1H), 7.84 - 7.79 (m, 2H), 7.74 (d, *J* = 7.8 Hz, 1H), 7.66 (t, *J* = 7.8 Hz, 1H), 7.52 (d, *J* = 15.8 Hz, 1H), 7.36 - 7.28 (m, 3H), 6.85 (d, *J =* 15.9 Hz, 1H), 3.33 - 3.23 (m, 1H), 3.19 - 3.08 (m, 2H), 2.79 (dd, *J* = 13.4, 5.3 Hz, 1H), 2.66 (dd, *J* = 13.6, 6.4 Hz, 1H). |
| **¹³CNMR** | (151 MHz, DMSO-*d*₆) δ 167.6, 164.8, 164.1, 142.1, 136.8, 136.1 , 132.1, 131.2, 130.0, 129.5 (q, *J* = 31.7 Hz), 129.0, 127.4, 125.6 (q, *J* = 3.3 Hz), 124.4, 123.9 (q, *J* = 272.5 Hz), 123.7 (q, *J* = 3.6 Hz), 52.0, 44.0, 39.6. |
| **Chiral HPLC** | system I: t_{R} = 16.1 min, purity (λ = 254 nm): ≥ 99 % |

### Example 15: (S,E)-4-(2-(Dimethylamino)-3-(3-(3-(trifluoromethyl)phenyl)acrylamido)propyl)benzamide formiate

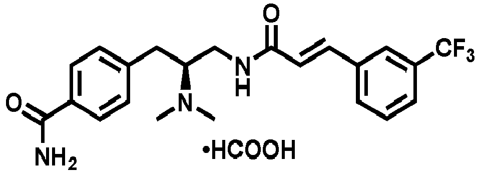

Formaldehyde was freshly prepared from paraformaldehyde (11 mg, 0.13 mmol, 5 eq., 35 % in water) by refluxing for 3h in a sealed tube. Example 14 (11 mg, 28 µmol, 1 eq.) was dissolved in a mixture of water: acetonitrile 0.5 mL : 2.5 mL. Sodium triacetoxyborohydride (16 mg, 75 µmol, 3 eq.) and subsequently formaldehyde (5 eq.) was added at ambient temperature. The reaction was stirred for 15 min. Saturated sodium bicarbonate solution (3 mL) and a spatula tip of sodium carbonate was added (pH = 9). The aqueous phase was extracted five times with 5 mL dichloromethane and evaporated to dryness. The crude was dissolved in 2.5 mL of acetonitrile and 0.5 mL of a 2 M sodium hydroxide solution and stirred at r.t. for 1 h. The aqueous phase was diluted with 2.5 mL dist. water, extracted five times with 5 mL dichloromethane and evaporated to dryness. The procedure was repeated two times until no N-hydroxymethyl side product was found by LC-MS analysis. The crude was purified by preparative HPLC (RP-C8, flow: 10 mL/min, solvent system: ACN/H₂O + 0.1 % formic acid, 10 % ACN for 3 min, 10 % to 50 % ACN in 12 min, 50 % to 100 % in 1 min, t_{R} = 12.4 min). The product was obtained as a white solid (7.5 mg, 16 µmol, 64 %).

| | |
|---|---|
| **[α]_{D}²⁴** | + 6.4° (c = 0.41, MeOH) |
| **ESI (m/z)** | 420.07 [M+H]⁺ |
| **HRMS (m/z)** | [M+H]⁺ calculated for C₂₂H₂₄F₃N₃O₂: 420.1893, found: 420.1890. |
| **¹H NMR** | (400 MHz, DMSO-*d*₆) δ 8.26 (s, 1H), 7.95 - 7.84 (m, 4H), 7.82 - 7.77 (m, 2H), 7.72 (d, *J* = *7.8* Hz, 1H), 7.64 (t, *J* = 7.8 Hz, 1H), 7.44 (d, *J* = 15.8 Hz, 1H), 7.35 - 7.25 (m, 3H), 6.90 (d, *J* = 15.8 Hz, 1H), 3.19 (t, *J* = 6.0 Hz, 2H), 2.94 - 2.80 (m, 2H), 2.53 - 2.48 (1H, under DMSO signal), 2.29 (s, 6H). |
| **¹³C NMR** | (151 MHz, DMSO-*d*₆) δ 167.7, 164.3, 163.9, 143.8, 136.5, 136.1, 131.8, 131.3, 129.9, 129.6 (q, *J* = 31.8 Hz), 128.7, 127.4, 125.5 (q, *J* = 3.7 Hz), 124.5, 123.9 (q, *J* = 272.4 Hz), 123.6 (q, *J* = 3.7 Hz), 64.5, 40.0, 38.5, 32.0. |
| **HPLC** | system 1: t_{R} = 17.4 min, (λ = 254 nm): ≥ 99 % |
| | system 2: t_{R} = 13.6 min, purity (λ = 230 nm): ≥ 99 %, (λ = 280 nm): ≥ 99 % |
| **Chiral HPLC** | system I: t_{R} = 17.4 min, purity (λ = 254 nm): ≥ 99 % |

### Example 16: (S,E)-N-(2-(Dimethylamino)-3-(4-hydroxyphenyl)propyl)-3-(4-fluoro-3-(trifluoromethyl)phenyl)acrylamide formiate

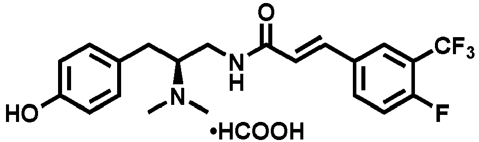

To a solution of Intermediate 6 (35 mg, 0.18 mmol, 1 eq.) in 0.5 mL dry DMF were added (*E*)-4-fluoro-3-trifluormethyl cinnamic acid (42 mg, 0.18 mmol, 1 eq.), PyBOP (94 mg, 0.18 mmol, 1 eq.) and triethylamine (50 µL, 0.36 mmol, 2 eq.). After stirring for 30 min at room temperature the solvent was removed under vacuum. The crude was passed through a silica plug (eluent: ethyl acetate + 0.1 % TEA) and then purified by preparative HPLC (RP-C8, flow: 10 mL/min, solvent system: ACN/H₂O + 0.1 % formic acid, 5 % ACN for 3 min, 5 % to 71 % ACN in 15 min, 71 % to 100 % in 3 min, t_{R} = 13.0 min). The product was obtained as a white solid (46 mg, 0.10 mmol, 56 %).

| | |
|---|---|
| **Melting point** | 106 - 108 °C |
| **[α]_{D}²⁵** | - 12.5° (c = 2.05, MeOH) |
| **ESI (m/z)** | 411.08 [M+H]⁺ |
| **HRMS (m/z)** | [M+H]⁺ calculated for C₂₁H₂₂F₄N₂O₂: 411.1690, found: 411.1688. |
| **¹H NMR** | (600 MHz, DMSO-*d*₆) δ 8.17 (s, 1H), 7.98 (dd, *J* = 7.0, 2.2 Hz, 1H), 7.94 (ddd, *J* = 8.4, 4.9, 2.2 Hz, 1H), 7.82 - 7.79 (m, 1H), 7.56 (dd, *J* = 10.6, 8.7 Hz, 1H), 7.43 (d, *J* = 15.8 Hz, 1H), 7.03 - 7.00 (m, 2H), 6.84 (d, *J* = 15.8 Hz, 2H), 6.70 - 6.67 (m, 2H), 3.22 (ddd, *J* = 14.0, 6.0, 4.8 Hz, 1H), 3.16 (ddd, *J* = 14.0, 8.3, 4.5 Hz, 1H), 2.83 - 2.75 (m, 2H), 2.36 - 2.30 (m, 7H). |
| **¹³C NMR** | (151 MHz, DMSO-*d*₆) δ 164.3, 163.2, 158.9 (d, *J* = 256.0 Hz), 155.4, 135.6, 133.9 (d, *J* = 8.9 Hz), 132.3 (d, *J* = 3.6 Hz), 129.7, 129.6, 125.9 (q, *J* = 4.3 Hz), 124.4 (d, *J* = 1.6 Hz), 122.4 (q, *J* = 272.2 Hz), 117.8 (d, *J* = 20.9 Hz), 117.0 (qd, *J* = 32.4, 12.7 Hz), 115.0, 64.9, 40.0, 38.4, 30.9. |
| **HPLC** | system 1: t_{R} = 16.5 min, purity (λ = 230 nm): ≥ 99 %, (λ = 280 nm): ≥ 99 % system 2: t_{R} = 14.4 min, purity (λ = 230 nm): ≥ 99 %, (λ = 280 nm): ≥ 99 % |
| **Chiral HPLC** | system K: t_{R} = 9.5 min, purity (λ = 254 nm): ≥ 99 % |

### Example 17: (S,E)-4-(2-Amino-3-(3-(naphthalen-1-yl)acrylamido)propyl)benzamide formiate

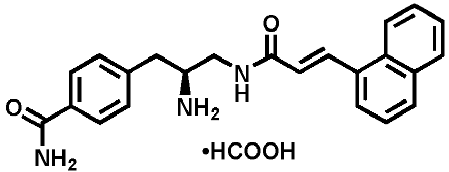

Intermediate 18 (40 mg, 87 µmol, 1 eq.) was dissolved in 0.5 mL dry DMF. PyBOP (50 mg, 95 µmol, 1.1 eq.), *E*-(α-naphtyl) cinnamic acid (19 mg, 95 mmol, 1.1 eq.) and TEA (25 µL, 0.18 mmol, 2.1 eq.) were dissolved in 0.5 mL dry DMF and slowly added at 0 °C. The colorless solution was stirred at r.t. for 1 h. Piperidine (0.25 mL, 20 vol. % final concentration in DMF) was added. The mixture was allowed to stir for 1 h. The solvent was evaporated under vacuum and the crude was purified by flash column chromatography (eluent: ethyl acetate + 0.1 % TEA, then DCM : MeOH + 0.1 % ammonia (25 % aq.), 99: 1 to 94: 6). The product was further purified by preparative HPLC (RP-C8, flow: 10 mL/min, solvent system: ACN/H₂O + 0.1 % formic acid, 10 % ACN for 3 min, 10 % to 50 % ACN in 12 min, 50 % to 100 % in 1 min, t_{R} = 11.5 min) to give a white solid (10 mg, 27 µmol, 31 %).

| | |
|---|---|
| **Melting point** | 116 °C |
| **[α]_{D}²⁵** | + 15.2° (c = 0.29, MeOH) |
| **ESI (m/z)** | 374.18 [M+H]⁺ |
| **HRMS (m/z)** | [M+H]⁺ calculated for C₂₃H₂₃N₃O₂: 374.1863, found: 374.1863. |
| **¹H NMR** | (600 MHz, DMSO-*d*₆) δ 8.41 (t, *J* = 5.3 Hz, 1H), 8.31 (s, 1H), 8.22 - 8.19 (m, 2H), 8.00 - 7.96 (m, 2H), 7.91 (s, 1H), 7.84 - 7.81 (m, 2H), 7.80 - 7.77 (m, 1H), 7.63 (ddd, *J* = 8.4, 6.8, 1.5 Hz, 1H), 7.60 - 7.55 (m, 2H), 7.36 - 7.33 (m, 2H), 7.28 (s, 1H), 6.76 (d, *J* = 15.6 Hz, 1H), 3.35 - 3.29 (m, 1H), 3.19 - 3.13 (m, 2H), 2.82 (dd, *J* = 13.5, 5.6 Hz, 1H), 2.67 (dd, *J* = 13.4, 6.8 Hz, 1H). |
| **¹³CNMR** | (151 MHz, DMSO) δ 167.6, 165.1, 164.4, 142.2, 135.0, 133.2, 132.1, 131.9, 130.7, 129.4, 129.0, 128.6, 127.4, 126.8, 126.1, 125.6, 125.2, 124.3, 123.1, 52.1, 44.2, 39.5. |
| **Chiral HPLC** | system J: t_{R} = 30.6 min, purity (λ = 254 nm): ≥ 99 % |

### Example 18: (S,E)-4-(2-(Dimethylamino)-3-(3-(naphthalen-1-yl)acrylamido)propyl)benzamide formiate

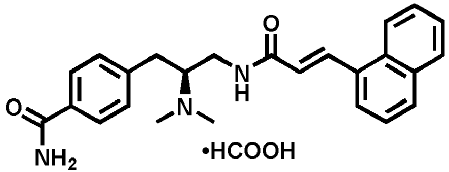

Formaldehyde was freshly prepared from paraformaldehyde (12 mg, 0.14 mmol, 10 eq., 35 % in water) by refluxing for 3 h in a sealed tube. Example 17 (6.0 mg, 14 mol, 1 eq.) was dissolved in a mixture of water: acetonitrile 0.5 mL : 2.5 mL. Sodium triacetoxyborohydride (15 mg, 72 µmol, 5 eq.) and subsequently formaldehyde (10 eq.) were added at ambient temperature. The reaction was stirred for 15 min. Saturated sodium bicarbonate solution (3 mL) and a spatula tip of sodium carbonate was added (pH = 9). The aqueous phase was extracted five times with 5 mL dichloromethane and evaporated to dryness. The crude was dissolved in 2.5 mL of acetonitrile and 0.5 mL of a 2 M sodium hydroxide solution and stirred at r.t. for 1 h. The aqueous phase was diluted with 2.5 mL dist. water, extracted five times with 5 mL dichloromethane and evaporated to dryness. The procedure was repeated two times until no N-hydroxymethyl side product was found by LC-MS analysis. The crude was purified by preparative HPLC (RP-C8, flow: 10 mL/min, solvent system: ACN/H₂O + 0.1 % formic acid, 10 % ACN for 3 min, 10 % to 33 % ACN in 14 min, 33 % to 100 % in 1 min, t_{R} = 15.1 min). The product was obtained as a white solid (5.0 mg, 11 µmol, 78 %).

| | |
|---|---|
| **Melting point** | 96 °C |
| **[α]_{D}²⁵** | + 24.4° (c = 0.19, MeOH) |
| **ESI (m/z)** | 402.20 [M+H]⁺ |
| **HRMS (m/z)** | [M+H]⁺ calculated for C₂₅H₂₇N₃O₂: 402.2176, found: 402.2175. |
| **¹H NMR** | (600 MHz, DMSO-*d*₆) δ 8.19 (s, 1H), 8.18 - 8.13 (m, 2H), 8.00 (t, *J* = 5.5 Hz, 1H), 7.99 - 7.95 (m, 2H), 7.89 (s, 1H), 7.82 - 7.79 (m, 2H), 7.77 - 7.76 (m, 1H), 7.61 (ddd, *J* = 8.4, 6.8, 1.5 Hz, 1H), 7.59 - 7.54 (m, 2H), 7.33 - 7.30 (m, 2H), 7.26 (s, 1H), 6.77 (d, *J* = 15.5 Hz, 1H), 3.28 - 3.20 (m, 2H), 2.94 - 2.86 (m, 2H), 2.55 - 2.52 (m, 1H), 2.31 (s, 6H). |
| **¹³CNMR** | (151 MHz, DMSO) δ 167.7, 164.6, 163.4, 143.9, 134.8, 133.2, 131.9, 131.7, 130.7, 129.3, 128.7, 128.5, 127.4, 126.8, 126.1, 125.6, 125.2, 124.3, 123.0, 64.6, 40.1, 38.5, 32.2. |
| **HPLC** | system 1: t_{R} = 15.7 min, purity (λ = 230 nm): 95 %, (λ = 254 nm): 97 % |
| | system 2: t_{R} = 13.5 min, purity (λ = 230 nm): 95 %, (λ = 254 nm): 96 % |
| **Chiral HPLC** | system J: t_{R} = 42.0 min, purity (λ = 280 nm): ≥ 99 % |

### Example 19: (S,E)-N-(2-(Dimethylamino)-3-(4-hydroxyphenyl)propyl)-3-(thiophen-3-yl)acrylamide

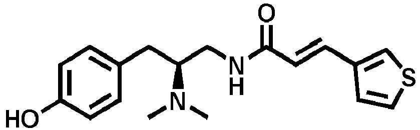

To a solution of Intermediate 6 (20 mg, 120 µmol, 1 eq.) in 0.5 mL dry DMF were added (*E*)-3-(thiophen-3-yl)acrylic acid (19 mg, 120 µmol, 1.0 eq.), PyBOP (63 mg, 120 µmol, 1.0 eq.) and TEA (34 µL, 0.24 mmol, 2 eq.) in 0.5 mL dry DMF. After stirring for 1 h at room temperature the solvent was removed under vacuum. The crude oil was dissolved in 5 mL of dichloromethane and extracted three times with 5 mL of saturated sodium bicarbonate solution. The organic phase was extracted with four times 5 mL of 2 N HCI solution. The combined acidic phases were basified to pH = 10 with 6 N NaOH solution and five times extracted with 5 mL of DCM to remove side products. Afterwards, the basic phase was extracted four times with 5 mL of ethyl acetate to extract the product. The combined ethyl acetate phases were dried over sodium sulfate and evaporated to dryness. The product was obtained as a white solid (23 mg, 70 µmol, 58 %). Further purification was not necessary.

| | |
|---|---|
| **Melting point** | 168 - 170 °C |
| **[α]_{D}²⁴** | + 28.9 ° (c = 0.56, MeOH) |
| **ESI (m/z)** | 331.18 [M+H]⁺ |
| **HRMS (m/z)** | [M+H]⁺ calculated for C₁₈H₂₂N₂O₂S: 331.1475, found: 331.1476. |
| **¹H NMR** | (600 MHz, Chloroform-d) δ 7.57 (d, *J* = 15.6 Hz, 1H), 7.43 (dd, *J* = 2.8, 1.2 Hz, 1H), 7.30 (dd, *J* = 5.1, 2.9 Hz, 1H), 7.28 - 7.25 (m, 1H), 7.02 - 6.96 (m, 2H), 6.81 - 6.76 (m, 2H), 6.27 (d, *J* = 15.5 Hz, 1H), 3.66 - 3.58 (m, 1H), 3.13 - 3.07 (m, 1H), 2.95 (dd, *J* = 13.5, 3.6 Hz, 1H), 2.89 - 2.83 (m, 1H), 2.43 (s, 6H), 2.40 - 2.32 (m, 1H). |
| **¹³C NMR** | (151 MHz, CDCl₃) δ 166.6, 155.4, 137.8, 134.8, 130.0, 129.2, 127.2, 126.6, 125.2, 120.2, 115.8, 65.3, 40.2, 39.5, 30.7. |
| **HPLC** | system 1: t_{R} = 13.7 min, purity (λ = 254 nm): ≥ 99 %, (λ = 280 nm): ≥ 99 % |
| | system 2: t_{R} = 12.6 min, purity (λ = 254 nm): ≥ 99 %, (λ = 280 nm): 99 % |
| **Chiral HPLC** | system K: t_{R} = 22.4 min, (λ = 254 nm): ≥ 99 % |

### A-4 Syntheses of comparative compounds:

### Comparative Example 1: (S)-2-(Dimethylamino)-3-(4-hydroxyphenyl)propyl (2-(thiophen-3-yl)ethyl)carbamate formiate

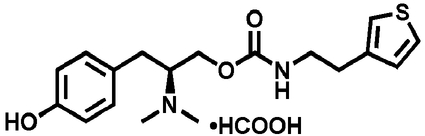

Intermediate 3 (17.8 mg, 91 µmol) and 4-nitrophenyl (2-(thiophen-3-yl)ethyl)carbamate (26.7 mg, 92 µmol, 1eq.) were dissolved in 0.5 mL dry DMF and the bright yellow solution stirred at r.t. for 22 h. The solvent was evaporated under high vacuum and the remaining oil was dissolved in 5 mL of dichloromethane, washed with saturated sodium hydrogen carbonate solution (three times 5 mL) and dried over magnesium sulfate. After evaporation the crude was purified by flash column chromatography (eluent: DCM . MeOH + 0.1 % ammonia (25 % aq.), 95:5 to 5:1) to give a pale yellow oil, which was purified by preparative HPLC (RP-C8, flow = 7 mL/min, solvent system: ACN/H₂O + 0.1 % formic acid, 5 % ACN for 3 min, 5 % ACN to 40 % ACN in 17 min, 40 % ACN to 95 % ACN in 2 min, t_{R} = 15.2 min). The product was obtained as a white solid (5 mg, 13 µmol, 14 %).

| | |
|---|---|
| **[α]_{D}²⁴** | + 4.0° (c = 0.20, MeOH) |
| **ESI (m/z)** | 349.12 [M+H]⁺ |
| **HRMS (m/z)** | [M+H]⁺ calculated for C₁₈H₂₄N₂O₃S: 349.1580, found: 349.1579. |
| **¹H NMR** | (400 MHz, Methanol-*d*₄) δ 8.52 (s, 1H), 7.34 (dd, *J* = 4.9, 2.9 Hz, 1H), 7.32 - 7.28 (m, 2H), 7.15 - 7.11 (m, 1H), 7.07 - 7.03 (m, 2H), 7.02 (dd, *J* = 5.0, 1.3 Hz, 1H), 3.69 (dd, *J* = 12.5, 3.7 Hz, 1H), 3.60 (dd, *J* = 12.5, 7.3 Hz, 1H), 3.54 - 3.44 (m, 1H), 3.41 (t, *J* = 7.2 Hz, 2H), 3.15 (dd, *J* = 13.5, 4.2 Hz, 1H), 2.91 (s, 6H), 2.89 - 2.85 (m, 3H). |
| **¹³C NMR** | (151 MHz, MeOD) δ 170.0, 157.2, 151.9, 140.6, 134.4, 131.2, 129.3, 126.6, 123.3, 122.3, 69.4, 58.8, 42.8, 40.8, 31.3, 31.2. |
| **HPLC** | system 1: t_{R} = 15.1 min, purity (λ = 230 nm): 97 %, (λ = 254 nm): 95 % |
| | system 2: t_{R} = 13.6 min, purity (λ = 230 nm): 97 %, (λ = 254 nm): 96 % |
| **Chiral HPLC** | system A: t_{R} = 15.7 min, purity (λ = 254 nm): ≥ 99 % |

### Comparative Example 2: (S)-2-(Dimethylamino)-3-(4-hydroxyphenyl)propyl cinnamate

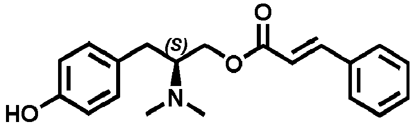

To a solution of Intermediate 3 (16 mg, 82 µmol,. 1 eq.) in 0.5 mL dry DMF were added (*E*)-cinnamic acid (15 mg, 98 µmol, 1.2 eq.), PyBOP (52 mg, 98 µmol, 1.2 eq.) and triethylamine (23 µL, 0.16 mmol, 2 eq.) in 0.5 mL dry DMF. After stirring for 17 h at room temperature the solvent was removed under vacuum. The crude oil was dissolved in 5 mL of chloroform and extracted three times with 5 mL of saturated sodium bicarbonate solution. The organic phase was dried over sodium sulfate and evaporated to dryness. The crude was first passed through a silica plug (eluent: ethyl acetate + 0.1 % TEA) and then purified by flash column chromatography (solvent system: DCM : MeOH + 0.1 % ammonia (25 % aq.), 95: 5). The product was obtained as a white solid (7.0 mg, 22 µmol, 26 %).

| | |
|---|---|
| **Melting point** | 158 °C |
| **[α]_{D}²⁵** | + 59.1° (c = 0.183, MeOH) |
| **ESI (m/z)** | 326.18 [M+H]⁺ |
| **HRMS (m/z)** | [M+H]⁺ calculated for C₂₀H₂₃NO₃: 326.1751, found: 326.1750. |
| **¹H NMR** | (400 MHz, Chloroform-d) δ 7.56 (d, *J* = 16.0 Hz, 1H), 7.46 - 7.40 (m, 2H), 7.33 - 7.29 (m, 3H), 7.02 - 6.96 (m, 2H), 6.70 - 6.63 (m, 2H), 6.36 (d, *J* = 16.0 Hz, 1H), 4.17 (dd, *J* = 11.9, 6.5 Hz, 1H), 4.12 (dd, *J* = 11.8, 4.2 Hz, 1H), 3.00-2.90 (m, 1H), 2.83 (dd, *J* = 13.8, 4.9 Hz, 1H), 2.50 (dd, *J* = 13.8, 9.4 Hz, 1H), 2.37 (s, 6H). |
| **¹³C NMR** | (101 MHz, CDCl₃) δ 167.1, 154.4, 145.0, 134.3, 130.9, 130.3, 130.1, 128.9, 128.1, 117.9, 115.5, 64.7, 62.7, 41.3, 32.6. |
| **HPLC** | system 1: t_{R} = 19.5 min, purity (λ = 280 nm): 95 % |
| | system 2: t_{R} = 18.0 min, purity (λ = 280 nm): 97 % |

### Comparative Example 3: (S,E)-N-(2-(Dimethylamino)-3-(4-hydroxyphenyl)propyl)-2-phenyldiazene-1-carboxamide formiate

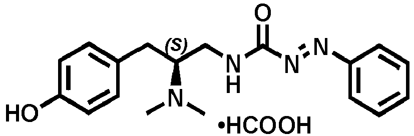

The synthesis of *tert*-butyl phenylazocarboxylate has previously been described in the literature (Pirzer, A.S., et al., *Benzyl Phenylsemicarbazides: A Chemistry-Driven Approach Leading to G Protein-Biased Dopamine D4 Receptor Agonists with High Subtype Selectivity.*

Journal of Medicinal Chemistry, 2019. 62(21): p. 9658-9679). *tert*-Butyl phenylazocarboxylate (20 mg, 0.10 mmol, 1 eq.) and potassium carbonate (71 mg, 0.51 mmol, 5 eq.) in 0.5 mL of dry DMF were slowly added a solution of Intermediate 6 in 0.5 mL DMF at 0 °C. After stirring at r.t. for 20 h the solvent was evaporated under vacuum and the crude was purified by preparative HPLC (RP-C8, flow: 10 mL/min, solvent system: ACN/H₂O + 0.1 % formic acid, 5 % ACN for 2 min, 5 % to 20 % ACN in 30 min, 20 % ACN to 95 % ACN in 2 min, t_{R} = 16.7 min). The product was obtained as an orange solid (14 mg, 38 µmol, 37 %).

| | |
|---|---|
| **Melting point** | 109 °C |
| **[α]_{D}²⁵** | + 5.8° (c = 0.80, MeOH) |
| **ESI (m/z)** | 327.13 [M+H]⁺ |
| **HRMS (m/z)** | [M+H]⁺ calculated for C₁₈H₂₂N₄O₂: 327.1816, found: 327.1817. |
| **¹H NMR** | (600 MHz, DMSO-*d*₆) δ 8.25 (t, *J* = 5.6 Hz, 1H), 8.20 (s, 1H), 7.85 - 7.81 (m, 2H), 7.66 - 7.59 (m, 3H), 7.04 - 6.99 (m, 2H), 6.69 - 6.66 (m, 2H), 3.32 (ddd, *J* = 14.0, 8.8, 5.4 Hz, 1H), 3.19 - 3.13 (m, 1H), 2.90 - 2.82 (m, 1H), 2.75 (dd, *J* = 13.8, 5.3 Hz, 1H), 2.36 (dd, *J* = 13.8, 8.4 Hz, 1H), 2.29 (s, 6H). |
| **¹³CNMR** | (151 MHz, DMSO) δ 163.4, 162.5, 155.3, 151.0, 133.0, 129.9, 129.7, 129.5, 122.7, 115.0, 64.6, 40.0, under DMSO signal, identified by 2D-NMR), 31.2. |
| **HPLC** | system 1: t_{R} = 13.2 min, purity (λ = 230 nm): ≥ 99 %, (λ = 280 nm): ≥ 99 % |
| | system 2: t_{R} = 11.6 min, purity (λ = 230 nm): 98 %, (λ = 280 nm): ≥ 99 % |
| **Chiral HPLC** | system J: t_{R} = 28.9 min, purity (λ = 280 nm): ≥ 99 % |

### A-5 Biochemical results for inventive compounds:

### Radioligand binding experiments

Radioligand binding assays are very useful for obtaining detailed information on the binding properties of the described examples. To verify the subtype selectivity of the described compounds for µOR over δOP and κOR, respectively, the binding affinities of the described examples to the different human opioid receptors in an in-vitro test system were measured.

For the determination of receptor binding affinity competitive radioligand binding experiments were performed as described in literature (H. Huebner, C. Haubmann, W. Utz, P. Gmeiner, J. Med. Chem., 2000, 43, 756-762). In brief, HEK 293T cells were transiently transfected with the cDNAs coding for the human opioid receptor subtypes µOR, δOR, and kOR, respectively, using a solution of polyethylenimine in PBS (PEI, linear, 25 kDa, Polysciences) as transfection reagent at a 3:1 PEI to cDNA ratio (I. Fish, A. Stoessel, K. Eitel, C. Valant, S. Albold, H. Huebner, D. Moeller, M.J. Clark, R.K. Sunahara, A. Christopoulos, B.K. Shoichet, P. Gmeiner, J. Med. Chem., 2017, 60, 9239-9250). After preparation of membranes, radioligand displacement assays were run in 96-well format at a final volume of 200 µL in binding buffer (50 mM Tris-HCI, 1 mM EDTA, 5 mM MgCl₂, 100 µg/mL bacitracin, 5 µg/mL soybean trypsin inhibitor, pH 7.4) with the radioligand [³H]diprenorphine (specific activity = 31 Ci/mmol) at a final concentration of 0.20-0.30 nM. Membranes were used with a protein concentration of 4-8 µg/assay tube, with a Bₘₐₓ value of 2,800 ± 530 fmol/mg, and a K_{D} value of 0.10 ± 0.021 nM for µOR, a Bₘₐₓ of 2,000 ± 300 fmol/mg, K_{D} of 0.22 ± 0.016 nM for δOR, and a Bₘₐₓ of 3,900 ± 1,000 fmol/mg, K_{D} of 0.12 ± 0.017 nM for kOR, respectively. Unspecific binding was determined in the presence of 10 µM of the naloxone. Protein concentrations were determined employing the method of Lowry with bovine serum albumin as standard (O.H. Lowry, N.J. Rosebrough, A.L. Farr, R.J. Randall, J. Biol. Chem., 1951, 193, 265-275). The resulting competition curves of the receptor binding experiments were analyzed by nonlinear regression. The data were initially fit using a sigmoid model to provide IC₅₀ values, which were subsequently transformed to Kᵢ values according to the equation of Cheng and Prusoff (Y.-C. Cheng, W. H. Prusoff, Biochem. Pharmacol., 1973, 22, 3099-3108). Three to seven experiments per compound were performed, with each concentration in triplicate. Results are displayed in the table below.

| Compound | δOR | κOR | µOR |
|---|---|---|---|
| | [³H]diprenorphine | | |
| Example 1 | (7) 25 | (7) 120 | (7) 13 |
| 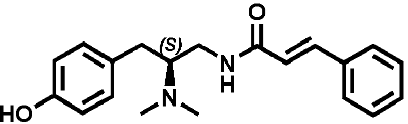 | | | |
| Example 2 | (2) 140 | (2) 230 | (2) 18 |
| 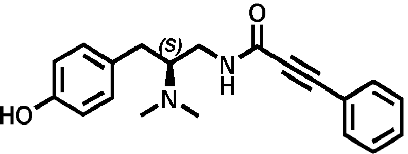 | | | |
| Example 3 | (5) 120 | (4) 120 | (5) 28 |
| 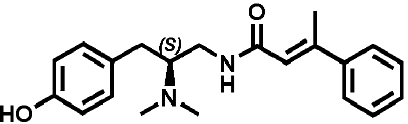 | | | |
| Example 4 | (3) 49 | (4) 110 | (6) 46 |
| 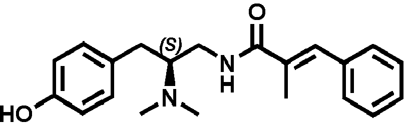 | | | |
| Example 5 | (3) 1300 | (3) 970 | (3) 310 |
| 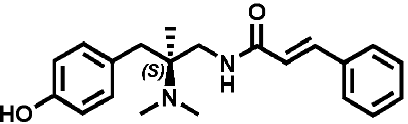 | | | |
| Example 6 | (3) 220 | (3) 180 | (4) 18 |
| 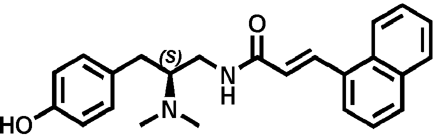 | | | |
| Example 7 | (4) 180 | (4) 140 | (4) 28 |
| 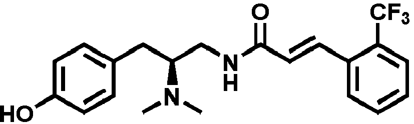 | | | |
| Example 8 | (5) 120 | (5) 580 | (5) 30 |
| 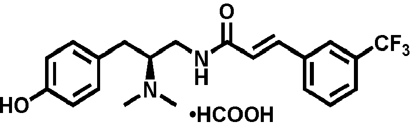 | | | |
| Example 9 | (3) 1100 | (3) 2800 | (3) 170 |
| 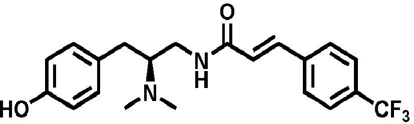 | | | |
| Example 10 | (2) 510 | (2) 440 | (2) 58 |
| 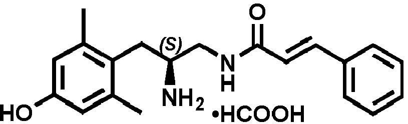 | | | |
| Example 11 | (5) 360 | (5) 220 | (5) 19 |
| 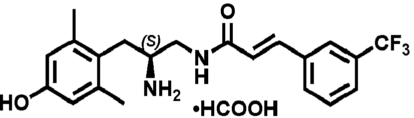 | | | |
| Example 12 | (3) 310 | (3) 310 | (3) 12 |
| 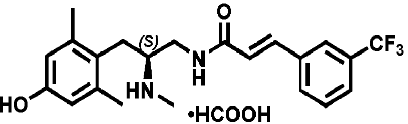 | | | |
| Example 13 | (4) 300 | (4) 500 | (5) 26 |
| 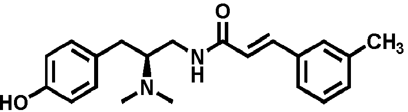 | | | |
| Example 14 | (3) 2900 | (3) 5600 | (3) 460 |
| 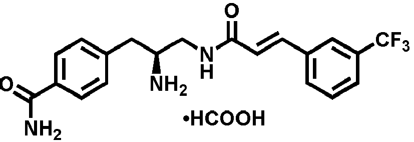 | | | |
| Example 15 | (4) 55 | (4) 450 | (5) 6.7 |
| 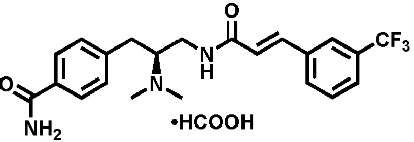 | | | |
| Example 16 | (4) 250 | (4) 600 | (4) 23 |
| 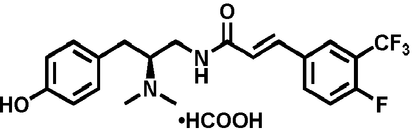 | | | |
| Example 18 | (4) 130 | (4) 200 | (5) 6.2 |
| 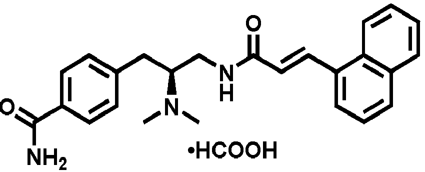 | | | |
| Example 19 | (4) 59 | (4) 130 | (4) 8.5 |
| 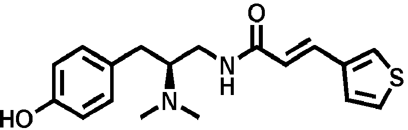 | | | |

In the above table, the number in brackets indicates the number of experiments and the subsequent number indicates the mean Kᵢ value in nM.

### Functional test on G-protein mediated signaling at µOR

Functionally selective drugs, which are biased towards Gi protein activation over β-arrestin signaling at the µOR are indicated to show an improved side effect profile. Thus, the characterization of the functional properties of the described examples at the µOR were performed by determination of the ligand stimulated G-protein related signaling and β-arrestin recruitment applying cell based in-vitro test systems. Full and partial agonists as well as antagonists for G protein related signaling were identified. Results are displayed in the table below.

The determination of G-protein stimulated µOR activation was measured applying an IP₁ accumulation assay as described previously (J. Kruell, S.K. Fehler, L. Hofmann, N. Nebel, S. Maschauer, O. Prante, P. Gmeiner, H. Lanig, H. Huebner, M.R. Heinrich. ChemMedChem 2020, 15, 1175-1186. C. Gentzsch, K. Seier, A. Drakopoulos, M.-L. Jobin, Y. Lanoiselee, Z. Koszegi, D. Maurel, R. Sounier, H. Huebner, P. Gmeiner, S. Granier, D. Calebiro, M. Decker. Ang. Chem. Int. Ed. 2020, 59, 2-9). In brief, HEK-293T cells were grown to confluence of approximately 70% and transiently transfected with the cDNA of the human µOR receptor and the hybrid G-protein Gα_{qi}. After one day cells were seeded into 384-well microplates. For testing cells were incubated with test compounds (final range of concentration from 1 pM up to 10 µM) for 180 min at 37 °C followed by addition of detection reagents. Time resolved fluorescence resonance energy transfer (HTRF) was determined using a microplate reader. Each compound was tested in duplicates in 3-9 individual experiments in comparison to the reference compound DAMGO. The resulting dose response curves were analyzed by nonlinear regression using the algorithms in PRISM 6.0 (GraphPad software, San Diego, CA), fitted with a sigmoid model and normalized to basal activity (0 %) and the maximal effect caused by the reference full agonist DAMGO (100 %).

### Recruitment of β-arrestin-2

Measurement of **β-arrestin-2** recruitment was done applying an enzyme-fragment complementation assay as described previously (C. Gentzsch, K. Seier, A. Drakopoulos, M.-L. Jobin, Y. Lanoiselee, Z. Koszegi, D. Maurel, R. Sounier, H. Huebner, P. Gmeiner, S. Granier, D. Calebiro, M. Decker. Ang. Chem. Int. Ed. 2020, 59, 2-9. J. Hellmann, M. Drabek, J. Yin, J. Gunera, T. Proell, F. Kraus, C.J. Langmead, H. Huebner, D. Weikert, P. Kolb, D.M. Rosenbaum, P. Gmeiner. PNAS 2020, 117, 18059-18067). In brief, HEK-293 cells stably expressing the enzyme acceptor (EA) tagged β-arrestin-2 fusion protein were transiently co-transfected with the ProLink tagged µOR-PK1 After 24 h cells were transferred into 384-well microplates and cultured for further 24 h. To start arrestin recruitment compounds were added to the cells to get a final concentration in a range of 10 pM to 100 µM. After incubation for 90 min reaction was stopped by addition of detection mix. Chemiluminescence was determined in duplicates (3-8 individual experiments for each compound in comparison to the reference agonist DAMGO). Data analysis was done as described above.

### Calculation of lipophilicity of the example compounds

Simultaneously, substances acting on the CNS-located opioid receptors should be able to reach sufficient brain concentrations for the mediation of analgesia. As a physico-chemical property like lipophilicity is a valuable parameter for describing compounds with improved pharmacokinetic properties the logP values of the example compounds have been calculated.

Therefore, molecules were drawn in their neutral form and converted to SMILES code in Chemdraw (version 18.2). SMILES codes were converted to logP values using the RDKit (RDKit. RDKit: Open-source cheminformatics. 2020; Available from: http://www.rdkit.org.; version 2019.03.01) module in Python (Van Rossum, G.D., F.L., Python 3 Reference Manual. 2009, Scotts Valley, CA: CreateSpace.; version 3.7.3). The implemented logP calculation method was developed by Crippen et al. (S.A. Wildman, and G.M. Crippen, Journal of Chemical Information and Computer Sciences, 1999, 39, 868-873). The calculated logP values are displayed in the table below.

| Compound | G-protein signaling | | β-arrestin recruitment | | lipophilicity |
|---|---|---|---|---|---|
| | EC₅₀ [nM] | Eₘₐₓ [%] | EC₅₀ [nM] | Eₘₐₓ [%] | calculated logP |
| 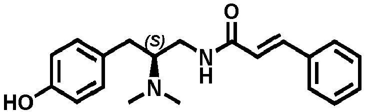 Example 1 | 7.0 | 63 | CBA | <5 | 2.70 |
| 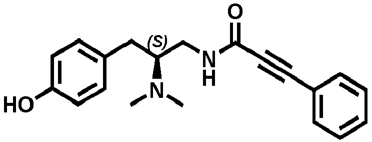 Example 2 | 22 | 42 | CBA | <5 | 2.03 |
| 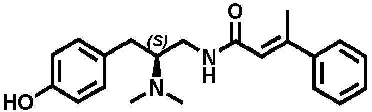 Example 3 | 11 | 76 | CBA | <5 | 3.09 |
| Example 4 | 17 | 62 | CBA | <5 | 3.09 |
| 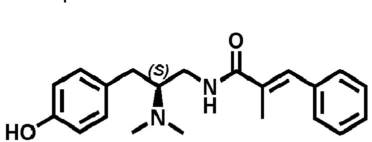 | | | | | |
| Example 5 | ND | ND | ND | ND | 3.09 |
| 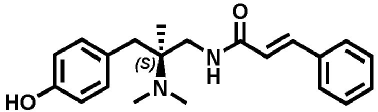 | | | | | |
| Example 6 | 20 | 92 | CBA | <5 | 3.85 |
| 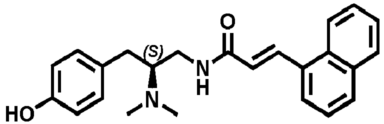 | | | | | |
| Example 7 | 13 | 44 | CBA | <5 | 3.71 |
| 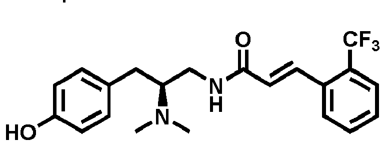 | | | | | |
| Example 8 | 15 | 100 | CBA | <5 | 3.71 |
| 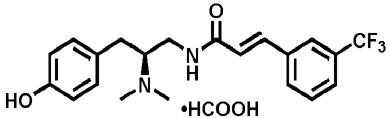 | | | | | |
| Example 9 | 67 | 92 | CBA | <5 | 3.71 |
| 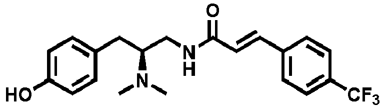 | | | | | |
| Example 10 | CBA | <5 | CBA | <5 | 2.71 |
| 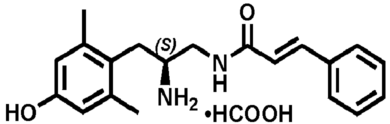 | | | | | |
| Example 11 | CBA | <5 | ND | ND | 3.73 |
| 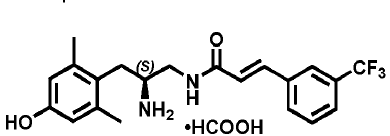 | | | | | |
| Example 12 | CBA | <5 | ND | ND | 3.99 |
| 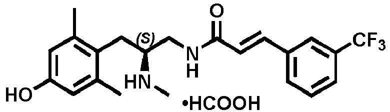 | | | | | |
| Example 13 | 41 | 78 | ND | ND | 3.00 |
| 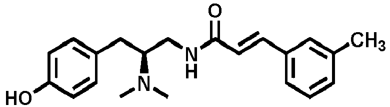 | | | | | |
| Example 14 | 2200 | 75 | CBA | <5 | 2.50 |
| 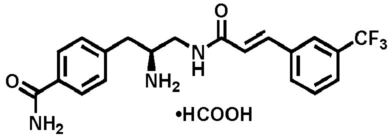 | | | | | |
| Example 15 | 6.3 | 95 | 130 | 5 | 3.11 |
| 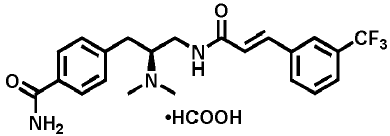 | | | | | |
| Example 16 | 40 | 102 | ND | ND | 3.15 |
| 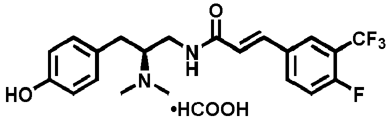 | | | | | |
| Example 18 | 9.8 | 92 | 120 | 4 | 3.24 |
| 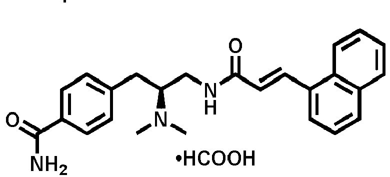 | | | | | |
| Example 19 | 7.5 | 53 | CBA | <5 | 2.76 |
| 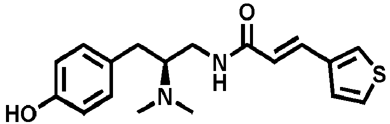 | | | | | |

In the above table, the numbers indicate mean EC₅₀ values in nM and Eₘₐₓ values relative to the full effect of reference agonist DAMGO [=100%], respectively and are derived from 3-9 individual experiments for G-protein signaling and 3-8 tests for arrestin recruitment. ND = not determined. CBA = could not be analyzed (in this case, the assay did not show a dose-response curve allowing an accurate determination of potency. This is typical for antagonists and very weak partial agonists)

### Determination of metabolic stability in rat liver microsomes and blood-brain barrier penetration in vivo

Pharmacokinetic properties such as metabolic stability and blood-brain-barrier permeability are important parameters for the development of drugs. Substances acting on the CNS-located opioid receptors should be able to reach sufficient brain concentrations for the mediation of analgesia. Thus, pharmacokinetic properties like metabolic stability and blood-brain-barrier permeability have been determined for selected compounds.

### Metabolic stability in rat liver microsomes

Metabolic stability experiments were performed as described previously (C. Hiller et al. Journal of Medicinal Chemistry, 2013, 56, 5130-5141.) with the only exception regarding the concentration of test compound of 20 µM used for the experiments. Pooled microsomes from rat liver (0.25 mg/test, male rat liver, Sprague-Dawley) were mixed with compound (20 µM final concentration) in a total volume of 500 µL buffer (48 mM Tris, 4.8 mM MgCl₂). Incubation started by adding NADPH to the mixture (final concentration of 1 mM). The assay was performed in a shaking incubator (350 rpm at 37 °C). Samples were collected after defined time intervals of 0 min, 15 min, 30 min and 60 min and the microsomal reaction was terminated by the addition of ice-cold acetonitrile solution containing an internal standard (c = 5 µM). The suspensions were centrifuged (13.000 rpm, 1 min) and the supernatant was analyzed by LC-MS (flow rate 0.4 mL/min, binary solvent system of methanol and water supplemented with 0.1 % formic acid, methanol gradient: 10 % for 1 min, 10 % to 100 % in 20 min, constant at 100 % for 5 min, 100 % to 10 % in 2 min, kept constant at 10 % for 2 min). The area under the curve (AUC) of the extracted ion count (EIC) chromatogram of the compound and the internal standard was determined for each time point. Control experiments for the determination of unspecific binding were conducted in parallel to the metabolic stability experiments in the absence of the cofactor NADPH. The assay was repeated in three independent experiments. The corresponding results are shown in Figure 1.

### Blood-brain barrier penetration in vivo.

Experiments on penetration of blood-brain barrier of example compound 8 were performed *in vivo* in mice. After subcutaneous administration of 20 mg/kg of example compound 8 the concentrations of test compound in plasma and brain were determined. The results are shown in Figure 2.

### A-6 Biochemical results for comparative compounds:

The following results were obtained in the same manner as set out above with respect to the inventive compounds in the section on "Radioligand binding experiments".

| Compound | δOR | κOR | µOR |
|---|---|---|---|
| | [³H]diprenorphine | | |
| Comparative Example 1 | (2) 3000 | (2) 3300 | (2) 2400 |
| 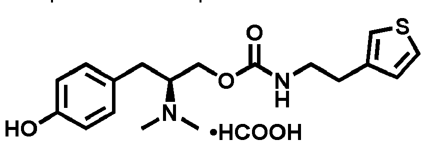 | | | |
| Comparative Example 2 | (2) 860 | (2) 320 | (2) 230 |
| 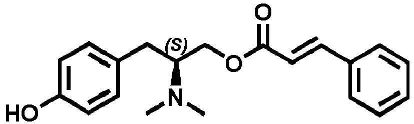 | | | |
| Comparative Example 3 | (3) 630 | (3) 540 | (3) 66 |
| 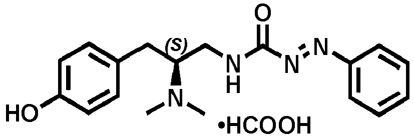 | | | |

In the above table, the number in brackets indicates the number of experiments and the subsequent number indicates the mean Kᵢ value in nM.

From a comparison of Example 1, which is according to the present invention, with Comparative Examples 1 to 3, it can be seen that the compounds of the present invention having the core structure shown in formula (I) are superior in activity and selectivity over compounds not having the N-X-groups and/or the adjacent C-C unsaturated group.

## Claims

1. A compound of the following formula (I) or (II), optionally in the form of a pharmaceutically acceptable salt, solvate, polymorph, cocrystal, tautomer, racemate, enantiomer, or diastereomer or mixture thereof: 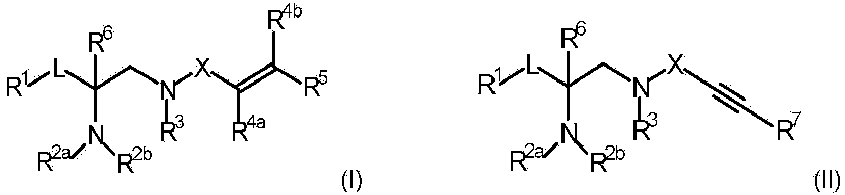 wherein
R¹ is an optionally substituted aryl group or an optionally substituted heteroaryl group,
L is selected from a bond and an optionally substituted alkylene and an optionally substituted heteroalkylene,
R^{2a} and R^{2b} are independently selected from hydrogen, optionally substituted alkyl, optionally substituted heteroalkyl, optionally substituted cycloalkyl, and optionally substituted heterocycloalkyl,
wherein, when at least one of R^{2a} and R^{2b} is optionally substituted alkyl, the optionally substituted alkyl of R^{2a} or R^{2b} is optionally linked to R¹ via a bond between a non-hydrogen atom of R^{2a} or R^{2b} and a non-hydrogen atom of R¹,
R³ is selected from hydrogen and optionally substituted alkyl,
X is selected from >C=O, >C=S, C=N(NO₂), C=C(NO₂)(H), >C=C(F)(H), >C=CF₂, >C=N(F), >C=C(CN)(H), >C=N(CN), >C=C(CN)(CN), >S(O), and >S(O)₂,
R^{4a} and R^{4b} are independently selected from hydrogen, optionally substituted alkyl and optionally substituted heteroalkyl,
R⁵ and R⁷ are independently selected from optionally substituted cycloalkyl, optionally substituted heterocycloalkyl, optionally substituted aryl and optionally substituted heteroaryl,
R⁶ is hydrogen or optionally substituted alkyl, wherein the optionally substituted alkyl of R⁶ is optionally linked to R¹ via a bond between a non-hydrogen atom of R⁶ and a non-hydrogen atom of R¹,
wherein
any hydrogen atoms in formulae (I) and (II) are optionally independently replaced by fluorine,
the one or more optional substituents of the optionally substituted aryl group and the optionally substituted heteroaryl group are independently selected from the group consisting of -halogen, -CN, -C(O)R**, -COOR**, -C(O)NR**R**, -NR**R**, -NR**-C(O)R**, -N(R**)-C(O)-OR**, -N(R**)-C(O) -NR**R**, -N(R**)-S(O)₂R**, -(optionally substituted alkyl), -OR**, -(optionally substituted heterocyclyl), -(optionally substituted alkylene)-(optionally substituted heterocyclyl), -(optionally substituted carbocyclyl), and -(optionally substituted alkylene)-(optionally substituted carbocyclyl), wherein R** is H, -(optionally substituted alkyl), -(optionally substituted heterocyclyl), or -(optionally substituted carbocyclyl), and
wherein the optional substituent of the optionally substituted alkyl and the optionally substituted alkylene can be independently -halogen, -CN, -NO₂, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar}, -N(R^{ar})-S(O)₂-NR^{ar}R^{ar}, -P(O)(OR^{ar})₂, or -O-P(O)(OR^{ar})2; wherein R^{ar} is H, alkyl, heterocyclyl or carbocyclyl;
wherein the optional substituent of the optionally substituted heterocyclyl, the optionally substituted carbocyclyl can be independently alkyl, -halogen, -CN, -NO₂, oxo, -C(O)R^{ar}, -COOR^{ar}, -C(O)NR^{ar}R^{ar}, -NR^{ar}R^{ar}, -NR^{ar}-C(O)R^{ar}, -N(R^{ar})-C(O)-OR^{ar}, -N(R^{ar})-C(O)-NR^{ar}R^{ar}, -N(R^{ar})-S(O)₂R^{ar}, -OR^{ar}, -O-C(O)R^{ar}, -O-C(O)-NR^{ar}R^{ar}, -SR^{ar}, -S(O)R^{ar}, -S(O)₂R^{ar}, -S(O)₂-NR^{ar}R^{ar}, -N(R^{ar})-S(O)₂-NR^{ar}R^{ar}, -P(O)(OR^{ar})₂, or -O-P(O)(OR^{ar})₂; wherein R^{ar} is H, alkyl, heterocyclyl or carbocyclyl;
the one or more optional substituents of the optionally substituted alkyl, optionally substituted alkylene, optionally substituted heteroalkylene, and optionally substituted heteroalkyl are independently selected from -halogen, -CN, -NO₂, oxo, -C(O)R^{alk}, -COOR^{alk}, -C(O)NR^{alk}R^{alk}, -NR^{alk}R^{alk}, -NR^{alk}-C(O)R^{alk}, -N(R^{alk})-C(O)-OR^{alk}, -N(R^{alk})-C(O)-NR^{alk}R^{alk}, -N(R^{alk})-S(O)₂R^{alk}, -OR^{alk}, -O-C(O)R^{alk}, -O-C(O)-NR^{alk}R^{alk}, -SR^{alk}, -S(O)R^{alk}, -S(O)₂R^{alk}, -S(O)₂-NR^{alk}R^{alk}, -N(R^{alk})-S(O)₂-NR^{alk}R^{alk}, -P(O)(OR^{alk})₂, or -O-P(O)(OR^{alk})₂; wherein R^{alk} is H, alkyl or alkyl substituted with one or more F, and
the one or more optional substituents of the optionally substituted cycloalkyl and optionally substituted heterocycloalkyl are independently selected from -alkyl, -halogen, -CN, -NO₂, oxo, -C(O)R^{cyc}, -COOR^{cyc}, -C(O)NR^{cyc}R^{cyc}, -NR^{cyc}R^{cyc}, -NR^{cyc}-C(O)R^{cyc}, -N(R^{cyc})-C(O)-OR^{cyc}, -N(R^{cyc})-C(O)-NR^{cyc}R^{cyc}, -N(R^{cyc})-S(O)₂R^{cyc}, -OR^{cyc}, -O-C(O)R^{cyc}, -O-C(O)-NR^{cyc}R^{cyc}, -SR^{cyc}, -S(O)R^{cyc}, -S(O)₂R^{cyc}, -S(O)₂-NR^{cyc}R^{cyc}, -N(R^{cyc})-S(O)₂-NR^{cyc}R^{cyc}, -P(O)(OR^{cyc})₂, or -O-P(O)(OR^{cyc})₂; wherein R^{cyc} is H, alkyl or alkyl substituted with one or more F.

2. The compound according to claim 1, wherein R¹ is an optionally substituted phenyl group which is optionally linked to R⁶ via a bond between a non-hydrogen atom of R⁶ and a non-hydrogen atom of R¹.

3. The compound according to claim 2, wherein the phenyl group in R¹ contains at least one hydroxy or amido substituent in para position.

4. The compound according to any one of the preceding claims, wherein L is selected from a bond, an optionally substituted methyl and an optionally substituted ethyl, preferably wherein L is an optionally substituted methylene group, preferably an unsubstituted methylene group.

5. The compound according to any one of the preceding claims, wherein R^{2a} and R^{2b} are independently selected from optionally substituted alkyl, preferably wherein R^{2a} and R^{2b} are independently selected from alkyl or haloalkyl.

6. The compound according to any one of the preceding claims, wherein R^{2a} and R^{2b} are independently selected from methyl, ethyl and propyl, wherein preferably both R^{2a} and R^{2b} are methyl.

7. The compound according to any one of the preceding claims, wherein R³ is hydrogen.

8. The compound according to any one of the preceding claims, wherein X is selected from >C=O, >C=S, >C=C(NO₂)(H), >C=C(CN)(H), >C=C(CN)(CN), >S(O), and >S(O)₂, preferably >C=O and >C=S, more preferably >C=O.

9. The compound according to any one of the preceding claims, wherein R^{4a} and R^{4b} are independently selected from hydrogen, optionally substituted methyl, optionally substituted ethyl and optionally substituted propyl, wherein preferably at least one of R^{4a} and R^{4b} is hydrogen.

10. The compound according to any one of the preceding claims, wherein R⁵ and R⁷ are independently selected from optionally substituted aryl and optionally substituted heteroaryl.

11. The compound according to any one of the preceding claims, wherein R⁵ and R⁷ are independently selected from optionally substituted phenyl, optionally substituted naphthyl, optionally substituted pyridyl and optionally substituted thienyl.

12. The compound according to any one of the preceding claims, wherein R⁶ is hydrogen or optionally substituted alkyl, wherein alkyl is preferably selected from methyl, ethyl and propyl, more preferably methyl or ethyl, even more preferably methyl, wherein R⁶ is most preferably hydrogen.

13. The compound according to any one of the preceding claims, wherein formulae (I) and (II) are represented by the following formulae (I-a) and (II-b), respectively: 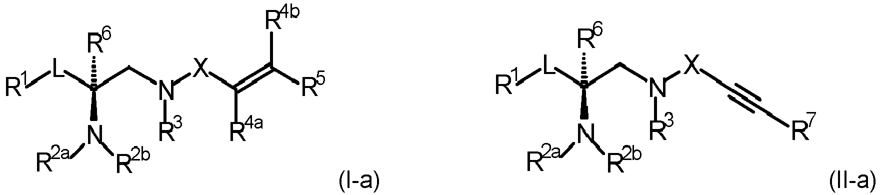 wherein R¹, R^{2a}, R^{2b}, R³, R^{4a}, R^{4b}, R⁵, R⁶, R⁷, L and X are as defined in any of the preceding claims.

14. A pharmaceutical composition comprising:
a compound having formula (I) or (II) as defined in any of claims 1 to 13, optionally in the form of a pharmaceutically acceptable salt, solvate, polymorph, cocrystal, tautomer, racemate, enantiomer, or diastereomer or mixture thereof,
and optionally one or more pharmaceutically acceptable excipient(s) and/or carrier(s).

15. A compound having formula (I) or (II) as defined in any of claims 1 to 13, optionally in the form of a pharmaceutically acceptable salt, solvate, polymorph, cocrystal, tautomer, racemate, enantiomer, or diastereomer or mixture thereof, wherein the compound is for use in the treatment, amelioration or prevention of pain, opioid overdose, addiction, neuropsychiatric disorder, Prader-Willi Syndrome, gastrointestinal disorder, skin disorder, dyspnea, and temporomandibular joint dysfunction.
